(19) Europäisches Patentamt / European Patent Office / Office européen des brevets

(11) EP 2 579 996 B1

(12) **EUROPEAN PATENT SPECIFICATION**

(45) Date of publication and mention of the grant of the patent:
**02.01.2019 Bulletin 2019/01**

(21) Application number: **11731554.9**

(22) Date of filing: **11.05.2011**

(51) Int Cl.:
***C23C 16/505*** *(2006.01)* ***C23C 16/52*** *(2006.01)*
***C23C 16/54*** *(2006.01)* ***B05D 1/00*** *(2006.01)*
***C23C 16/04*** *(2006.01)* ***C23C 16/40*** *(2006.01)*
***G01N 15/08*** *(2006.01)* ***B05D 5/08*** *(2006.01)*
***B05D 7/02*** *(2006.01)* *G01N 33/00* *(2006.01)*

(86) International application number:
**PCT/US2011/036097**

(87) International publication number:
**WO 2011/143329 (17.11.2011 Gazette 2011/46)**

(54) **LUBRICITY VESSEL COATING PROCESS**

SCHMIERFÄHIGKEITSBESCHICHTUNGSVERFAHREN EINES GEFÄSSES

PROCÉDÉ DE REVÊTEMENT LUBRIFIANT POUR CUVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:
**14.03.2011 US 452526 P**
**12.11.2010 US 413334 P**
**29.06.2010 US 359434 P**
**12.05.2010 PCT/US2010/034586**
**12.05.2010 EP 10162761**
**12.05.2010 US 779007**

(43) Date of publication of application:
**17.04.2013 Bulletin 2013/16**

(73) Proprietor: **SiO2 Medical Products, Inc.**
**Auburn, AL 36832 (US)**

(72) Inventors:
- **FELTS, John, T.**
  **Alameda**
  **California 94501 (US)**
- **FISK, Thomas, E.**
  **Green Valley**
  **Arizona 85614 (US)**
- **ABRAMS, Robert, S.**
  **Albany**
  **New York 12203 (US)**
- **FERGUSON, John**
  **Auburn**
  **Alabama 36832 (US)**
- **FREEDMAN, Jonathan, R.**
  **Auburn**
  **Alabama 36830 (US)**
- **PANGBORN, Robert, J.**
  **Harbor Springs**
  **Michigan 49740 (US)**
- **SAGONA, Peter, J.**
  **Pottstown**
  **Pennsylvania 19465 (US)**

(74) Representative: **Maiwald Patent- und Rechtsanwaltsgesellschaft mbH**
**Elisenhof**
**Elisenstraße 3**
**80335 München (DE)**

(56) References cited:
**EP-A2- 0 329 041** **US-A- 5 494 712**
**US-A- 5 679 413**

- **"Oxford instruments plasmalab 80plus", , 1 January 2011 (2011-01-01), XP55015205, Retrieved from the Internet: URL:http://www.oxfordplasma.de/pdf_inst/plas_80.pdf [retrieved on 2011-12-20]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The following European patent applications: EP10162755.2 filed May 12, 2010; EP10162760.2 filed May 12, 2010; EP10162756.0 filed May 12, 2010; EP10162758.6 filed May 12, 2010; EP10162761.0 filed May 12, 2010; and EP10162757.8 filed May 12, 2010, describe apparatus, vessels, precursors, coatings and methods (in particular coating methods and test methods for examining the coatings) which can generally be used in performing the present invention, unless stated otherwise herein. They also describe $SiO_x$ barrier coatings to which reference is made herein.

FIELD OF THE INVENTION

The present invention relates to the technical field of fabrication of coated vessels for storing biologically active compounds or blood.

A method for coating a substrate surface by PECVD is provided, the method comprising generating a plasma from a gaseous reactant comprising an organosilicon precursor and $O_2$. A carrier gas may also be present. The lubricity, hydrophobicity and/or barrier properties of the coating are set by setting the ratio of the $O_2$ to the organosilicon precursor and to the carrier gas in the gaseous reactant, and by setting the electric power used for generating the plasma. In particular, a lubricity coating on at least a part of the interior surface of a syringe is made by said method.

BACKGROUND OF THE INVENTION

An important consideration when regarding syringes is to ensure that the plunger can move at a constant speed and with a constant force when it is pressed into the barrel. For this purpose, a lubricity layer, either on one or on both of the barrel and the plunger, is desirable.

There are additional considerations to be taken into account when manufacturing a prefilled syringe. Prefilled syringes are commonly prepared and sold so the syringe does not need to be filled before use. The syringe can be prefilled with saline solution, a dye for injection, or a pharmaceutically active preparation, for some examples.

Commonly, the prefilled syringe is capped at the distal end, as with a cap, and is closed at the proximal end by its drawn plunger. The prefilled syringe can be wrapped in a sterile package before use. To use the prefilled syringe, the packaging and cap are removed, optionally a hypodermic needle or another delivery conduit is attached to the distal end of the barrel, the delivery conduit or syringe is moved to a use position (such as by inserting the hypodermic needle into a patient's blood vessel or into apparatus to be rinsed with the contents of the syringe), and the plunger is advanced in the barrel to inject the contents of the barrel.

One important consideration in manufacturing pre-filled syringes is that the contents of the syringe desirably will have a substantial shelf life, during which it is important to isolate the material filling the syringe from the barrel wall containing it, to avoid leaching material from the barrel into the prefilled contents or vice versa.

Since many of these vessels are inexpensive and used in large quantities, for certain applications it will be useful to reliably obtain the necessary shelf life without increasing the manufacturing cost to a prohibitive level. For decades, most parenteral therapeutics have been delivered to end users in Type I medical grade borosilicate glass containers such as vials or pre-filled syringes. The relatively strong, impermeable and inert surface of borosilicate glass has performed adequately for most drug products. However, the recent advent of costly, complex and sensitive biologics as well as such advanced delivery systems as auto injectors has exposed glass' physical and chemical shortcomings including possible contamination from metals and breakage, among other problems. Moreover, glass contains several components which can leach out during storage and cause damage to the stored material. In more detail, borosilicate vessels exhibit a number of drawbacks:

- Glass is manufactured from sand containing a heterogeneous mixture of many elements (silicon, oxygen, boron, aluminum, sodium, calcium) with trace levels of other alkali and earth metals. Type I borosilicate glass consists of approximately 76% $SiO_2$, 10.5% $B_2O_3$, 5% $Al_2O_3$, 7% $Na_2O$ and 1.5% CaO and often contains trace metals such as iron, magnesium, zinc, copper and others. The heterogeneous nature of borosilicate glass creates a non-uniform surface chemistry at the molecular level. Glass forming processes used to create glass containers expose some portions of the containers to temperatures as great as 1200°C. Under such high temperatures alkali ions migrate to the local surface and form oxides. The presence of ions extracted from borosilicate glass devices may be involved in degradation, aggregation and denaturation of some biologics. Many proteins and other biologics must be lyophilized (freeze dried), because they are not sufficiently stable in solution in glass vials or syringes.
- In glass syringes, silicon oil is typically used as a lubricant to allow the plunger to slide in the barrel. Silicon oil has been implicated in the precipitation of protein solutions such as insulin and some other biologics. Additionally, the silicon oil coating is often non-uniform, resulting in syringe failures in the market.
- Glass vessels are prone to breakage or degradation during manufacture, filling operations, shipping and use, which

means that glass particulates may enter the drug. The presence of glass particles has led to many FDA Warning Letters and to product recalls.
- Glass-forming processes do not yield the tight dimensional tolerances required for some of the newer auto-injectors and delivery systems.

As a result, some companies have turned to plastic vessels, which provide greater dimensional tolerance and less breakage than glass but lack its impermeability.
Although plastic is superior to glass with respect to breakage, dimensional tolerances and surface uniformity, plastic's use for primary pharmaceutical packaging remains limited due to the following shortcomings:

- Surface characteristics: Plastics suitable for pre-syringes and vials generally exhibit hydrophobic surfaces, which often reduce the stability of the biologic drug contained in the device.
- Gas (oxygen) permeability: Plastic allows small molecule gases to permeate into (or out of) the device. Plastics' permeability to gases is significantly greater than that of glass and, in many cases (as with oxygen-sensitive drugs such as epinephrine), plastics are unacceptable for that reason.
- Water vapor transmission: Plastics allow water vapors to pass through devices to a greater degree than glass. This can be detrimental to the shelf life of a solid (lyophilized) drug. Alternatively, a liquid product may lose water in an arid environment.
- Leachables and extractables: Plastic vessels contain organic compounds that can leach out or be extracted into the drug product. These compounds can contaminate the drug and/or negatively impact the drug's stability.

Clearly, while plastic and glass vessels each offer certain advantages in pharmaceutical primary packaging, neither is optimal for all drugs, biologics or other therapeutics. Thus, there is a desire for plastic syringes, with gas and solute barrier properties which approach the properties of glass. Moreover, there is a need for plastic syringes with sufficient lubricity properties and a lubricity coating which is compatible with the syringe contents.
A non-exhaustive list of patents of possible relevance includes US Patents 6,068,884 and 4,844,986 and U.S. Published Applications 20060046006 and 20040267194.
EP 0 329 041 A2 describes an organosilicon-based layer applied by PECVD to provide a primer coating that does not possess lubricity. Conventional silicone oil is applied over the primer coating to provide lubiricity.
US 5,494,712 and US 5,679,413 describe an abrasion-protective coating made by PECVD using an organosilicon precursor,

SUMMARY OF THE INVENTION

The present invention pertains to a method for coating plastic syringes with thin PECVD coatings made from organosilicon precursors. The resulting syringes offer the superior barrier properties of glass and the dimensional tolerances and breakage resistance of plastics, yet eliminate the drawbacks of both materials. With designed modifications to the PECVD process, the surface chemistry of the coating can be predictably varied. In particular, a plasma coating (SiOxCyHz) is provided which improves lubricity ("lubricity coating"), thus eliminating the need for traditional silicon oil lubricants e.g. in syringes.
The subject-matter of the present invention is defined by the claims attached to this description. The present invention pertains to a method for preparing a lubricity coating on at least a part of the interior surface of a plastic (in particular, COC) syringe. The syringe is coated with a $Si_wO_xC_yH_z$ coating providing lubricity to the syringe interior, thus eliminating the extractables from traditional silicon oil. The lubricity coating can be on the syringe barrel, the plunger (or one of its parts, e.g. the side walls of the piston), or both. Such syringe can also in addition have a $SiO_x$ barrier coating made by PECVD as described herein. A very particular embodiment is a method for making a syringe having a cyclic olefin copolymer (COC) barrel, a $SiO_x$ barrier layer on the inner wall of said barrel, and a lubricity layer on said barrier layer. A SiOx barrier coating typically is 20 to 30 nm thick.
The coatings described herein are glass-like, but do not contain other elements such as boron, sodium, calcium, aluminum and impurities found in glass.
The coatings have a surface free of deleterious elements and impurities found in Type I medical grade borosilicate glass. The coating is deposited on a plastic substrate from plasma, which utilizes organosilicons, creating a uniform layer.
A vessel processing system for coating of a vessel, the system comprising a processing station arrangement configured for performing the above and/or below mentioned method steps is also described. Examples of such processing stations 5501-5504 are depicted in Fig. 12-14.
In the following, coating methods and coated devices which are made by these methods are described. The methods can be carried out on the equipment (vessel processing system and vessel holder) which is also described below.

**PECVD Coating Method**

The present invention pertains to a method of preparing a coating by plasma enhanced chemical vapor deposition treatment (PECVD), namely a method of coating the interior surface of a syringe.
An interior syringe surface is provided, as is a reaction mixture comprising an organosilicon compound gas, $O_2$, optionally a hydrocarbon gas, and optionally a carrier gas. For preparing a lubricity coating, a mixture of an organosilicon precursor (e.g. OMCTS), Oxygen and Argon is preferred.
The surface is contacted with the reaction mixture. Plasma is formed in the reaction mixture. The coating is deposited on at least a portion of the surface, e.g. a portion of the syringe interior wall.
The method is carried out as follows.
A precursor is provided. Said precursor is an organosilicon compound (in the following also designated as "organosilicon precursor"), preferably an organosilicon compound selected from the group consisting of a linear siloxane, a monocyclic siloxane, a polycyclic siloxane, a polysilsesquioxane, an alkyl trimethoxysilane, an aza analogue of any of these precursors (i.e. a linear siloxazane, a monocyclic siloxazane, a polycyclic siloxazane, a polysilsesquioxazane), and a combination of any two or more of these precursors. The precursor is applied to a substrate under conditions effective to form a coating by PECVD. The precursor is thus polymerized, crosslinked, partially or fully oxidized, or any combination of these.
In the method of the present invention, the coating is a lubricity coating, i.e. it forms a surface having a lower frictional resistance than the uncoated substrate.
A lubricity coating of the present invention may also be a passivating coating. Such passivating coating, for example a hydrophobic coating results, e.g., in a lower precipitation of components of a composition in contact with the coated surface. Such hydrophobic coating is characterized by a lower wetting tension than its uncoated counterpart.
In a further aspect of the invention, the coated syringe may also comprise a barrier coating, for example an $SiO_x$ coating. Typically, the barrier is against a gas or liquid, preferably against water vapor, oxygen and/or air.
The method of the invention may comprise the application of one or more coatings made by PECVD from the same or different organosilicon precursors under the same or different reaction conditions. E.g. a syringe may first be coated with an $SiO_x$ barrier coating using HMDSO as organosilicon precursor, and subsequently with a lubricity coating using OMCTS as organosilicon precursor.

**Lubricity Coating**

In its main aspect, the present invention provides a method for preparing a lubricity coating on an interior surface of a syringe.
This coating is advantageously made by the PECVD method and using the precursors as described above. A preferred precursor for the lubricity coating is a monocyclic siloxane, for example octamethylcyclotetrasiloxane (OMCTS).
The present invention provides a method for preparing a lubricity coating on a plastic substrate surface, wherein the substrate which is coated is at least a part of the interior surface of a syringe comprising a barrel having an inner surface, a piston or plunger having an outer surface engaging the inner surface of the barrel, wherein at least one of said inner surface and outer surface is coated, the method comprising the steps:

(a) providing a gas comprising an organosilicon precursor and $O_2$ and optionally a noble gas (e.g. Argon) in the vicinity of the substrate surface, wherein $O_2$ is present in a volume-volume ratio to the organosilicon precursor of from 0.01:1 to 5:1; and
(b) generating a plasma from the gas with an electric power of from 0.1 to 25 W for a deposition time from 1 to 30 sec, thus forming a lubricity coating on the substrate surface by plasma enhanced chemical vapor deposition (PECVD).

The lubricity characteristics of the coating are set by setting the ratio of the $O_2$ to the organosilicon precursor in the gaseous reactant, and/or by setting the electric power used for generating the plasma, and/or by setting the ratio of the noble gas to the organosilicon precursor.
The resulting coated surface has a lower frictional resistance than the untreated substrate. The coated surface is the inside of a syringe barrel and/or a syringe plunger, and the lubricity coating is effective to provide a breakout force or plunger sliding force, or both, that is less than the corresponding force required in the absence of the lubricating coating.
The article coated with the lubricity coating may be a syringe barrel having the lubricating coating on the interior wall, or a syringe plunger having said coating on the syringe contacting surface.
The lubricity coating typically has a formula $Si_wO_xC_yH_z$. It generally has an atomic ratio $Si_wO_xC_y$ wherein w is 1, x is from about 0.5 to about 2.4, y is from about 0.6 to about 3, preferably w is 1, x is from about 0.5 to 1.5, and y is from 0.9 to 2.o, more preferably w is 1, x is from 0.7 to 1.2 and y is from 0.9 to 2.0. The atomic ratio can be determined

by XPS (X-ray photoelectron spectroscopy). Taking into account the H atoms, the lubricity coating may thus in one aspect have the formula $Si_wO_xC_yH_z$, for example where w is 1, x is from about 0.5 to about 2.4, y is from about 0.6 to about 3, and z is from about 2 to about 9. Typically, the atomic ratios are Si 100 : O 80-110 : C 100-150 in a particular lubricity coating made by the method of present invention. Specifically, the atomic ratio may be Si 100 : O 92-107 : C 116-133, and such lubricity coating would hence contain 36% to 41% carbon normalized to 100% carbon plus oxygen plus silicon.

**Passivating, for example Hydrophobic Coating**

The passivating coating according also described herein is for example a hydrophobic coating.

A preferred precursor for the passivating, for example the hydrophobic coating is a linear siloxane, for example hexamethyldisiloxane (HMDSO).

A passivating coating as described herein prevents or reduces mechanical and/or chemical effects of the uncoated surface on a compound or composition contained in the vessel. For example, precipitation and/or clotting or platelet activation of a compound or component of a composition in contact with the surface are prevented or reduced, e.g. blood clotting or platelet activation or precipitation of insulin, or wetting of the uncoated surface by an aqueous fluid is prevented.

A particular hydrophobic coating has the formula $Si_wO_xC_yH_z$. It generally has an atomic ratio $Si_wO_xC_y$ wherein w is 1, x is from about 0.5 to about 2.4, y is from about 0.6 to about 3, preferably w is 1, x is from about 0.5 to 1.5, and y is from 0.9 to 2.0, more preferably w is 1, x is from 0.7 to 1.2 and y is from 0.9 to 2.0. The atomic ratio can be determined by XPS (X-ray photoelectron spectroscopy). Taking into account the H atoms, the hydrophobic coating may thus in one aspect have the formula $Si_wO_xC_yH_z$, for example where w is 1, x is from about 0.5 to about 2.4, y is from about 0.6 to about 3, and z is from about 2 to about 9. Typically, the atomic ratios are Si 100 : O 80-110 : C 100-150 in a particular hydrophobic coating. Specifically, the atomic ratio may be Si 100 : O 92-107 : C 116-133, and such coating would hence contain 36% to 41% carbon normalized to 100% carbon plus oxygen plus silicon.

**Coating of a Vessel**

When a vessel is coated by the above coating method using PECVD, the coating method comprises several steps. A vessel is provided having an open end, a closed end, and an interior surface. At least one gaseous reactant is introduced within the vessel. Plasma is formed within the vessel under conditions effective to form a reaction product of the reactant, i.e. a coating, on the interior surface of the vessel.

Preferably, the method is performed by seating the open end of the vessel on a vessel holder as described herein, establishing a sealed communication between the vessel holder and the interior of the vessel. In this preferred aspect, the gaseous reactant is introduced into the vessel through the vessel holder. In a particularly preferred aspect of the invention, a plasma enhanced chemical vapor deposition (PECVD) apparatus comprising a vessel holder, an inner electrode, an outer electrode, and a power supply is used for the coating method according to the present invention.

The vessel holder has a port to receive a vessel in a seated position for processing. The inner electrode is positioned to be received within a vessel seated on a vessel holder. The outer electrode has an interior portion positioned to receive a vessel seated on the vessel holder. The power supply feeds alternating current to the inner and/or outer electrodes to form a plasma within the vessel seated on the vessel holder. Typically, the power supply feeds alternating current to the outer electrode while the inner electrode is grounded. In this embodiment, the vessel defines the plasma reaction chamber.

In a particular aspect, the PECVD apparatus as described in the preceding paragraphs comprises a gas drain, not necessarily including a source of vacuum, to transfer gas to or from the interior of a vessel seated on the port to define a closed chamber.

In a further particular aspect, the PECVD apparatus includes a vessel holder, a first gripper, a seat on the vessel holder, a reactant supply, a plasma generator, and a vessel release.

The vessel holder is configured for seating to the open end of a vessel. The first gripper is configured for selectively holding and releasing the closed end of a vessel and, while gripping the closed end of the vessel, transporting the vessel to the vicinity of the vessel holder. The vessel holder has a seat configured for establishing sealed communication between the vessel holder and the interior space of the first vessel.

The reactant supply is operatively connected for introducing at least one gaseous reactant within the first vessel through the vessel holder. The plasma generator is configured for forming plasma within the first vessel under conditions effective to form a reaction product of the reactant on the interior surface of the first vessel.

The vessel release is provided for unseating the first vessel from the vessel holder. A gripper which is the first gripper or another gripper is configured for axially transporting the first vessel away from the vessel holder and then releasing the first vessel.

In a particular aspect of the invention, the method is for coating an inner surface of a restricted opening of a syringe, for example a generally tubular syringe, by PECVD. The syringe includes an outer surface, an inner surface defining a lumen, a larger opening having an inner diameter, and a restricted opening that is defined by an inner surface and has an inner diameter smaller than the larger opening inner diameter. A processing vessel is provided having a lumen and a processing vessel opening. The processing vessel opening is connected with the restricted opening of the syringe to establish communication between the lumen of the syringe to be processed and the processing vessel lumen via the restricted opening. At least a partial vacuum is drawn within the lumen of the syringe to be processed and the processing vessel lumen. A PECVD reactant is flowed through the first opening, then through the lumen of the syringe to be processed, then through the restricted opening, then into the processing vessel lumen. Plasma is generated adjacent to the restricted opening under conditions effective to deposit a coating of a PECVD reaction product on the inner surface of the restricted opening.

**COATED VESSEL AND VESSEL PARTS**

The surface coated with the coating, e.g. the syringe wall or a part thereof, is a polymer, preferably a thermoplastic polymer, more preferably a polymer selected from the group consisting of a polycarbonate, an olefin polymer, a cyclic olefin copolymer and a polyester. For example, it is a cyclic olefin copolymer (COC), a polyethylene terephthalate or a polypropylene. For syringe barrels, COC is particularly considered.

In a particular aspect of the invention, the syringe wall has an interior polymer layer enclosed by at least one exterior polymer layer. The polymers may be same or different. E.g., one of the polymer layers is a cyclic olefin copolymer (COC) resin (e.g., defining a water vapor barrier), another polymer layer is a layer of a polyester resin. Such syringe may be made by a process including introducing COC and polyester resin layers into an injection mold through concentric injection nozzles.

The method according to the invention results in a syringe including a plunger, a syringe barrel, and a lubricity coating as defined above on either one or both of these syringe parts, preferably on the inside wall of the syringe barrel. The syringe barrel includes a barrel having an interior surface slidably receiving the plunger. The lubricity coating may be disposed on the interior surface of the syringe barrel, or on the plunger surface contacting the barrel, or on both said surfaces. The lubricity coating is effective to reduce the breakout force or the plunger sliding force necessary to move the plunger within the barrel.

The syringe barrel is advantageously made of thermoplastic material. A lubricity coating is applied to the barrel interior surface, the plunger, or both, by plasma-enhanced chemical vapor deposition (PECVD). In a specific aspect, a solute retainer is applied over the lubricity coating by surface treatment, e.g. in an amount effective to reduce a leaching of the lubricity coating, the thermoplastic material, or both into the lumen. The lubricity coating and solute retainer are composed, and present in relative amounts, effective to provide a breakout force, plunger sliding force, or both that is less than the corresponding force required in the absence of the lubricity coating and solute retainer.

Still another syringe which can be made by the method of the present invention is a syringe including a plunger, syringe barrel, and interior and exterior coatings. The barrel has an interior surface slidably receiving the plunger and an exterior surface. A lubricity coating is on the interior surface, and an additional barrier coating of $SiO_X$, in which x is from about 1.5 to about 2.9, may be provided on the interior surface of the barrel. A barrier coating, e.g. of a resin or of a further $SiO_x$ coating, may additionally be provided on the exterior surface of the barrel.

In another aspect of the invention the syringe is a syringe including a plunger, a syringe barrel, and a staked needle (a "staked needle syringe"). The needle is hollow with a typical size ranging from 18-29 gauge. The syringe barrel has an interior surface slidably receiving the plunger. The staked needle may be affixed to the syringe during the injection molding of the syringe or may be assembled to the formed syringe using an adhesive. A cover is placed over the staked needle to seal the syringe assembly. The syringe assembly must be sealed so that a vacuum can be maintained within the syringe to enable the PECVD coating process. Such syringes with staked needles are described in U.S. Provisional Application No. 61/359,434, filed on June 24, 2010.

In another aspect of the invention the syringe is a syringe including a plunger, a syringe barrel, and a Luer fitting. The syringe barrel has an interior surface slidably receiving the plunger. The Luer fitting includes a Luer taper having an internal passage defined by an internal surface. The Luer fitting is formed as a separate piece from the syringe barrel and joined to the syringe barrel by a coupling. The internal passage of the Luer taper has a barrier coating of $SiO_X$, in which x is from about 1.5 to about 2.9.

In one aspect of the invention, the plunger for a syringe includes a piston and a push rod. The piston has a front face, a generally cylindrical side face, and a back portion, the side face being configured to movably seat within a syringe barrel. The plunger has a lubricity coating according to the present invention on its side face. The push rod engages the back portion of the piston and is configured for advancing the piston in a syringe barrel. The plunger may additionally comprise a $SiO_x$ coating.

**(Pre)filled Coated Syringe**

A coated syringe as described above may be prefilled or used for being filled with a compound or composition in its lumen. Said compound or composition may be

(i) a biologically active compound or composition, preferably a medicament, more preferably insulin or a composition comprising insulin; or
(ii) a biological fluid, preferably a bodily fluid, more preferably blood or a blood fraction (e.g. blood cells); or
(iii) a compound or composition for combination with another compound or composition directly in the syringe, e.g. a compound for the prevention of blood clotting or platelet activation, like citrate or a citrate containing composition.

Generally, the coated syringe made by the method of the present invention is particularly useful for collecting or storing a compound or composition which is sensitive to mechanical and/or chemical effects of the surface of the uncoated syringe material, preferably for preventing or reducing precipitation and/or clotting or platelet activation of a compound or a component of the composition in contact with the interior surface of the syringe.
A syringe made by the method of the present invention may be prefilled, e.g. prefilled with a medicament, a diagnostic compound or composition, or any other biologically of chemically active compound or composition which is intended to be dispensed using the syringe.
The present invention thus provides the following:

1. A method for preparing a lubricity coating on a plastic substrate, wherein the substrate which is coated is at least a part of the interior surface of a syringe comprising a barrel having an inner surface, a piston or plunger having an outer surface engaging the inner surface of the barrel, wherein at least one of said inner surface and outer surface is coated, the method comprising the steps

(a) providing a gas comprising an organosilicon precursor and $O_2$, and optionally a noble gas, in the vicinity of the substrate surface, wherein $O_2$ is present in a volume-volume ratio to the organosilicon precursor of from 0.01:1 to 5:1; and (b) generating a plasma in the gas with an electric power of from 0.1 to 25 W for a deposition time from 1 to 30 sec, thus forming a lubricity coating on the substrate surface by plasma enhanced chemical vapor deposition (PECVD).

2. The method of (1), wherein the organosilicon precursor is a monocyclic siloxane, preferably is OMCTS.

3. The method according to any one of (1) to (2), wherein $O_2$ is present, preferably in a volume-volume ratio to the organosilicon precursor of from 0.01:1 to 0.5:1.

4. The method according to any one of (1) to (3), wherein Ar is present as the noble gas.

5. The method according to any of the preceding, wherein the gas comprises from 1 to 6 standard volumes of the organosilicon precursor, from 1 to 100 standard volumes of the noble gas, and from 0.1 to 2 standard volumes of $O_2$.

6. The method according to any one of the preceding wherein the plasma is generated with an electric power of from 2 to 4 W; and/or

(ii) wherein the ratio of the electrode power to the plasma volume is less than 10 W/ml, preferably from 6 W/ml to 0.1 W/ml.

7. The method according to any one of the preceding, wherein the resulting coating has a roughness when determined by AFM and expressed as RMS of from more than 0 to 25 nm, preferably from 7 to 20 nm, optionally from 10 to 20 nm, optionally from 13 to 17 nm, optionally from 13 to 15 nm.

8. The method according to any one of the preceding, additionally comprising a step for preparing a barrier coating on the substrate before the lubricity coating is applied, the additional step comprising the steps

(a) providing a gas comprising an organosilicon precursor and $O_2$ in the vicinity of the substrate surface; and

(b) generating a plasma from the gas, thus forming a SiOx barrier coating on the substrate surface by plasma enhanced chemical vapor deposition (PECVD).

9. The method according to (8) wherein in the step for preparing a barrier coating

(i) the plasma is generated with electrodes powered with sufficient power to form a SiOx barrier coating on the substrate surface, preferably with electrodes supplied with an electric power of from 8 to 500 W, preferably from 20 to 400 W, more preferably from 35 to 350 W, even more preferably of from 44 to 300 W, most preferably of from 44 to 70 W; and/or

(ii) the ratio of the electrode power to the plasma volume is equal or more than 5 W/ml, preferably is from 6 W/ml to 150 W/ml, more preferably is from 7 W/ml to 100 W/ml, most preferably from 7 W/ml to 20 W/ml; and/or

(iii) the $O_2$ is present in a volume:volume ratio of from 1 : 1 to 100 : 1 in relation to the silicon containing precursor, preferably in a ratio of from 5 : 1 to 30 : 1, more preferably in a ratio of from 10 : 1 to 20 : 1, even more preferably in a ratio of 15 : 1.

10. The method of (8) or (9), wherein the organosilicon precursor for the barrier coating is a linear siloxane, preferably HMDSO.

11. The method according to any one of the preceding, wherein the substrate is a polymer selected from the group consisting of a polycarbonate, an olefin polymer, a cyclic olefin copolymer and a polyester, and preferably is a cyclic olefin copolymer, a polyethylene terephthalate or a polypropylene, and more preferably is COC.

12. The method according to any one of the preceding, wherein the plasma is generated with electrodes powered at a radiofrequency, preferably at 13.56 MHz.

13. The method according to any one of the preceding, wherein the resulting lubricity coating has an atomic ratio SiwOxCy or SiwNxCy wherein w is 1, x is from about 0.5 to about 2.4, y is from about 0.6 to about 3.

14. The method according to any one of the preceding, wherein the lubricity coating has a lower frictional resistance than the uncoated surface, wherein preferably the frictional resistance is reduced by at least 25 %, more preferably by at least 45%, even more preferably by at least 60% in comparison to the uncoated surface.

15. The method according to any one of the preceding, wherein the syringe additionally comprises at least one layer of SiOx, wherein x is from 1.5 to 2.9, wherein

(i) the lubricity coating is situated between the SiOx layer and the substrate surface or vice versa, or wherein

(ii) the lubricity coating is situated between two SiOx layers or vice versa, or wherein

(iii) the layers of SiOx and the lubricity coating are a graded composite of SiwOxCyHz to SiOx or vice versa.

16. The method according to (15), wherein the SiOx barrier coating has a thickness of from 20 to 30 nm and the lubricity coating has an average thickness of from 1 to 5000 nm, preferably of from 30 to 1000 nm, more preferably of from 80 to 150 nm.

17. The method according to any one of the preceding, wherein the lubricity coating

(i) has a lower wetting tension than the uncoated surface, preferably a wetting tension of from 20 to 72 dyne/cm, more preferably a wetting tension of from 30 to 60 dynes/cm, more preferably a wetting tension of from 30 to 40 dynes/cm, preferably 34 dyne/cm; and/or

(iv) is more hydrophobic than the uncoated surface.

18. The method according to any one of the preceding, wherein the plunger initation force Fi is from 2.5 to 5 lbs and the plunger maintenance force Fm is from 2.5 to 8 lbs.

19. The method according to any one of the preceding, wherein the lubricity coating has an average thickness of from 10 to 1000 nm.

20. The method according to any one of the preceding, wherein the plastic substrate is COC, wherein the gas in step (a) comprises octamethylcyclotetrasiloxane, $O_2$ and Ar, and wherein the power for generating the plasma is from 6 W/ml to 0.1 W/ml in relation to the volume of the syringe lumen.

A particular syringe barrel which may form part of a syringe is made according to the method of (1), wherein the plastic substrate is COC, wherein the gas in step (a) comprises octamethylcyclotetrasiloxane, $O_2$ and Ar, and wherein preferably the power for generating the plasma is from 6 W/ml to 0.1 W/ml, in a particular aspect from 0.8 to 1.3 W/ml in relation to the volume of the syringe lumen.

A chemical vapor deposition apparatus for applying a coating to a substrate includes a source of an organosilicon precursor, a source of a carrier gas, and a source of an oxidizing agent. The chemical vapor deposition apparatus still further includes one or more conduits for conveying to the substrate a gaseous reactant or process gas comprising from 1 to 6 standard volumes of the precursor, from 5 to 100 standard volumes of the carrier gas, and from 0.1 to 2 standard volumes of the oxidizing agent. The chemical vapor deposition apparatus further includes a source of microwave or radio frequency energy and an applicator powered by the source of microwave or radio frequency energy for generating plasma in the gaseous reactant or process gas.

A syringe coated by the method of the invention may be part of a medical or diagnostic kit. Optionally, the kit additionally includes a medicament or diagnostic agent which is contained in the coated syringe in contact with the coating; and/or a hypodermic needle, double-ended needle, or other delivery conduit; and/or an instruction sheet.

Additional options for use of a coated syringe made according to the invention include any one or more of the following:

Use for reception and/or storage and/or delivery of a compound or composition.

Use for storing insulin.

Use for storing blood. Optionally, the stored blood is viable for return to the vascular system of a patient.

A lubricity coating prepared by the method according to any described embodiment may additionally serve as a hydrophobic layer or coating that is more hydrophobic than the uncoated surface.

Other aspects of the invention will become apparent to a person of ordinary skill in the art after reviewing the present disclosure and claims.

## BRIEF DESCRIPTION OF THE DRAWINGS

FIG. 1 is a schematic sectional view of a vessel holder in a coating station for use in the present invention.

FIG. 2 is a section taken along section lines A-A of FIG. 1.

FIG. 3 shows the drawbacks of silicon oil (or any other oil) as lubricant. Non-uniformity of silicon oil occurs because it is not covalently bound to the surface and flows. A.) Silicon oil is pushed off the syringe barrel wall by the plunger following insertion of the plunger B) Silicon oil is forced out of the area between the plunger and syringe wall leading to high break loose forces. C.) Silicon oil flows over time due to gravitational forces.

FIG. 4 is an exploded longitudinal sectional view of a syringe and cap adapted for use as a prefilled syringe.

FIG. 8 is a view showing the processing of syringe barrels.

FIG. 9 is an enlarged detail view of the processing vessel of FIG. 8.

FIG. 10 is an alternative construction for a vessel holder useful with any embodiment of the invention, for example those of the other Figures.

FIG. 11 is a schematic view of an assembly for treating vessels. The assembly is usable with the apparatus in any of the preceding figures.

FIG. 12 shows a schematic representation of an exemplary vessel processing system.

FIG. 13 shows a schematic representation of an exemplary vessel processing system.

FIG. 14 shows a processing station of an exemplary vessel processing system.

FIG. 15 shows a portable vessel holder.

FIG. 16 shows a SEM image of Example P. The horizontal edge-to-edge scale is 5 $\mu$m.

FIG. 17 shows a SEM image of Example S. The horizontal edge-to-edge scale is 5 $\mu$m.

FIG. 18A shows the results of the AFM imaging performed on a first portion of Example Q. A 10 $\mu$m x 10 $\mu$m area was imaged. A top down view of the area is shown, together with the topography differences along a first cross section indicated by the line drawn in the top down view. The vertical depth of the features was measured using the cross section tool. Results for the parameters measured, like RMS, Ra and Rmax are indicated in the top right box.

FIG. 18B is similar to FIG. 18A, but shows the results of the AFM imaging performed on Example Q along a second cross section indicated by the line drawn in the top down view.

FIG. 18C is similar to FIG. 18A, but shows the results of the AFM imaging performed on Example Q along a third

cross section indicated by the line drawn in the top down view.

FIG. 19A is similar to FIG. 18A, but shows the results of the AFM imaging performed on Example T along a first cross section indicated by the line drawn in the top down view.

FIG. 19B is similar to FIG. 19A, but shows the results of the AFM imaging performed on Example T along a second cross section indicated by the line drawn in the top down view.

FIG. 19C is similar to FIG. 19A, but shows the results of the AFM imaging performed on Example T along a third cross section indicated by the line drawn in the top down view.

FIG. 20A is similar to FIG. 18A, but shows the results of the AFM imaging performed on Example V along a first cross section indicated by the line drawn in the top down view.

FIG. 20B is similar to FIG. 20A, but shows the results of the AFM imaging performed on Example V along a second cross section indicated by the line drawn in the top down view.

FIG. 20C is similar to FIG. 20A, but shows the results of the AFM imaging performed on Example V along a third cross section indicated by the line drawn in the top down view.

FIG. 21 shows a TEM image of a lubricity coating prepared according to the invention coated on a $SiO_2$ barrier coating, which in turn is coated on a COC substrate.

FIG. 22 shows a TEM image of a $SiO_2$ barrier coating which is coated on a COC substrate.

FIG. 23 shows the meniscus made by water in a.) glass tube, b.) hydrophilic $SiO_2$ coated COC tube, c.) hydrophobic $Si_wO_xC_yH_z$ coated COC tube, and d.) uncoated COC tube. The hydrophilic $SiO_2$ coated tube and borosilicate glass tube have a similar meniscus, demonstrating that the hydrophilicity of the hydrophilic $SiO_2$ coating is comparable to a glass surface. The hydophobic coated tube and the uncoated COC tube each have a meniscus that is expected from a hydrophobic surface.

FIG. 24 is a longitudinal section of a syringe with a staked needle.

FIG. 25 is a longitudinal section of the dispensing end of an alternative syringe with a staked needle.

FIG. 26 is a longitudinal section of an alternative syringe with a staked needle.

FIG. 27 is a diagrammatic view showing a flexible diaphragm 71144 to which the needle is attached.

The following reference characters are used in the drawing figures:

| | |
|---|---|
| 20 | Vessel processing system |
| 28 | Coating station |
| 38 | Vessel holder |
| 50 | Vessel holder |
| 70 | Conveyor |
| 72 | Transfer mechanism (on) |
| 74 | Transfer mechanism (off) |
| 80 | Vessel |
| 82 | Opening |
| 86 | Wall |
| 88 | Interior surface |
| 90 | Barrier layer |
| 92 | Vessel port |
| 94 | Vacuum duct |
| 96 | Vacuum port |
| 98 | Vacuum source |
| 100 | O-ring (of 92) |
| 102 | O-ring (of 96) |
| 104 | Gas inlet port |
| 106 | O-ring (of 100) |
| 108 | Probe (counter electrode) |
| 110 | Gas delivery port (of 108) |
| 114 | Housing (of 50) |
| 116 | Collar |
| 118 | Exterior surface (of 80) |
| 144 | PECVD gas source |
| 152 | Pressure gauge |
| 160 | Electrode |
| 162 | Power supply |
| 164 | Sidewall (of 160) |
| 166 | Sidewall (of 160) |
| 168 | Closed end (of 160) |
| 200 | Electrode |
| 250 | Syringe barrel |
| 252 | Syringe |
| 254 | Interior surface (of 250) |
| 256 | Back end (of 250) |

| | |
|---|---|
| 258 | Plunger (of 252) |
| 260 | Front end (of 250) |
| 262 | Cap |
| 264 | Interior surface (of 262) |
| 290 | Apparatus for coating, for example, 250 |
| 292 | Inner surface (of 294) |
| 294 | Restricted opening (of 250) |
| 296 | Processing vessel |
| 298 | Outer surface (of 250) |
| 300 | Lumen (of 250) |
| 302 | Larger opening (of 250) |
| 304 | Processing vessel lumen |
| 306 | Processing vessel opening |
| 308 | Inner electrode |
| 310 | Interior passage (of 308) |
| 312 | Proximal end (of 308) |
| 314 | Distal end (of 308) |
| 316 | Distal opening (of 308) |
| 318 | Plasma |
| 332 | First fitting (male Luer taper) |
| 334 | Second fitting (female Luer taper) |
| 336 | Locking collar (of 332) |
| 338 | First abutment (of 332) |
| 340 | Second abutment (of 332) |
| 342 | O-ring |
| 344 | Dog |
| 482 | Vessel holder body |
| 484 | Upper portion (of 482) |
| 486 | Base portion (of 482) |
| 488 | Joint (between 484 and 486) |
| 490 | O-ring |
| 492 | Annular pocket |
| 494 | Radially extending abutment surface |
| 496 | Radially extending wall |

| | |
|---|---|
| 498 | Screw |
| 500 | Screw |
| 502 | Vessel port |
| 504 | Second O-ring |
| 506 | Inner diameter (of 490) |
| 508 | Vacuum duct (of 482) |
| 574 | Main vacuum valve |
| 576 | Vacuum line |
| 578 | Manual bypass valve |
| 580 | Bypass line |
| 582 | Vent valve |
| 584 | Main reactant gas valve |
| 586 | Main reactant feed line |
| 588 | Organosilicon liquid reservoir |
| 590 | Organosilicon feed line (capillary) |
| 592 | Organosilicon shut-off valve |
| 594 | Oxygen tank |
| 596 | Oxygen feed line |
| 598 | Mass flow controller |
| 600 | Oxygen shut-off valve |
| 614 | Headspace |
| 616 | Pressure source |
| 618 | Pressure line |
| 620 | Capillary connection |
| 5501 | First processing station |
| 5502 | Second processing station |
| 5503 | Third processing station |
| 5504 | Fourth processing station |
| 5505 | Processor |
| 5506 | User interface |
| 5507 | Bus |
| 5701 | PECVD apparatus |
| 5702 | First detector |
| 5703 | Second detector |
| 5704 | Detector |
| 5705 | Detector |

| | |
|---|---|
| 5706 | Detector |
| 5707 | Detector |
| 7001 | Conveyor exit branch |
| 7002 | Conveyor exit branch |
| 7003 | Conveyor exit branch |
| 7004 | Conveyor exit branch |
| 7120 | Syringe |
| 7122 | Needle |
| 7124 | Barrel |
| 7126 | Cap |
| 7128 | Barrier coating |
| 7130 | Lubricity coating |
| 7132 | Outside surface |
| 7134 | Delivery outlet |
| 7136 | Base (of 7122) |
| 7138 | Internal passage |
| 7140 | Generally cylindrical interior surface portion |
| 7142 | Generally hemispherical interior surface portion |
| 7144 | Front passage |
| 7146 | Lumen |
| 7148 | Lumen |
| 7150 | Ambient air |
| 7152 | Rim |
| 7154 | Exterior portion (of 7124) |
| 7156 | Opening |
| 7158 | Fluid |
| 7160 | Material (of 7124) |
| 7164 | Non-cylindrical portion (of 7122) |
| 7166 | Plunger |
| 7168 | Base |
| 7170 | Coupling |
| 7172 | Flexible lip seal |
| 7174 | Detent |
| 7176 | Projection |
| 7196 | Internal portion (of 7126) |
| 7198 | External portion (of 7126) |

| | |
|---|---|
| 71106 | Rear passage (of barrel) |
| 71110 | Tapered nose (of 7120) |
| 71112 | Tapered throat (of 7126) |
| 71114 | Collar (of syringe) |
| 71116 | Interior thread (of 71114) |
| 71118 | Dog (of 26) |
| 71120 | Dog (of 26) |
| 71122 | Syringe barrel |
| 71124 | Syringe cap |
| 71126 | (Syringe cap (flexible) |
| 71128 | Cap-syringe interface |
| 71130 | Syringe barrel |
| 71134 | Delivery outlet |
| 71136 | Base (of 71122) |
| 71140 | Finger grip |
| 71144 | Flexible diaphragm |

DETAILED DESCRIPTION

The present invention, which has the full scope indicated by the language of the claims, will now be described more fully, *inter alia* with reference to the accompanying drawings, in which several embodiments are shown. Like numbers refer to like or corresponding elements throughout. The following disclosure relates to all embodiments unless specifically limited to a certain embodiment.

DEFINITION SECTION

In the context of the present invention, the following definitions and abbreviations are used:

RF is radio frequency; sccm is standard cubic centimeters per minute.

The term "at least" in the context of the present invention means "equal or more" than the integer following the term. The word "comprising" does not exclude other elements or steps, and the indefinite article "a" or "an" does not exclude a plurality unless indicated otherwise. Whenever a parameter range is indicated, it is intended to disclose the parameter values given as limits of the range and all values of the parameter falling within said range.

"First" and "second" or similar references to, e.g., processing stations or processing devices refer to the minimum number of processing stations or devices that are present, but do not necessarily represent the order or total number of processing stations and devices. These terms do not limit the number of processing stations or the particular processing carried out at the respective stations.

For purposes of the present invention, an "organosilicon precursor" is a compound having at least one of the linkage:

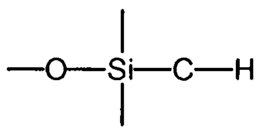

or

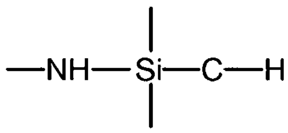

which is a tetravalent silicon atom connected to an oxygen or nitrogen atom and an organic carbon atom (an organic carbon atom being a carbon atom bonded to at least one hydrogen atom). A volatile organosilicon precursor, defined as such a precursor that can be supplied as a vapor in a PECVD apparatus, is an optional organosilicon precursor. Optionally, the organosilicon precursor is selected from the group consisting of a linear siloxane, a monocyclic siloxane, a polycyclic siloxane, a polysilsesquioxane, an alkyl trimethoxysilane, a linear silazane, a monocyclic silazane, a polycyclic silazane, a polysilsesquiazane, and a combination of any two or more of these precursors.

The feed amounts of PECVD precursors, gaseous reactant or process gases, and carrier gas are sometimes expressed in "standard volumes" in the specification. The standard volume of a charge or other fixed amount of gas is the volume the fixed amount of the gas would occupy at a standard temperature and pressure (without regard to the actual temperature and pressure of delivery). Standard volumes can be measured using different units of volume, and still be within the scope of the present disclosure. For example, the same fixed amount of gas could be expressed as the number of standard cubic centimeters, the number of standard cubic meters, or the number of standard cubic feet. Standard volumes can also be defined using different standard temperatures and pressures, and still be within the scope of the present disclosure. For example, the standard temperature might be 0°C and the standard pressure might be 760 Torr (as is conventional), or the standard temperature might be 20°C and the standard pressure might be 1 Torr. But whatever standard is used in a given case, when comparing relative amounts of two or more different gases without specifying particular parameters, the same units of volume, standard temperature, and standard pressure are to be used relative to each gas, unless otherwise indicated.

The corresponding feed rates of PECVD precursors, gaseous reactant or process gases, and carrier gas are expressed in standard volumes per unit of time in the specification. For example, in the working examples the flow rates are expressed as standard cubic centimeters per minute, abbreviated as sccm. As with the other parameters, other units of time can be used, such as seconds or hours, but consistent parameters are to be used when comparing the flow rates of two or more gases, unless otherwise indicated.

A "vessel" in the context of the present invention can be any type of vessel with at least one opening and a wall defining an interior surface. Though the invention is not necessarily limited to vessels of a particular volume, vessels are contemplated in which the lumen has a void volume of from 0.5 to 50 mL, optionally from 1 to 10 mL, optionally from 0.5 to 5 mL, optionally from 1 to 3 mL.

The term "at least" in the context of the present invention means "equal or more" than the integer following the term. Thus, a vessel in the context of the present invention has one or more openings. One or two openings, like the openings of a sample tube (one opening) or a syringe barrel (two openings) are preferred. If the vessel has two openings, they can be of same or different size. If there is more than one opening, one opening can be used for the gas inlet for a PECVD coating method according to the present invention, while the other openings are either capped or open.

A vessel can be of any shape, a vessel having a substantially cylindrical wall adjacent to at least one of its open ends being preferred. Generally, the interior wall of the vessel is cylindrically shaped, like, e.g. in a syringe barrel.

A "hydrophobic layer" in the context of the present invention means that the coating lowers the wetting tension of a surface coated with the coating, compared to the corresponding uncoated surface. Hydrophobicity is thus a function of both the uncoated substrate and the coating. The same applies with appropriate alterations for other contexts wherein the term "hydrophobic" is used. The term "hydrophilic" means the opposite, i.e. that the wetting tension is increased compared to reference sample. The present hydrophobic layers are primarily defined by their hydrophobicity and the process conditions providing hydrophobicity, and optionally can have a composition according to the empirical composition or sum formula $Si_wO_xC_yH_z$. It generally has an atomic ratio $Si_wO_xC_y$ wherein w is 1, x is from about 0.5 to about 2.4, y is from about 0.6 to about 3, preferably w is 1, x is from about 0.5 to 1.5, and y is from 0.9 to 2.0, more preferably w is 1, x is from 0.7 to 1.2 and y is from 0.9 to 2.0. The atomic ratio can be determined by XPS (X-ray photoelectron spectroscopy). Taking into account the H atoms, the coating may thus in one aspect have the formula $Si_wO_xC_yH_z$, for example where w is 1, x is from about 0.5 to about 2.4, y is from about 0.6 to about 3, and z is from about 2 to about 9. Typically, the atomic ratios are Si 100 : O 80-110 : C 100-150 in a particular coating of present invention. Specifically, the atomic ratio may be Si 100 : O 92-107 : C 116-133, and such coating would hence contain 36% to 41% carbon normalized to 100% carbon plus oxygen plus silicon.

These values of w, x, y, and z are applicable to the empirical composition $Si_wO_xC_yH_z$ throughout this specification. The values of w, x, y, and z used throughout this specification should be understood as ratios or an empirical formula (e.g. for a coating), rather than as a limit on the number or type of atoms in a molecule. For example, octamethylcyclotetrasiloxane, which has the molecular composition $Si_4O_4C_8H_{24}$, can be described by the following empirical

formula, arrived at by dividing each of w, x, y, and z in the molecular formula by 4, the largest common factor: $Si_1O_1C_2H_6$. The values of w, x, y, and z are also not limited to integers. For example, (acyclic) octamethyltrisiloxane, molecular composition $Si_3O_2C_8H_{24}$, is reducible to $Si_1O_{0.67}C_{2.67}H_8$.

"Wetting tension" is a specific measure for the hydrophobicity or hydrophilicity of a surface. An optional wetting tension measurement method in the context of the present invention is ASTM D 2578 or a modification of the method described in ASTM D 2578. This method uses standard wetting tension solutions (called dyne solutions) to determine the solution that comes nearest to wetting a plastic film surface for exactly two seconds. This is the film's wetting tension. The procedure utilized is varied herein from ASTM D 2578 in that the substrates are not flat plastic films, but are tubular (syringes).

A "lubricity layer" in the context of the present invention is a coating which has a lower frictional resistance than the uncoated surface. In other words, it reduces the frictional resistance of the coated surface in comparison to a reference surface that is uncoated. The present lubricity layers are primarily defined by their lower frictional resistance than the uncoated surface and the process conditions providing lower frictional resistance than the uncoated surface, and optionally can have a composition according to the empirical composition $Si_wO_xC_yH_z$, as defined herein. It generally has an atomic ratio $Si_wO_xC_y$ wherein w is 1, x is from about 0.5 to about 2.4, y is from about 0.6 to about 3, preferably w is 1, x is from about 0.5 to 1.5, and y is from 0.9 to 2.0, more preferably w is 1, x is from 0.7 to 1.2 and y is from 0.9 to 2.0. The atomic ratio can be determined by XPS (X-ray photoelectron spectroscopy). Taking into account the H atoms, the coating may thus in one aspect have the formula $Si_wO_xC_yH_z$, for example where w is 1, x is from about 0.5 to about 2.4, y is from about 0.6 to about 3, and z is from about 2 to about 9. Typically, the atomic ratios are Si 100 : O 80-110 : C 100-150 in a particular coating of present invention. Specifically, the atomic ratio may be Si 100 : O 92-107 : C 116-133, and such coating would hence contain 36% to 41% carbon normalized to 100% carbon plus oxygen plus silicon.

"Frictional resistance" can be static frictional resistance and/or kinetic frictional resistance.

For a syringe part, e.g. a syringe barrel or plunger, coated with a lubricity layer the relevant static frictional resistance in the context of the present invention is the breakout force as defined herein, and the relevant kinetic frictional resistance in the context of the present invention is the plunger sliding force as defined herein. For example, the plunger sliding force as defined and determined herein is suitable to determine the presence or absence and the lubricity characteristics of a lubricity layer or coating in the context of the present invention whenever the coating is applied to any syringe or syringe part, for example to the inner wall of a syringe barrel. The breakout force is of particular relevance for evaluation of the coating effect on a prefilled syringe, i.e. a syringe which is filled after coating and can be stored for some time, e.g. several months or even years, before the plunger is moved again (has to be "broken out").

The "plunger sliding force" (synonym to "glide force", "maintenance force", Fm, also used in this description) in the context of the present invention is the force required to maintain movement of a plunger in a syringe barrel, e.g. during aspiration or dispense. It can advantageously be determined using the ISO 7886-1:1993 test described herein and known in the art. A synonym for "plunger sliding force" often used in the art is "plunger force" or "pushing force".

The "plunger breakout force" (synonym to "breakout force", "break loose force", "initation force", Fi, also used in this description) in the context of the present invention is the initial force required to move the plunger in a syringe, for example in a prefilled syringe.

Both "plunger sliding force" and "plunger breakout force" and methods for their measurement are described in more detail in subsequent parts of this description. These two forces can be expressed in N, lbs or kg and all three units are used herein. These units correlate as follows: 1N = 0.102 kg = 0.2248 lbs (pounds).

"Slidably" means that the plunger is permitted to slide in a syringe barrel.

"Substantially rigid" means that the assembled components (ports, duct, and housing, explained further below) can be moved as a unit by handling the housing, without significant displacement of any of the assembled components respecting the others. Specifically, none of the components are connected by hoses or the like that allow substantial relative movement among the parts in normal use. The provision of a substantially rigid relation of these parts allows the location of the vessel seated on the vessel holder to be nearly as well known and precise as the locations of these parts secured to the housing.

DESCRIPTION SECTION

The present invention pertains to a method of applying a coating such as 90 to a syringe 252 (FIG. 4). The method can be used with any disclosed embodiment. The method includes providing a vaporizable organosilicon precursor and applying the precursor to the substrate by chemical vapor deposition. The precursor is applied, for example in the apparatus of FIG. 1, 8, of the EP applications cited in paragraph [002], under conditions effective to form a coating.

A gaseous reactant or process gas can be employed having a standard volume ratio of, when a lubricity coating is prepared:

- from 1 to 6 standard volumes, optionally from 2 to 4 standard volumes, optionally equal to or less than 2.5 standard

volumes, optionally equal to or less than 1.5 standard volumes, optionally equal to or less than 1.25 standard volumes of the precursor;

- from 1 to 100 standard volumes, optionally from 5 to 100 standard volumes, optionally from 10 to 70 standard volumes, of a carrier gas;
- from 0.1 to 2 standard volumes, optionally from 0.2 to 1.5 standard volumes, optionally from 0.2 to 1 standard volumes, optionally from 0.5 to 1.5 standard volumes, optionally from 0.8 to 1.2 standard volumes of $O_2$.

The syringe 252 (FIG. 4) to be coated includes a lumen defined by a surface defining a substrate. The coating is prepared on at least a portion of the substrate by the previously defined process.

A chemical vapor deposition apparatus such as the apparatus 28 illustrated in FIG. 11 may be used for applying the coating to a substrate.

Fig. 12 shows a vessel processing system 20 useful for performing the present invention. The vessel processing system 20 comprises, inter alia, a first processing station 5501 and may or may not also comprise a second processing station 5502.

The first vessel processing station 5501 contains a vessel holder 38 which holds a seated vessel 80. Although Fig. 12 depicts a blood tube 80, the vessel may also be a syringe body. In case of plastic vessels, the first processing station may also comprise a mould for moulding the plastic vessel.

After the first processing at the first processing station (which processing may comprise moulding of the vessel, a first inspection of the vessel for defects, coating of the interior surface of the vessel and a second inspection of the vessel for defects, in particular of the interior coating), the vessel holder 38 may be transported together with the vessel 80 to the second vessel processing station 5502. This transportation is performed by a conveyor arrangement 70, 72, 74. For example, a gripper or several grippers may be provided for gripping the vessel holder 38 and/or the vessel 80 in order to move the vessel/holder combination to the next processing station 5502. Alternatively, only the vessel may be moved without the holder. However, it may be advantageous to move the holder together with the vessel in which case the holder is adapted such that it can be transported by the conveyor arrangement.

Fig. 13 shows another vessel processing system 20. Again, two vessel processing stations 5501, 5502 are provided. Furthermore, additional vessel processing stations 5503, 5504 may be provided which are arranged in series and in which the vessel can be processed, i.e. inspected and/or coated.

A vessel can be moved from a stock to the left processing station 5504. Alternatively, the vessel can be moulded in the first processing station 5504. In any case, a first vessel processing is performed in the processing station 5504, such as a moulding, an inspection and/or a coating, which may be followed by a second inspection. Then, the vessel is moved to the next processing station 5501 via the conveyor arrangement 70, 72, 74. Typically, the vessel is moved together with the vessel holder. A second processing is performed in the second processing station 5501 after which the vessel and holder are moved to the next processing station 5502 in which a third processing is performed. The vessel is then moved (again together with the holder) to the fourth processing station 5503 for a fourth processing, after which it is conveyed to a storage.

Before and after each coating step or moulding step or any other step which manipulates the vessel an inspection of the whole vessel, of part of the vessel and in particular of an interior surface of the vessel may be performed. The result of each inspection can be transferred to a central processing unit 5505 via a data bus 5507. Each processing station is connected to the data bus 5507. The above described program element may run on the processor 5505, and the processor, which may be adapted in form of a central control and regulation unit, controls the system and may also be adapted to process the inspection data, to analyze the data and to determine whether the last processing step was successful.

If it is determined that the last processing step was not successful, because for example the coating comprises holes or because the surface of the coating is determined to be regular or not smooth enough, the vessel does not enter the next processing station but is either removed from the production process (see conveyor sections 7001, 7002, 7003, 7004) or conveyed back in order to become re-processed.

The processor 5505 may be connected to a user interface 5506 for inputting control or regulation parameters.

Fig. 14 shows a vessel processing station 5501. The station comprises a PECVD apparatus 5701 for coating an interior surface of the vessel. Furthermore, several detectors 5702-5707 may be provided for vessel inspection. Such detectors may for example be electrodes for performing electric measurements, optical detectors, like CCD cameras, gas detectors or pressure detectors.

Fig. 15 shows a vessel holder 38, together with several detectors 5702, 5703, 5704 and an electrode with gas inlet port 108, 110.

The electrode and the detector 5702 may be adapted to be moved into the interior space of the vessel 80 when the vessel is seated on the holder 38.

The optical inspection may be particularly performed during a coating step, for example with the help of optical detectors 5703, 5704 which are arranged outside the seated vessel 80 or even with the help of an optical detector 5705 arranged inside the interior space of the vessel 80.

The detectors may comprise colour filters such that different wavelengths can be detected during the coating process. The processing unit 5505 analyzes the optical data and determines whether the coating was successful or not to a predetermined level of certainty. If it is determined that the coating was most probably unsuccessful, the respective vessel is separated from the processing system or re-processed.

Referring now to FIG 11, the chemical vapor deposition apparatus includes a source of an organosilicon precursor such as the reservoir 588, a source of a carrier gas such as 602, and a source of an oxidizing agent such as 594. The chemical vapor deposition apparatus still further includes one or more conduits, such as the conduits 108, 586, 590, 604, and 596, for conveying to the substrate a gaseous reactant or process gas comprising, e.g., from 1 to 6 standard volumes of the precursor, from 5 to 100 standard volumes of the carrier gas, and from 0.1 to 2 standard volumes of the oxidizing agent. The chemical vapor deposition apparatus further includes a source 162 of microwave or radio frequency energy and an applicator or electrode such as 160 powered by the source of microwave or radio frequency energy for generating plasma in the gaseous reactant or process gas.

The syringe coated by the method of the present invention 252 comprises a plunger 258, a barrel 250, and a coating on the interior surface 264. The barrel 250 is a vessel and has an interior surface 264 defining the vessel lumen and receiving the plunger 258 for sliding. The vessel interior surface 264 is a substrate. The coating is a lubricity layer on the substrate 264, the plunger 258, or both, applied by chemical vapor deposition, employing as the gaseous reactant or process gas, e.g., from 1 to 6 standard volumes of an organosilicon precursor, from 5 to 100 standard volumes of a carrier gas, and from 0.1 to 2 standard volumes of $O_2$. In addition to this lubricity coating, the syringe may contain one or more other coatings, e.g. a $SiO_x$ barrier coating as described herein. Said additional coating(s) may be located under or over the lubricity coating, i.e. nearer to the coated substrate or nearer to the lumen of the syringe.

A concern of converting from glass to plastic syringes centers around the potential for leachable materials from plastics. With plasma coating technology, the coating, being derived from non-metal gaseous precursors e.g. HMDSO, will itself contain no trace metals and function as a barrier to inorganic, metals and organic solutes, preventing leaching of these species from the coated substrate into syringe fluids. In addition to leaching control of plastic syringes, the same plasma coating technology offers potential to provide a solute barrier to the plunger tip, typically made of elastomeric plastic compositions containing even higher levels of leachable organic oligomers and catalysts.

Moreover, certain syringes prefilled with synthetic and biological pharmaceutical formulations are very oxygen and moisture sensitive. A critical factor in the conversion from glass to plastic syringe barrels will be the improvement of plastic oxygen and moisture barrier performance. The plasma coating technology is suitable to provide a $SiO_x$ barrier coating for protection against oxygen and moisture.

A plunger 258 for a syringe 252 made by the method of the present inveiont may comprise a piston or tip, a coating, and a push rod. The piston or tip has a front face, a generally cylindrical side face that slides within the barrel 250, comprising a substrate, and a back portion. The side face is configured to movably seat within a syringe barrel. The coating is on the substrate and is a lubricity layer interfacing with the side face. The lubricity layer is produced from the chemical vapor deposition (CVD) process according to the present invention. The push rod engages the back portion of the piston and is configured for advancing the piston in a syringe barrel.

A syringe coated by the method of the invention may be part of a medical or diagnostic kit . Optionally, the kit additionally includes a medicament or diagnostic agent which is contained in the coated syringe in contact with the coating; and/or a hypodermic needle, double-ended needle, or other delivery conduit; and/or an instruction sheet.

Additional options for use of a coated syringe made according to the invention include any one or more of the following:

Use for reception and/or storage and/or delivery of a compound or composition.

Use for storing insulin.

Use for storing blood. Optionally, the stored blood is viable for return to the vascular system of a patient.

A lubricity coating prepared by the method according to any described embodiment may additionally serve as a hydrophobic layer or coating that is more hydrophobic than the uncoated surface.

The following is a more detailed description of the invention. It starts with a general description of the lubricity coating made by the method of present invention, and of a hydrophobic coating, then describes the equipment suitable to prepare the lubricity coating according to the method of the present invention, and subsequently describes the coatings, the coated vessels, and the methods for their production.

## IA. LUBRICITY COATING

Devices designed to deliver parenteral drug products have moveable elastomeric plungers to push the product from the device. Plungers often are provided with a lubricious surface to ease movement of the plunger. Free silicon oil

is traditionally employed to create a lubricious surface, but free oil has been implicated in aggregation and denaturation of proteins.

Silicon oil, a low molecular weight polydimethylsiloxane (PDMS), has been the primary traditional means of making glass and plastic surfaces lubricious and compatible with elastomeric plungers. It is generally sprayed or wiped on the inside of the device. These methods deposit a thin liquid layer of silicon oil. Attempts to permanently adhere the oil on the surface of the device through a baking process have improved the adhesion but silicon oil extractables are still found. Non-uniformity of silicon oil on devices is problematic and can lead to syringe breakage or misfiring when employed in auto injectors.

Non-uniformity of silicon oil arises from improper or poor application of the oil, settling/flow over time under the effects of gravity, and pressure from plungers. During vacuum placement of plungers into the device, the plunger will push silicon oil from the top of the device down to the final resting location of the plunger, See Figure 3a. The silicon oil between the ribs of the plunger and the glass surface of the device will, over time, flow out of the space between the plunger and syringe wall under the pressure of the plunger. Additionally it has been found that silicon oil "settles" or flows under gravity to alter the distribution of the oil. Figure 3 demonstrates examples of silicon oil non-uniformity.

Non uniformity of silicon oil is responsible for a variety of problems, including localized exposure of drug product to a large depot of oil, high break loose forces, and unsmooth operation of the devices due to variable glide forces. Variable glide forces and high break loose forces are particularly problematic with auto injectors, since auto injectors are designed to work with a known and consistent force.

The lubricity coating made by the method of the present invention is created from a plasma that produces a uniform, firmly attached lubricious coating. It has a superior performance relative to existing lubricity approaches, comparing wetting tension, plunger force, and extractables and leachables.

The lubricity coating made by the method of the invention is deposited using a PECVD process that utilizes an organosilicon precursor (preferably a cyclic organosilicon precursor, in particular octamethylcyclotetrasiloxane (OMCTS)), oxygen, radiofrequency and charged electrodes to create the plasma. Without being bound by theory, it is believed that at the pressures and powers that are used, the plasma process is driven by electron impact ionization; that is, the electrons in the process are the driving force behind the chemistry. The process utilizes radiofrequency to excite electrons, resulting in lower temperatures than the other standard method of adding energy to electrons in plasmas, microwaves. The plasma, containing a mixture of high energy electrons and ions of the gases, deposits a coating containing silicon and oxygen and methyl groups attached to the silicon. High energy electrons activate the substrate surface and bonds reform between the surface and the silicon/oxygen/methyl species from the OMCTS. A covalently bound uniform, continuous coating is deposited on the surface.

Since the coating is deposited from plasma, which uniformly fills the container it occupies, at a molecular level, a uniform composition coat is believed to be achieved. The lubricity coating is essentially comprised of silicon, oxygen and methyl groups. AFM, FTIR, TOF/SIMS, XPS and scanning electron microscopy confirm the purity and uniformity.

TABLE I

| Lubricity Coating on Syringes | | |
|---|---|---|
| **Package Type** | **Wetting Tension (dyne/cm)** | **Standard Deviation** |
| Cyclic Olefin Syringe with SiOxCyHz Lubricity Coating | 36 | 0.57 |
| Borosilicate Glass Syringe with Silicone Oil | 37 | 0.57 |
| Cyclic Olefin Container with Triboglide Coating | <30 | N/A |
| Precision of the wetting tension measurement is +/- 3 dyne/cm | | |

Table I, above, shows the wetting tension measured for a COC syringe with SiOxCyHz lubricity coating, a borosilicate glass syringe coated with silicon oil (Dow Corning Medical Grade 360), and a COC container with Triboglide Coating (another known liquid lubricant). A wetting tension of 30 dyne/cm is considered very hydrophobic, a wetting tension of 70 dyne/cm very hydrophilic. All three surfaces therefore show appreciable hydrophobicity. The lubricity coating made by the method of the invention shows a hydrophobicity similar to silicon oil, but less than Triboglide.

The lubricity coating made by the method of present invention can also be applied onto a $SiO_x$ barrier coating. This is shown in Figure 21, which contains a TEM picture of a lubricity coating on a $SiO_2$ layer.

The determination of an extractables profile for an exemplary lubricity coating is described in Example Z. The lubricity coating preferably provides less extractables than a silicon oil coated glass syringe (Example Z), typically less than 10% of the extractables of the latter. In general, the lubricity coating extractables amount ranges from 1 to 500 $\mu$g/L, preferably from 5 to 300 $\mu$g/L. Typically, it may range from 80 to 300 $\mu$g/L, based on the static method of determination.

Since the lubricity coating is attached to the coated surface, the coating will remain uniform over time and consistent, reproducible break loose and glide forces will be maintained. Exemplary break loose and glide forces are shown in Table II:

TABLE II

| Plunger Force Comparison of Lubricity Coatings | | |
|---|---|---|
| **Package Type** | **Initiation Force (N)** | **Maintenance Force (N)** |
| Uncoated Cyclic Olefin Syringe | >15 | >15 |
| Cyclic Olefin Syringe with SiOxCyHz Lubricity Coating | 4.1 | 3.5 |
| Cyclic Olefin Syringe with Silicone Oil | 8.2 | 6.3 |
| Cyclic Olefin Container with Triboglide Coating | 5.7 | 2.0 |

The lubricity coating optionally provides a consistent plunger force that reduces the difference between the break loose force (Fi) and the glide force (Fm). These two forces are important performance measures for the effectiveness of a lubricity coating. For Fi and Fm, it is desired to have a low, but not too low value. With too low Fi, which means a too low level of resistance (the extreme being zero), premature/unintended flow may occur, which might e.g. lead to an unintentional premature or uncontrolled discharge of the content of a prefilled syringe.

In order to achieve a sufficient lubricity (e.g. to ensure that a syringe plunger can be moved in the syringe, but to avoid uncontrolled movement of the plunger), the following ranges of Fi and Fm should be advantageously maintained:

Fi: 2.5 to 5 lbs, preferably 2.7 to 4.9 lbs, and in particular 2.9 to 4.7 lbs;
Fm: 2.5 to 8.0 lbs, preferably 3.3 to 7.6 lbs, and in particular 3.3 to 4 lbs.

Further advantageous Fi and Fm values can be found in the Tables of the Examples. From the Examples, it can also be seen that lower Fi and Fm values can be achieved than the ranges indicated above.

Table II compares a lubricity coating made according to the invention on a syringe with silicon oil and Triboglide lubricity coatings. The results demonstrate that the lubricity coating preferably provides superior consistency between Fi and Fm.

Break-loose and glide forces are important throughout a device's shelf life especially in automated devices such as auto-injectors. Changes in break-loose and/or glide forces can lead to misfiring of auto injectors.

The present lubricity coatings can optionally have more than 10-times less silicon extractables compared to a silicon oil coated syringe. During the PECVD process, the lubricity coating is bonded to the syringe. This results in dramatically lower extractables. Through process optimization, the total silicon extractables from the lubricity coating can be further reduced.

The lubricity coating made by the method according to present invention is prepared by PECVD using an organosilicon precursor and $O_2$. In a particular embodiment, these two precursors are mixed with a carrier gas, typically a noble gas, and most typically Argon.

The organosilicon precursor may be any of the precursors listed elsewhere in present description. However, cyclic organosilicon precursors, in particular monocyclic organosilicon precursors (like the monocyclic precursors listed elsewhere in present description), and specifically OMCTS, are particularly suitable to achieve a lubricious coating.

The presence of $O_2$ and optionally of a carrier gas, in particular of Argon, can increase the lubricity of the resulting coating. The presence of both $O_2$ and Ar together with the organosilicon precursor is particularly advantageous. Generally, in order to get a lubricity coating, $O_2$ is present in an amount (which can, e.g. be expressed by the flow rate in sccm) which does not very much exceed the organosilicon amount and preferably is lower than the organosilicon amount. In contrast, in order to achieve a barrier coating, the amount of $O_2$ typically is at least one order of magnitude higher than the amount of organosilicon precursor. In particular, the volume ratio (in sccm) of $O_2$ to organosilicon precursor for a lubricity coating is from 0.01:1 to 5:1, optionally from 0.01:1 to 1:1, even optionally from 0.01:1 to 0.5:1 or even from 0.01:1 to 0.1:1. It is required that some $O_2$ is present, optionally in an amount of from 0.01:1 to 0.5:1, even optionally from 0.05:1 to 0.4:1, in particular from 0.1:1 to 0.2:1 in relation to the organosilicon precursor. The presence of $O_2$ in a volume of about 5 % to about 35 % (v/v in sccm) in relation to the organosilicon precursor, in particular of about 10 % to about 20 % and in a ratio as given in the Examples is specifically suitable to achieve a lubricity coating.

In one aspect of the invention, a carrier gas is absent in the reaction mixture, in another aspect of the invention, it is present. In a particular aspect of the invention, the carrier gas is present and it is Argon. When Ar is the carrier gas

and it is present in the reaction mixture, it is typically present in a volume (in sccm) exceeding the volume of the organosilicon precursor (and the volume of $O_2$, if present).

Typically, the plasma in the PECVD process is generated at RF frequency. The plasma is generated with an electric power of from 0.1 to 25 W, optionally from 1 to 22 W, optionally from 1 to 10 W, even optionally from 1 to 5 W, optionally from 2 to 4 W, for example of 3 W, optionally from 3 to 17 W, even optionally from 5 to 14 W, for example 6 or 7.5 W, optionally from 7 to 11 W, for example of 8 W. The ratio of the electrode power to the plasma volume can be less than 10 W/ml, optionally is from 5 W/ml to 0.1 W/ml, optionally is from 6 W/ml to 0.1 W/ml, optionally is from 4 W/ml to 0.1 W/ml, optionally from 2 W/ml to 0.2 W/ml. Low power levels are believed by the inventors to be most advantageous (e.g. power levels of from 2 to 3.5 W and the power levels given in the Examples) to prepare a lubricity coating. These power levels are suitable for applying lubricity coatings to syringes having a void volume of 1 to 3 mL in which PECVD plasma is generated. It is contemplated that for larger or smaller objects the power applied should be increased or reduced accordingly to scale the process to the size of the substrate.

The substrate of the lubricity coating is a surface made of plastic (e.g. the interior surface of a plastic syringe). Typical plastic substrates are listed elsewhere in present description. Particularly suitable substrates in the context of present invention are COC, PET, and polypropylene, with COC being specifically suitable.

In one specific aspect of present invention, the substrate is a plastic which is already coated with a coating, e.g. a SiOx barrier coating. On said existing coating, the lubricity coating is applied. Vice versa, the lubricity coating can also be coated with another coating, e.g. a barrier coating.

In a very particular aspect of the present invention, the lubricity is influenced by the roughness of the lubricity coating. It has surprisingly been found that a rough surface of the coating is correlated with enhanced lubricity. The roughness of the lubricity coating is increased with decreasing power (in Watts) energizing the plasma, and by the presence of $O_2$ in the amounts described above.

The vessels (e.g. syringe barrels and/or plungers) coated with a lubricity coating according to present invention have a higher lubricity (determined, e.g. by measuring the Fi and/or Fm) than the uncoated vessels. They also have a higher lubricity than vessels coated with a SiOx coating as described herein.

Exemplary reaction conditions for preparing a lubricity coating according to the present invention in a 3 ml sample size syringe with a 1/8" diameter tube (open at the end) are as follows:

Flow rate ranges:

| | |
|---|---|
| OMCTS: | 0.5 - 5.0 sccm |
| Oxygen: | 0.1 - 5.0 sccm |
| Argon: | 1.0 - 20 sccm |
| Power: | 0.1 - 10 watts |

Specific Flow rates:

| | |
|---|---|
| OMCTS: | 2.0 sccm |
| Oxygen: | 0.7 sccm |
| Argon: | 7.0 sccm |
| Power: | 3.5 watts |

The coating apparatus can advantageously include heated delivery lines from the exit of the OMCTS reservoir to as close as possible to the gas inlet into the syringe.

The lubricity coating is described in more detail under V.C below.

**IB. HYDROPHOBIC COATING**

Silicon, like carbon, is tetravalent, thus preferring to form four bonds. In glass, silicon bonds to oxygen, which is bonded to another silicon (siloxane bonds, Si-O-Si), resulting in a $SiO_2$ polymer. A network of siloxane bonds form, creating silica. At the surface of silica, oxygen atoms that are not bonded to other silicon atoms exist as hydroxyl (OH) groups, known as silanols. Terminal groups at a glass surface can thus be silanols with one or more OH groups, or siloxane bonds. Lone silanols are found in crystalline silica surfaces such as the $SiO_x$ coating described herein. Both lone and vicinal silanols are found in amorphous silicas such as traditional glasses. Without being bound by theory, the chemical nature of the surface chemistry is believed to be largely determined by the density of silanol groups on it. A fully hydroxylated glass surface, that has the maximum density of silanols possible, is quite hydrophilic. A surface on which the silanols are condensed to form siloxane bonds (i.e., minimal density of silanols) has a hydrophobic nature.

Using the coating technologies as described herein, it is possible to control the density of silanols on the surface of the coating. The chemistry of the plasma deposition can be controlled to create either a fully hydroxylated, hydrophilic surface or a minimally hydroxylated, hydrophobic surface.

Table III, below, shows the wetting tension of four different drug container surfaces. A wetting tension of 20 dyne/cm is considered very hydrophobic, while a wetting tension of 80 dyne/cm is very hydrophilic. The data in Table III shows the $SiO_2$ barrier coating is as hydrophilic as traditional glass. In contrast, the lubricity coating of present invention (designated as SiOxCyHz Coating in the table) has a hydrophobic nature similar to that of COC.

Table III

| **Content Contact Surfaces** | |
|---|---|
| **Package Type** | **Wetting Tension (dyne/cm)** |
| Cyclic Olefin Container with $SiO_2$ Coating | >70 |
| Borosilicate Glass Container | >70 |
| Cyclic Olefin Container with SiOxCyHz Coating | 46 |
| Uncoated Cyclic Olefin Container | 36 |
| Precision of the wetting tension measurement was +/- 3 dyne/cm | |

"Hydrophobic" in the context of present invention may mean more hydrophobic than the uncoated substrate (may it be plastic or another coating). However, as is demonstrated in Fig. 23, it can also mean that the surface is as hydrophobic as a comparative hydrophobic surface (like the COC surface in Fig. 23d). Preferably, "hydrophobic" means a wetting tension of less than 60 dyne/cm, more preferably less than 50 dyne/cm, in particular a wetting tension of from 15 dyne/cm to 46 dyne/cm or from 20 dyne/cm to 35 dyne/cm.

The PECVD conditions for a hydrophobic surface coating are contemplated to be similar to those for a lubricity coating, and in fact it is possible that one coating can provide both functions to a useful degree.

The difference in conditions to apply a hydrophobic coating to an SiOx coating is illustrated by comparing a SiOx coating protocol (US2010/0298738 A1, par. 1000 to 1011) with a hydrophobic coating protocol (US2010/0298738 A1, par. 1012 to 1023). The equipment and the precursor (HMDSO) are the same in these two protocols, but the conditions are different, and generally milder for the hydrophobic coating, as illustrated by the exemplary conditions below:

| **Parameter** | **$SiO_x$ Protocol** | **Hydrophobic Protocol** |
|---|---|---|
| **$O_2$ flow rate** | 90 sccm | 60 sccm |
| **Pressure within tube during gas delivery** | 300 mTorr | 270 mTorr |
| **PECVD RF Power** | 50 Watts | 39 Watts |
| **Power on time** | 5 sec | 7 sec |

An advantageous feature of the hydrophobic coating is that it optionally can be applied using the same equipment as a SiOx coating and/or the lubricity coating, so all PECVD coatings can be applied sequentially in a single process, with minor changes in conditions.

The hydrophobic coating can have a lower wetting tension than the uncoated surface, optionally a wetting tension of from 20 to 72 dyne/cm, optionally from 30 to 60 dyne/cm, optionally from 30 to 50 dyne/cm, 30 to 40 dyne/cm, optionally 34 dyne/cm. One proposed wetting tension, namely 34 dyne/cm, is similar to that of a fluid silicone coating on borosilicate glass (30 dynes/cm).

Fig. 23 shows the effect of a hydrophobic coating and of a hydrophilic coating.

## II. VESSEL HOLDERS

For producing the coating according to present invention, a vessel holder is provided. The portable vessel holders 38, 50, and 482 are provided for holding and conveying a vessel having an opening while the vessel is processed. The vessel holder includes a vessel port, a second port, a duct, and a conveyable housing.

The vessel port is configured to seat a vessel opening in a mutually communicating relation. The second port is configured to receive an outside gas supply or vent. The duct is configured for passing one or more gases between a vessel opening seated on the vessel port and the second port. The vessel port, second port, and duct are attached in substantially rigid relation to the conveyable housing. Optionally, the portable vessel holder weighs less than five pounds.

An advantage of a lightweight vessel holder is that it can more readily be transported from one processing station to another.

In certain vessel holders the duct more specifically is a vacuum duct and the second port more specifically is a vacuum port. The vacuum duct is configured for withdrawing a gas via the vessel port from a vessel seated on the vessel port. The vacuum port is configured for communicating between the vacuum duct and an outside source of vacuum. The vessel port, vacuum duct, and vacuum port can be attached in substantially rigid relation to the conveyable housing.

The vessel holders are shown, for example, in FIG. 1. The vessel holder 50 has a vessel port 82 configured to receive and seat the opening of a vessel 80. The interior surface of a seated vessel 80 can be processed via the vessel port 82. The vessel holder 50 can include a duct, for example a vacuum duct 94, for withdrawing a gas from a vessel 80 seated on the vessel port 92. The vessel holder can include a second port, for example a vacuum port 96 communicating between the vacuum duct 94 and an outside source of vacuum, such as the vacuum pump 98. The vessel port 92 and vacuum port 96 can have sealing elements, for example O-ring butt seals, respectively 100 and 102, or side seals between an inner or outer cylindrical wall of the vessel port 82 and an inner or outer cylindrical wall of the vessel 80 to receive and form a seal with the vessel 80 or outside source of vacuum 98 while allowing communication through the port. Gaskets or other sealing arrangements can or also be used.

The vessel holder such as 50 can be made of any material, for example thermoplastic material and/or electrically nonconductive material. Or, the vessel holder such as 50 can be made partially, or even primarily, of electrically conductive material and faced with electrically nonconductive material, for example in the passages defined by the vessel port 92, vacuum duct 94, and vacuum port 96. Examples of suitable materials for the vessel holder 50 are: a polyacetal, for example Delrin® acetal material sold by E. I. du Pont De Nemours and Company, Wilmington Delaware; polytetrafluoroethylene (PTFE), for example Teflon® PTFE sold by E. I. du Pont De Nemours and Company, Wilmington Delaware; Ultra-High-Molecular-Weight Polyethylene (UHMWPE); High density Polyethylene (HDPE).

FIG. 1 also illustrates that the vessel holder, for example 50, can have a collar 116 for centering the vessel 80 when it is approaching or seated on the port 92.

FIG. 10 is an alternative construction for a vessel holder 482. The vessel holder 482 comprises an upper portion 484 and a base 486 joined together at a joint 488. A sealing element, for example an O-ring 490 (the right side of which is cut away to allow the pocket retaining it to be described) is captured between the upper portion 484 and the base 486 at the joint 488. In the illustrated embodiment, the O-ring 490 is received in an annular pocket 492 to locate the O-ring when the upper portion 484 is joined to the base 486.

The O-ring 490 is captured and bears against a radially extending abutment surface 494 and the radially extending wall 496 partially defining the pocket 492 when the upper portion 484 and the base 486 are joined, in this case by the screws 498 and 500. The O-ring 490 thus seats between the upper portion 484 and base 486. The O-ring 490 captured between the upper portion 484 and the base 486 also receives the vessel 80 (removed in this figure for clarity of illustration of other features) and forms a first O-ring seal of the vessel port 502 about the vessel 80 opening.

Though not a requirement, the vessel port 502 has both the first O-ring 490 seal and a second axially spaced O-ring 504 seal, each having an inner diameter such as 506 sized to receive the outer diameter of a vessel such as 80 for sealing between the vessel port 502 and a vessel such as 80. The spacing between the O-rings 490 and 504 provides support for a vessel such as 80 at two axially spaced points, preventing the vessel such as 80 from being skewed with respect to the O-rings 490 and 504 or the vessel port 502. Though not a requirement, the radially extending abutment surface 494 is located proximal of the O-ring 490 and 506 seals and surrounding the vacuum duct 508.

## III. PROCESSING VESSELS SEATED ON VESSEL HOLDERS

FIG. 1 shows a method for processing a vessel 80. The method can be carried out as follows.

A vessel 80 can be provided having an opening 82 and a wall 86 defining an interior surface 88. As one embodiment, the vessel 80 can be formed in and then removed from a mold. Optionally within 60 seconds, or within 30 seconds, or within 25 seconds, or within 20 seconds, or within 15 seconds, or within 10 seconds, or within 5 seconds, or within 3 seconds, or within 1 second after removing the vessel from the mold, or as soon as the vessel 80 can be moved without distorting it during processing (assuming that it is made at an elevated temperature, from which it progressively cools), the vessel opening 82 can be seated on the vessel port 92. Quickly moving the vessel 80 from the mold to the vessel port 92 reduces the dust or other impurities that can reach the surface 88 and occlude or prevent adhesion of the barrier or other type of coating 90. Also, the sooner a vacuum is drawn on the vessel 80 after it is made, the less chance any particulate impurities have of adhering to the interior surface 88.

A vessel holder such as 50 comprising a vessel port 92 can be provided. The opening 82 of the vessel 80 can be seated on the vessel port 92. Before, during, or after seating the opening 82 of the vessel 80 on the vessel port 92, the vessel holder can be transported to position the vessel holder 50 with respect to the processing device or station.

The interior surface 88 of the seated vessel 80 can be then processed via the vessel port 92 at the first processing

station, which can be, as one example, the coating station 28 shown in FIG. 1. The vessel holder 50 and seated vessel 80 are transported from the first processing station 28 to the second processing station. The interior surface 88 of the seated vessel 80 can be processed via the vessel port 92 at the second processing station.

Any of the above methods can include the further step of removing the vessel 80 from the vessel holder following processing the interior surface 88 of the seated vessel 80 at the second processing station or device.

Any of the above methods can include the further step, after the removing step, of providing a second vessel 80 having an opening 82 and a wall 86 defining an interior surface 88. The opening 82 of the second vessel such as 80 can be seated on the vessel port 92 of another vessel holder. The interior surface of the seated second vessel 80 can be processed via the vessel port 92 at the first processing station or device. The vessel holder and seated second vessel 80 can be transported from the first processing station or device to the second processing station or device. The seated second vessel 80 can be processed via the vessel port 92 by the second processing station or device.

## IV. PECVD APPARATUS FOR MAKING VESSELS INCLUDING VESSEL HOLDER, INTERNAL ELECTRODE, VESSEL AS REACTION CHAMBER

A PECVD apparatus suitable for performing the present invention includes a vessel holder, an inner electrode, an outer electrode, and a power supply. A vessel seated on the vessel holder defines a plasma reaction chamber, which optionally can be a vacuum chamber. Optionally, a source of vacuum, a reactant gas source, a gas feed or a combination of two or more of these can be supplied. Optionally, a gas drain, not necessarily including a source of vacuum, is provided to transfer gas to or from the interior of a vessel seated on the port to define a closed chamber.

The PECVD apparatus can be used for atmospheric-pressure PECVD, in which case the plasma reaction chamber does not need to function as a vacuum chamber.

In FIG. 1, the vessel holder 50 comprises a gas inlet port 104 for conveying a gas into a vessel seated on the vessel port. The gas inlet port 104 has a sliding seal provided by at least one O-ring 106, or two O-rings in series, or three O-rings in series, which can seat against a cylindrical probe 108 when the probe 108 is inserted through the gas inlet port 104. The probe 108 can be a gas inlet conduit that extends to a gas delivery port at its distal end 110. The distal end 110 can be inserted deep into the vessel 80 for providing one or more PECVD reactants and other gaseous reactant or process gases.

FIG. 11 shows additional optional details of the coating station 28. The coating station 28 can also have a main vacuum valve 574 in its vacuum line 576 leading to the pressure sensor 152. A manual bypass valve 578 is provided in the bypass line 580. A vent valve 582 controls flow at the vent 404.

Flow out of the PECVD gas or precursor source 144 is controlled by a main reactant gas valve 584 regulating flow through the main reactant feed line 586. One component of the gas source 144 is the organosilicon liquid reservoir 588. The contents of the reservoir 588 are drawn through the organosilicon capillary line 590, which is provided at a suitable length to provide the desired flow rate. Flow of organosilicon vapor is controlled by the organosilicon shut-off valve 592. Pressure is applied to the headspace 614 of the liquid reservoir 588, for example a pressure in the range of 0-15 psi (0 to 78 cm. Hg), from a pressure source 616 such as pressurized air connected to the headspace 614 by a pressure line 618 to establish repeatable organosilicon liquid delivery that is not dependent on atmospheric pressure (and the fluctuations therein). The reservoir 588 is sealed and the capillary connection 620 is at the bottom of the reservoir 588 to ensure that only neat organosilicon liquid (not the pressurized gas from the headspace 614) flows through the capillary tube 590. The organosilicon liquid can be heated above ambient temperature to cause the organosilicon liquid to evaporate, forming an organosilicon vapor. Oxygen is provided from the oxygen tank 594 via an oxygen feed line 596 controlled by a mass flow controller 598 and provided with an oxygen shut-off valve 600.

Referring especially to FIG. 1, the processing station 28 can include an electrode 160 fed by a radio frequency power supply 162 for providing an electric field for generating plasma within the vessel 80 during processing. The probe 108 is also electrically conductive and is grounded, thus providing a counter-electrode within the vessel 80. Alternatively, the outer electrode 160 can be grounded and the probe 108 directly connected to the power supply 162.

In FIG. 1, the outer electrode 160 can either be generally cylindrical as illustrated in FIGS 1 and 2 or a generally U-shaped elongated channel as illustrated in FIG. 1 (FIG. 2 showing a section taken along section line A-A of FIG. 1). Each illustrated electrode has one or more sidewalls, such as 164 and 166, and optionally a top end 168, disposed about the vessel 80 in close proximity.

The electrode 160 shown in FIG. 1 can be shaped like a "U" channel with its length into the page and the puck or vessel holder 50 can move through the activated (powered) electrode during the treatment/coating process. Note that since external and internal electrodes are used, this apparatus can employ a frequency between 50 Hz and 1 GHz applied from a power supply 162 to the U channel electrode 160. The probe 108 can be grounded to complete the electrical circuit, allowing current to flow through the low-pressure gas(es) inside of the vessel 80. The current creates plasma to allow the selective treatment and/or coating of the interior surface 88 of the device.

The electrode in FIG. 1 can also be powered by a pulsed power supply. Pulsing allows for depletion of reactive

gases and then removal of by-products prior to activation and depletion (again) of the reactive gases. Pulsed power systems are typically characterized by their duty cycle which determines the amount of time that the electric field (and therefore the plasma) is present. The power-on time is relative to the power-off time. For example a duty cycle of 10% can correspond to a power on time of 10% of a cycle where the power was off for 90% of the time. As a specific example, the power might be on for 0.1 second and off for 1 second. Pulsed power systems reduce the effective power input for a given power supply 162, since the off-time results in increased processing time. When the system is pulsed, the resulting coating can be very pure (no by products or contaminants). Another result of pulsed systems is the possibility to achieve atomic layer or coating deposition (ALD). In this case, the duty cycle can be adjusted so that the power-on time results in the deposition of a single layer or coating of a desired material. In this manner, a single atomic layer or coating is contemplated to be deposited in each cycle. This approach can result in highly pure and highly structured coatings (although at the temperatures required for deposition on polymeric surfaces, temperatures optionally are kept low (<100°C) and the low-temperature coatings can be amorphous).

An alternative coating station employs a microwave cavity instead of an outer electrode. The energy applied can be a microwave frequency, for example 2.45 GHz. However, in the context of present invention, a radiofrequency is preferred.

### V.1 Precursors for PECVD Coating

The precursor for the PECVD coating of the present invention an organosilicon precursor. An "organosilicon precursor" is defined throughout this specification most broadly as a compound having at least one of the linkages:

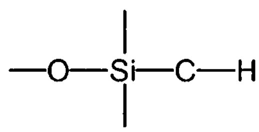

or

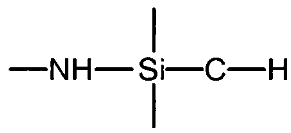

The first structure immediately above is a tetravalent silicon atom connected to an oxygen atom and an organic carbon atom (an organic carbon atom being a carbon atom bonded to at least one hydrogen atom). The second structure immediately above is a tetravalent silicon atom connected to an -NH- linkage and an organic carbon atom (an organic carbon atom being a carbon atom bonded to at least one hydrogen atom). Optionally, the organosilicon precursor is selected from the group consisting of a linear siloxane, a monocyclic siloxane, a polycyclic siloxane, a polysilsesquioxane, a linear silazane, a monocyclic silazane, a polycyclic silazane, a polysilsesquiazane, and a combination of any two or more of these precursors. Also contemplated as a precursor, though not within the two formulas immediately above, is an alkyl trimethoxysilane. If an oxygen-containing precursor (e.g. a siloxane) is used, a representative predicted empirical composition resulting from PECVD under conditions forming a hydrophobic or lubricating coating would be $Si_wO_xC_yH_z$ as defined in the Definition Section, while a representative predicted empirical composition resulting from PECVD under conditions forming a barrier coating would be $SiO_x$, where x in this formula is from about 1.5 to about 2.9. If a nitrogen-containing precursor (e.g. a silazane) is used, the predicted composition would be $Si_{w*}N_{x*}C_{y*}H_{z*}$, i.e. in $Si_wO_xC_yH_z$ as specified in the Definition Section, O is replaced by N and the indices are adapted to the higher valency of N as compared to O (3 instead of 2). The latter adaptation will generally follow the ratio of w, x, y and z in a siloxane to the corresponding indices in its aza counterpart. In a particular aspect of the invention, $Si_{w*}N_{x*}C_{y*}H_{z*}$ in which w*, x*, y*, and z* are defined the same as w, x, y, and z for the siloxane counterparts, but for an optional deviation in the number of hydrogen atoms.

One type of precursor starting material having the above empirical formula is a linear siloxane, for example a material having the following formula:

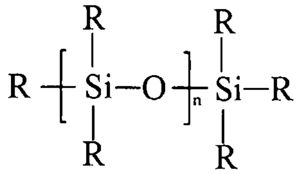

in which each R is independently selected from alkyl, for example methyl, ethyl, propyl, isopropyl, butyl, isobutyl, t-butyl, vinyl, alkyne, or others, and n is 1, 2, 3, 4, or greater, optionally two or greater. Several examples of contemplated linear siloxanes are

- hexamethyldisiloxane (HMDSO),
- octamethyltrisiloxane,
- decamethyltetrasiloxane,
- dodecamethylpentasiloxane,

or combinations of two or more of these. The analogous silazanes in which -NH- is substituted for the oxygen atom in the above structure are also useful for making analogous coatings. Several examples of contemplated linear silazanes are octamethyltrisilazane, decamethyltetrasilazane, or combinations of two or more of these.

Another type of precursor starting material is a monocyclic siloxane, for example a material having the following structural formula:

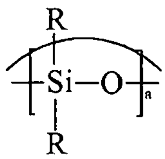

in which R is defined as for the linear structure and "a" is from 3 to about 10, or the analogous monocyclic silazanes. Several examples of contemplated hetero-substituted and unsubstituted monocyclic siloxanes and silazanes include

- 1,3,5-trimethyl-1,3,5-tris(3,3,3-trifluoropropyl)methyl]cyclotrisiloxane
- 2,4,6,8-tetramethyl-2,4,6,8-tetravinylcyclotetrasiloxane,
- pentamethylcyclopentasiloxane,
- pentavinylpentamethylcyclopentasiloxane,
- hexamethylcyclotrisiloxane,
- hexaphenylcyclotrisiloxane,
- octamethylcyclotetrasiloxane (OMCTS),
- octaphenylcyclotetrasiloxane,
- decamethylcyclopentasiloxane
- dodecamethylcyclohexasiloxane,
- methyl(3,3,3-trifluoropropl)cyclosiloxane,
- Cyclic organosilazanes are also contemplated, such as
- Octamethylcyclotetrasilazane,
- 1,3,5,7-tetravinyl-1,3,5,7-tetramethylcyclotetrasilazane hexamethylcyclotrisilazane,
- octamethylcyclotetrasilazane,
- decamethylcyclopentasilazane,
- dodecamethylcyclohexasilazane, or

combinations of any two or more of these.

Another type of precursor starting material is a polycyclic siloxane, for example a material having one of the following structural formulas:

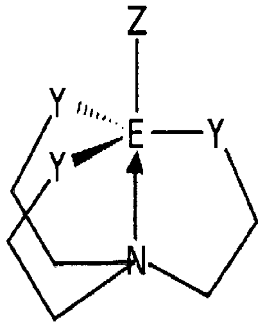
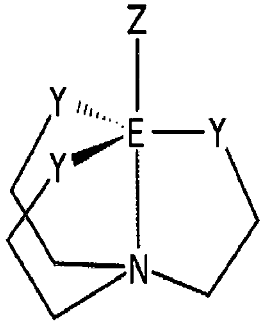
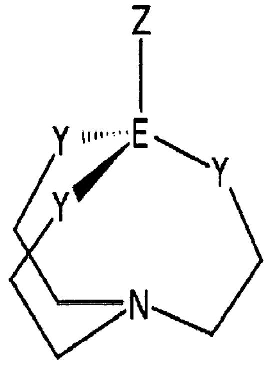

in which Y can be oxygen or nitrogen, E is silicon, and Z is a hydrogen atom or an organic substituent, for example alkyl such as methyl, ethyl, propyl, isopropyl, butyl, isobutyl, t-butyl, vinyl, alkyne, or others. When each Y is oxygen, the respective structures, from left to right, are a silatrane, a silquasilatrane, and a silproatrane. When Y is nitrogen, the respective structures are an azasilatrane, an azasilquasiatrane, and an azasilproatrane.

Another type of polycyclic siloxane precursor starting material is a polysilsesquioxane, with the empirical formula $RSiO_{1.5}$ and the structural formula:

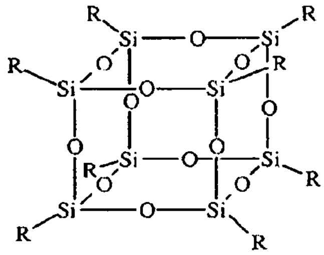

$T_8$ cube

in which each R is a hydrogen atom or an organic substituent, for example alkyl such as methyl, ethyl, propyl, isopropyl, butyl, isobutyl, t-butyl, vinyl, alkyne, or others. Two commercial materials of this sort are SST-eM01 poly(methylsilsesquioxane), in which each R is methyl, and SST-3MH1.1 poly(Methyl-Hydridosilsesquioxane), in which 90% of the R groups are methyl, 10% are hydrogen atoms. This material is available in a 10% solution in tetrahydrofuran, for example. Combinations of two or more of these are also contemplated. Other examples of a contemplated precursor are methylsilatrane, CAS No. 2288-13-3, in which each Y is oxygen and Z is methyl, methylazasilatrane, poly(methylsilsesquioxane) (e.g. SST-eM01 poly(methylsilsesquioxane)), in which each R optionally can be methyl, SST-3MH1.1 poly(Methyl-Hydridosilsesquioxane) (e.g. SST-3MH1.1 poly(Methyl-Hydridosilsesquioxane)), in which 90% of the R groups are methyl and 10% are hydrogen atoms, or a combination of any two or more of these.

The analogous polysilsesquiazanes in which -NH- is substituted for the oxygen atom in the above structure are also useful for making analogous coatings. Examples of contemplated polysilsesquiazanes are a poly(methylsilsesquiazane), in which each R is methyl, and a poly(Methyl-Hydridosilsesquiazane, in which 90% of the R groups are methyl, 10% are hydrogen atoms. Combinations of two or more of these are also contemplated.

One particularly contemplated precursor for the lubricity layer or coating made according to the present invention is a monocyclic siloxane, for example is octamethylcyclotetrasiloxane.

One particularly contemplated precursor for a hydrophobic layer or coating is a monocyclic siloxane, for example is octamethylcyclotetrasiloxane. Another particularly contemplated precursor for a hydrophobic layer or coating is a linear siloxane, for example HMDSO.

One particularly contemplated precursor for a barrier coating is a linear siloxane, for example is HMDSO.

In any of the coating methods according to the present invention, the applying step optionally can be carried out by vaporizing the precursor and providing it in the vicinity of the substrate. E.g., OMCTS is usually vaporized by heating it to about 50°C before applying it to the PECVD apparatus.

## V.2 General PECVD Method

In the context of the present invention, the following PECVD method is generally applied, which contains the following steps:

(a) providing a process gas comprising a precursor as defined herein, oxygen, optionally a carrier gas, and optionally a hydrocarbon; and

(b) generating a plasma from the process gas, thus forming a coating on the substrate surface by plasma enhanced chemical vapor deposition (PECVD).

The plasma coating technology used herein is based on Plasma Enhanced Chemical Vapor Deposition (PECVD). Methods and apparatus suitable to perform said PECVD coatings are described in EP10162755.2 filed May 12, 2010; EP10162760.2 filed May 12, 2010; EP10162756.0 filed May 12, 2010; EP10162758.6 filed May 12, 2010; EP10162761.0 filed May 12, 2010; and EP10162757.8 filed May 12, 2010. The PECVD methods and apparatus as described therein are suitable to perform the present invention.

Without being bound by theory, it is assumed that the coating is covalently attached to the coated surface (may it be the plastic substrate surface or the surface of a coating which is already present on the substrate surface, e.g. a $SiO_x$ barrier coating) in the PECVD process of the invention. The process uses a silicon source (e.g. HMDSO or OMCTS), oxygen, radiofrequency (RF) and charged electrodes to create the plasma. Optionally, a carrier gas like Argon is present as well. A hydrocarbon gas may also be present in specific applications. At the pressures and powers that are used, the plasma process is driven by electron impact ionization; that is, the electrons in the process are the driving force of the reaction. The process utilizes RF to excite the electrons, resulting in lower temperatures than traditional standard methods of energizing electrons in plasmas, i.e., microwaves.

The plasma, which contains a mixture of high energy electrons and ions of the gases, deposits a silicon and oxide containing coating onto the plastic (e.g. COC) surface. Electrons from the plasma interact at the polymer surface upon initiation of the plasma reaction, "etching" the surface by breaking C-H bonds (a similar "etching by breaking bonds" takes place when a coating is coated again, e.g. when a lubricity coating is applied on a SiOx barrier coating). Under the right conditions, these sites then are believed to act as nucleation points where the Si-O-Si backbone (which is formed from the ionization of the silicon containing molecule in the gas phase) bonds with the polymer, from which the coating grows, eventually forming a uniform, continuous coating over the entire polymer surface. The silicon-carbon (silicon to methyl groups) bonds in the organosilicon precursor can react with oxygen, breaking the bond between the methyl group and silicon and reforming bonds with the plastic surface or other Si-O groups already on the surface.

Since the coating is grown from a plasma, which is an ionized gas, completely filling the container it occupies, a dense, uniform and conformal coating is achieved at a molecular level. See Figures 21 and 22. The purity of the coating is assured through the use of pure precursor gases. This process results in a surface with uniform, controllable energy. Analytical characterization of a coating with atomic force microscopy (AFM), FTIR, TOF-SIMS, XPS, electron spectroscopy for chemical analysis (ESCA) and scanning electron microscopy can confirm the purity and uniformity.

An exemplary preferred embodiment of the PECVD technology will be described in the following sections.

The process utilizes a silicon containing vapor that can be combined with oxygen at reduced pressures (mTorr range - atmospheric pressure is 760 Torr) inside a container.

An electrical field generated at, e.g., 13.56 MHz [radio frequency range] is then applied between an external electrode and an internal grounded gas inlet to create a plasma. At the pressures and powers that are used to coat a container, the plasma process is driven by electron impact ionization, which means the electrons in the process are the driving force behind the chemistry. Specifically, the plasma drives the chemical reaction through electron impact ionization of the silicon containing material [e.g., hexamethyldisiloxane (HMDSO) or other reactants like octamethylcyclotretrasiloxane (OMCTS)] resulting in a silicon dioxide or $Si_wO_xC_yH_z$ coating deposited onto the interior surfaces of the container. These coatings are in a typical embodiment on the order of 20 or more nanometers in thickness. HMDSO consists of an Si-O-Si backbone with six (6) methyl groups attached to the silicon atoms. The process breaks the Si-C bonds and (at the surface of the tube or syringe) reacts with oxygen to create silicon dioxide. Since the coating is grown on an atomic basis, dense, conformal coatings with thicknesses of 20-30 nanometers can achieve significant barrier properties. The silicon oxide acts as a physical barrier to gases, moisture, and small organic molecules, and is of greater purity than commercial glasses. OMCTS results in coatings with lubricity or anti-adhesion properties. Their average thickness is generally higher than the thickness of the $SiO_x$ barrier coating, e.g. from 30 to 1000 nm on average. A certain roughness may enhance the lubricious properties of the lubricity coating, thus its thickness is advantageously not uniform throughout the coating (see below). However, a uniform lubricity coating is also considered.

The technology is unique in several aspects:

(a) The process utilizes the rigid container as the vacuum chamber. PECVD conventionally uses a secondary vacuum vessel into which the part(s) are loaded and coated. Utilizing the container as a vacuum chamber significantly simplifies the process apparatus and reduces cycle/processing time, and thus manufacturing cost and capital. This approach also reduces scale-up issues since scale-up is as simple as replicating the number of syringes required to meet the throughput requirements.

(b) Radio Frequency excitation of the plasma allows energy to be imparted to the ionized gas with little heating of

the part. Unlike microwave excitation energies, typically used in PECVD, which will impart significant energy to water molecules in the part itself, radio frequency will not preferentially heat the polymeric syringes. Controlled heat absorption is critical to prevent substrate temperature increases approaching plastic glass transition temperatures, causing loss of dimensional integrity (collapse under vacuum).

(c) Single layer gas barrier coating - the new technology can generate a single layer of silicon dioxide directly on the interior surface of the part. Most other barrier technologies (thin film) require at least two layers.

(d) Combination barrier-lubricity coatings - the new technology utilizes a combination $SiO_x$ / $Si_wO_xC_yH_z$ coating to provide multiple performance attributes (barrier/lubricity). The lubricity coating can also be coated again with a barrier coating.

The plasma deposition technology in a preferred aspect utilizes a simple manufacturing configuration. The system is based on a "puck," which is used in transportation of syringes in and out of the coating station. The device-puck interface is critical, since once coating/characterization conditions are established at the pilot scale, there are no scaling issues when moving to full scale production; one simply increases the number of pucks through the same process. The puck is manufactured from a polymeric material (e.g. Delrin™) to provide an electrically insulated base. The container is mounted into the puck with the largest opening sealing against an o-ring (mounted in the puck itself). The o-ring provides the vacuum seal between the part and the puck so that the ambient air (principally nitrogen and oxygen with some water vapor) can be removed (pressure reduced) and the process gases introduced. The puck has several key features in addition to the o-ring seal. The puck provides a means of connection to the vacuum pump (which pumps away the atmospheric gases and the byproducts of the silicon dioxide reaction), a means of accurately aligning the gas inlet in the part, and a means of providing a vacuum seal between the puck and gas inlet.

For $SiO_2$ deposition, HMDSO and oxygen gases are then admitted into the container through the grounded gas inlet which extends up into the part. At this point, the puck and container are moved into the electrode area. The electrode is constructed from a conductive material (for example copper) and provides a tunnel through which the part passes. The electrode does not make physical contact with the container or the puck and is supported independently. An RF impedance matching network and power supply are connected directly to the electrode. The power supply provides energy (at 13.56 MHz) to the impedance matched network. The RF matching network acts to match the output impedance of the power supply to the complex (capacitive and inductive) impedance of the ionized gases. The matching network delivers maximum power delivery to the ionized gas which ensures deposition of the silicon dioxide coating.

Once the container is coated (as the puck moves the container through the electrode channel - which is stationary), the gases are stopped and atmospheric air (or pure nitrogen) is allowed inside the puck/container to bring it back to atmospheric pressure. At this time, the container can be removed from the puck and moved to the next processing station.

The above describes clearly the means of coating a container having just one opening. Syringes require an additional step before and after loading onto the puck. Since the syringes have openings at both ends (one for connection to a needle and the second for installation of a plunger), the needle end must be sealed prior to coating. The above process allows reaction gases to be admitted into the plastic part interior, an electrical current to pass through the gas inside of the part and a plasma to be established inside the part. The plasma (an ionized composition of the HMDSO or OMCTS and oxygen gases) is what drives the chemistry and the deposition of the plasma coating.

In the method, the coating characteristics are advantageously set by one or more of the following conditions: the plasma properties, the pressure under which the plasma is applied, the power applied to generate the plasma, the presence and relative amount of $O_2$ in the gaseous reactant, the presence and relative amount of a carrier gas, e.g. of Argon in the gaseous reactant, the plasma volume, and the organosilicon precursor. Optionally, the coating characteristics are set by the presence and relative amount of $O_2$ in the gaseous reactant, and/or the presence and relative amount of the carrier gas (e.g. Argon) and/or the power applied to generate the plasma.

The plasma is in an optional aspect a non-hollow-cathode plasma, in particular when an $SiO_x$ coating is formed. In an alternative optional aspect, there is a hollow-cathode plasma, in particular when a lubricity coating is formed.

In a further preferred aspect, the plasma is generated at reduced pressure (as compared to the ambient or atmospheric pressure). Optionally, the reduced pressure is less than 300 mTorr, optionally less than 200 mTorr, even optionally less than 100 mTorr.

The PECVD optionally is performed by energizing the gaseous reactant containing the precursor with electrodes powered at a frequency at microwave or radio frequency, and optionally at a radio frequency. The radio frequency preferred to perform an embodiment of the invention will also be addressed as "RF frequency". A typical radio frequency range for performing the present invention is a frequency of from 10 kHz to less than 300 MHz, optionally from 1 to 50 MHz, even optionally from 10 to 15 MHz. A frequency of 13.56 MHz is most preferred, this being a government sanctioned frequency for conducting PECVD work.

There are several advantages for using a RF power source versus a microwave source: Since RF operates a lower power, there is less heating of the substrate/vessel. Because the focus of the present invention is putting a plasma

coating on plastic substrates, lower processing temperature are desired to prevent melting/distortion of the substrate. To prevent substrate overheating when using microwave PECVD, the microwave PECVD is applied in short bursts, by pulsing the power. The power pulsing extends the cycle time for the coating, which is undesired in the present invention. The higher frequency microwave can also cause offgassing of volatile substances like residual water, oligomers and other materials in the plastic substrate. This offgassing can interfere with the PECVD coating. A major concern with using microwave for PECVD is delamination of the coating from the substrate. Delamination occurs because the microwaves change the surface of the substrate prior to depositing the coating layer. To mitigate the possibility of delamination, interface coating layers have been developed for microwave PECVD to achieve good bonding between the coating and the substrate. No such interface coating layer or coating is needed with RF PECVD as there is no risk of delamination. Finally, the lubricity layer or coating according to the present invention and hydrophobic layer or coating are advantageously applied using lower power. RF power operates at lower power and provides more control over the PECVD process than microwave power. Nonetheless, microwave power, though less preferred, is usable under suitable process conditions.

Furthermore, for all PECVD methods described herein, there is a specific correlation between the power (in Watts) used to generate the plasma and the volume of the lumen wherein the plasma is generated. Typically, the lumen is the lumen of a vessel coated according to the present invention. The RF power should scale with the volume of the vessel if the same electrode system is employed. Once the composition of a gaseous reactant, for example the ratio of the precursor to $O_2$, and all other parameters of the PECVD coating method but the power have been set, they will typically not change when the geometry of a vessel is maintained and only its volume is varied. In this case, the power will be directly proportional to the volume. Thus, starting from the power to volume ratios provided by present description, the power which has to be applied in order to achieve the same or a similar coating in a vessel of same geometry, but different size, can easily be found.

For any coating of the present invention, the plasma is generated with electrodes powered with sufficient power to form a coating on the substrate surface. For a lubricity layer or coating, or hydrophobic layer or coating (one layer may also be both lubricant and hydrophobic), in the method according to an embodiment of the invention the plasma is generated with electrodes supplied with an electric power of from 0.1 to 25 W, optionally from 1 to 22 W, optionally from 1 to 10 W, even optionally from 1 to 5 W, optionally from 2 to 4 W, for example of 3 W, optionally from 3 to 17 W, even optionally from 5 to 14 W, for example 6 or 7.5 W, optionally from 7 to 11 W, for example of 8 W. The ratio of the electrode power to the plasma volume may be less than 10 W/ml, optionally is from 6 W/ml to 0.1 W/ml, optionally is from 5 W/ml to 0.1 W/ml, optionally is from 4 W/ml to 0.1 W/ml, optionally is from 3 W/ml to 0.2 W/ml, optionally is from 2 W/ml to 0.2 W/ml.

For a barrier coating or $SiO_x$ coating, the plasma is optionally generated

(i) with electrodes supplied with an electric power of from 8 to 500 W, optionally from 20 to 400 W, optionally from 35 to 350 W, even optionally from 44 to 300 W, optionally from 44 to 70 W; and/or
(ii) the ratio of the electrode power to the plasma volume is equal or more than 5 W/ml, optionally is from 6 W/ml to 150 W/ml, optionally is from 7 W/ml to 100 W/ml, optionally from 7 W/ml to 20 W/ml.

Low power levels are believed by the inventors to be most advantageous (e.g. power levels of from 2 to 3.5 W and the power levels given in the Examples) to prepare a lubricity coating. These power levels are suitable for applying lubricity coatings to syringes and sample tubes and vessels of similar geometry having a void volume of 1 to 3 mL in which PECVD plasma is generated. It is contemplated that for larger or smaller objects the power applied should be increased or reduced accordingly to scale the process to the size of the substrate.

The vessel geometry can also influence the choice of the gas inlet used for the PECVD coating. In a particular aspect, a syringe can be coated with an open tube inlet.

The power (in Watts) used for PECVD also has an influence on the coating properties. Typically, an increase of the power will increase the barrier properties of the coating, and a decrease of the power will increase the lubricity and hydrophobicity of the coating. This is demonstrated in several Examples, in particular in Examples E to V. It is also demonstrated in Examples of EP 2 251 455 A2, to which explicit reference is made herewith.

A further parameter determining the coating properties is the ratio of $O_2$ to the precursor (e.g. organosilicon precursor) in the gaseous reactant used for generating the plasma. Typically, an increase of the $O_2$ ratio in the gaseous reactant will increase the barrier properties of the coating, and a decrease of the $O_2$ ratio will increase the lubricity and hydrophobicity of the coating.

If a lubricity layer or coating is desired, then $O_2$ is present in a volume-volume ratio to the gaseous reactant of from 0.01:1 to 5:1, optionally from 0.01:1 to 1:1, even optionally from 0.01:1 to 0.5:1 or even from 0.01:1 to 0.1:1. It is required that some $O_2$ is present, optionally in an amount of from 0.01:1 to 0.5:1, even optionally from 0.05:1 to 0.4:1, in particular from 0.1:1 to 0.2:1 in relation to the organosilicon precursor. The presence of $O_2$ in a volume of about 5 % to about 35 % (v/v in sccm) in relation to the organosilicon precursor, in particular of about 10 % to about 20 % and in a ratio as

given in the Examples is specifically suitable to achieve a lubricity coating.

If, on the other hand, a barrier or $SiO_x$ coating is desired, then the $O_2$ is optionally present in a volume : volume ratio to the gaseous reactant of from 1:1 to 100:1 in relation to the silicon containing precursor, optionally in a ratio of from 5:1 to 30:1, optionally in a ratio of from 10:1 to 20:1, even optionally in a ratio of 15:1.

### V.A. PECVD to apply $SiO_x$ barrier coating, using plasma that is substantially free of hollow cathode plasma

In the following described is a method of applying a barrier coating of $SiO_x$, defined in this specification (unless otherwise specified in a particular instance) as a coating containing silicon and oxygen, in which x, the ratio of oxygen to silicon atoms, is from about 1.5 to about 2.9, or 1.5 to about 2.6, or about 2. These alternative definitions of x apply to any use of the term $SiO_x$ in this specification. The barrier coating is applied to the interior of a vessel, for example a syringe barrel. The method includes several steps.

A vessel wall is provided, as is a reaction mixture comprising plasma forming gas, i.e. an organosilicon compound gas, optionally an oxidizing gas, and optionally a hydrocarbon gas.

Plasma is formed in the reaction mixture that is substantially free of hollow cathode plasma. The vessel wall is contacted with the reaction mixture, and the coating of $SiO_x$ is deposited on at least a portion of the vessel wall.

The generation of a uniform plasma throughout the portion of the vessel to be coated is contemplated, as it has been found in certain instances to generate an $SiO_x$ coating providing a better barrier against oxygen. Uniform plasma means regular plasma that does not include a substantial amount of hollow cathode plasma (which has a higher emission intensity than regular plasma and is manifested as a localized area of higher intensity interrupting the more uniform intensity of the regular plasma).

The hollow cathode effect is generated by a pair of conductive surfaces opposing each other with the same negative potential with respect to a common anode. If the spacing is made (depending on the pressure and gas type) such that the space charge sheaths overlap, electrons start to oscillate between the reflecting potentials of the opposite wall sheaths leading to multiple collisions as the electrons are accelerated by the potential gradient across the sheath region. The electrons are confined in the space charge sheath overlap which results in very high ionization and high ion density plasmas. This phenomenon is described as the hollow cathode effect. Those skilled in the art are able to vary the processing conditions, such as the power level and the feed rates or pressure of the gases, to form uniform plasma throughout or to form plasma including various degrees of hollow cathode plasma.

The plasma is typically generated using RF energy for the reasons given above. In an alternate, but less typical method, microwave energy can be used to generate the plasma in a PECVD process. The processing conditions can be different, however, as microwave energy applied to a thermoplastic vessel will excite (vibrate) water molecules. Since there is a small amount of water in all plastic materials, the microwaves will heat the plastic. As the plastic heats, the large driving force created by the vacuum inside of the device relative to atmospheric pressure outside the device will pull free or easily desorb materials to the interior surface 88 where they will either become volatile or will be weakly bound to the surface. The weakly bound materials will then create an interface that can hinder subsequent coatings (deposited from the plasma) from adhering to the plastic interior surface 88 of the device.

As one way to negate this coating hindering effect, a coating can be deposited at very low power (e.g., 5 to 20 Watts at 2.45 GHz) creating a cap onto which subsequent coatings can adhere. This results in a two-step coating process (and two coating layers). The initial gas flows (for the capping layer) can be changed to 2 sccm ("standard cubic centimeters per minute") HMDSO and 20 sccm oxygen with a process power of 5 to 20 Watts for approximately 2-10 seconds. Then the gases can be adjusted and the power level increased to 20-50 Watts so that an $SiO_x$ coating, in which x in this formula is from about 1.5 to about 2.9, alternatively from about 1.5 to about 2.6, alternatively about 2, can be deposited. Note that the capping layer or coating might provide little to no functionality, except to stop materials from migrating to the vessel interior surface 88 during the higher power $SiO_x$ coating deposition. Note also that migration of easily desorbed materials in the device walls typically is not an issue at lower frequencies such as most of the RF range, since the lower frequencies do not excite (vibrate) molecular species.

As another way to negate the coating hindering effect described above, the vessel 80 can be dried to remove embedded water before applying microwave energy. Desiccation or drying of the vessel 80 can be accomplished, for example, by thermally heating the vessel 80, as by using an electric heater or forced air heating. Desiccation or drying of the vessel 80 also can be accomplished by exposing the interior of the vessel 80, or gas contacting the interior of the vessel 80, to a desiccant. Other expedients for drying the vessel, such as vacuum drying, can also be used. These expedients can be carried out in one or more of the stations or devices illustrated or by a separate station or device.

Additionally, the coating hindering effect described above can be addressed by selection or processing of the resin from which the vessels 80 are molded to minimize the water content of the resin.

### V.B. PECVD Coating Restricted Opening of Vessel (Syringe Capillary)

FIGS. 8 and 9 show a method and apparatus generally indicated at 290 for coating an inner surface 292 of a restricted opening 294 of a generally tubular vessel 250 to be processed, for example the restricted front opening 294 of a syringe barrel 250, by PECVD. The previously described process is modified by connecting the restricted opening 294 to a processing vessel 296 and optionally making certain other modifications.

The generally tubular vessel 250 to be processed includes an outer surface 298, an inner or interior surface 254 defining a lumen 300, a larger opening 302 having an inner diameter, and a restricted opening 294 that is defined by an inner surface 292 and has an inner diameter smaller than the inner diameter of the larger opening 302.

The processing vessel 296 has a lumen 304 and a processing vessel opening 306, which optionally is the only opening, although a second opening can be provided that optionally is closed off during processing. The processing vessel opening 306 is connected with the restricted opening 294 of the vessel 250 to be processed to establish communication between the lumen 300 of the vessel 250 to be processed and the processing vessel lumen via the restricted opening 294.

At least a partial vacuum is drawn within the lumen 300 of the vessel 250 to be processed and lumen 304 of the processing vessel 296. A PECVD reactant is flowed from the gas source 144 through the first opening 302, then through the lumen 300 of the vessel 250 to be processed, then through the restricted opening 294, then into the lumen 304 of the processing vessel 296.

The PECVD reactant can be introduced through the larger opening 302 of the vessel 250 by providing a generally tubular inner electrode 308 having an interior passage 310, a proximal end 312, a distal end 314, and a distal opening 316; in an alternative, multiple distal openings can be provided adjacent to the distal end 314 and communicating with the interior passage 310. The distal end of the electrode 308 can be placed adjacent to or into the larger opening 302 of the vessel 250 to be processed. A reactant gas can be fed through the distal opening 316 of the electrode 308 into the lumen 300 of the vessel 250 to be processed. The reactant will flow through the restricted opening 294, then into the lumen 304, to the extent the PECVD reactant is provided at a higher pressure than the vacuum initially drawn before introducing the PECVD reactant.

Plasma 318 is generated adjacent to the restricted opening 294 under conditions effective to deposit a coating of a PECVD reaction product on the inner surface 292 of the restricted opening 294. In the embodiment shown in FIG. 8, the plasma is generated by feeding RF energy to the generally U-shaped outer electrode 160 and grounding the inner electrode 308. The feed and ground connections to the electrodes could also be reversed, though this reversal can introduce complexity if the vessel 250 to be processed, and thus also the inner electrode 308, are moving through the U-shaped outer electrode while the plasma is being generated.

A syringe including a needle and a barrel (a "staked needle syringe") as described in US Serial No. 61/359,434, filed June 29, 2010 may be used in the method of the present invention. The needle has an outside surface, a delivery outlet at one end, a base at the other end, and an internal passage extending from the base to the delivery outlet. The barrel has a, for example generally cylindrical, interior surface defining a lumen. The barrel also has a front passage molded around and in fluid-sealing contact with the outside surface of the needle.

The "staked needle" syringe optionally can further include a cap configured to isolate the delivery outlet of the needle from ambient air.

The cap of any "staked needle" syringe optionally can further include a lumen having an opening defined by a rim and sized to receive the delivery outlet, and the rim can be seatable against an exterior portion of the barrel.

In any "staked needle" syringe, the barrel optionally can further include a generally hemispheric interior surface portion adjacent to its front passage.

In any "staked needle" syringe, the base of the needle optionally can be at least substantially flush with the hemispheric interior surface portion of the barrel.

The "staked needle" syringe optionally can further include a PECVD-applied barrier coating on at least the hemispheric interior surface portion of the barrel.

In any "staked needle" syringe, the barrier coating optionally can extend over at least a portion of the generally cylindrical interior surface portion of the barrel.

In any "staked needle" syringe, the barrier coating optionally can form a barrier between the base of the needle and the generally cylindrical interior surface portion of the barrel.

In the "staked needle" syringe of FIG. 24, the cap 7126 is held in place on the nose 71110 of the syringe 7120 by a conventional Luer lock arrangement. The tapered nose 71110 of the syringe mates with a corresponding tapered throat 71112 of the cap 7126, and the syringe has a collar 71114 with an interior thread 71116 receiving the dogs 71118 and 71120 of the cap 7126 to lock the tapers 71110 and 71112 together. The cap 7126 can be substantially rigid.

Referring now to FIG. 25, a variation on the syringe barrel 71122 and cap 71124 of the "staked needle" syringe is shown. In this embodiment, the cap 71124 includes a flexible lip seal 7172 at its base to form a moisture-tight seal with the syringe barrel 71122.

Optionally in the "staked needle" syringes of FIGS. 24 and 25, the caps 7126 and 71124 can withstand vacuum during the PECVD coating process. The caps 7126 and 71124 can be made of LDPE. Alternative rigid plastic materials can be used as well, for example polypropylene. Additional sealing elements can be provided as well.

In another option of the "staked needle" syringe, illustrated in FIG. 26, the cap 71126 is flexible, and is designed to seal around the top end of the syringe 7120. A deformable material - like a rubber or a thermoplastic elastomer (TPE) can be used for the cap 71126. Preferred TPE materials include fluoroelastomers, and in particular, medical grade fluoroelastomers. Examples include VITON® and TECHNOFLON®. VITON® is preferable in some embodiments. An example of a suitable rubber is EPDM rubber.

During molding, in certain "staked needle" syringes (illustrated for example in FIG. 26) a small amount of the cap material 71132 will be drawn into the tip or delivery outlet 7134 of the needle 7122 to create a seal. The material 71132 should have a durometer such as to permit an appropriate amount of material to be drawn into the needle 7122, and to cause the material drawn into the needle 7122 to continue to adhere to the cap 71126 when it is removed, unplugging the needle 7122 for use.

In other "staked needle" syringes, the cap material 71132 can block the delivery outlet 7134 of the needle 7122 without being drawn into the delivery outlet 7134. Suitable material selection to accomplish the desired purposes is within the capabilities of a person of ordinary skill in the art.

An additional seal can be created by coupling an undercut 71134 formed in the syringe barrel and projections 71138 in the interior of the cap 71126, defining a coupling to retain the cap 71126. Alternative "staked needle" syringes can include either one or both of the seals described above.

Optionally, with reference to FIG. 25, the cap 71124 can have a base 7168 and a coupling 7170 configured for securing the cap 7126 in a seated position on the barrel. Alternatively or in addition, a flexible lip seal 7172 can optionally be provided at the base 7168 of the cap 71124 for seating against the barrel 71122 when the cap 71124 is secured on the barrel 71122.

Optionally, referring now to FIG. 26, the delivery outlet 7134 of the needle 7122 can be seated on the cap 71126 when the cap 7126 is secured on the barrel. This expedient is useful for sealing the delivery outlet 7134 against the ingress or egress of air or other fluids, when that is desired.

Optionally, in the "staked needle" syringe the coupling 7170 can include a detent or groove 7174 on one of the barrel 71122 and the cap 71124 and a projection or rib 7176 on the other of the barrel 71122 and the cap 71124, the projection 7176 being adapted to mate with the detent 7174 when the cap 7126 is in its seated position on the barrel. A detent 7174 can be on the barrel and a projection 7176 can be on the cap 7126. In another case, a detent 7174 can be on the cap 7126 and a projection 7176 can be on the barrel. In yet another case, a first detent 7174 can be on the barrel and a first projection 7176 mating with the detent 7174 can be on the cap 7126, while a second detent 7175 can be on the cap 7126 and the mating second projection 7177 can be on the barrel. A detent 7174 can be molded in the syringe barrel as an undercut by incorporating side draws such as 7192 and 7194 in the mold.

The detents 7174 mate with the complementary projections 7176 to assemble (snap) the cap 7126 onto the syringe 7120. In this respect the cap 7126 is desirably flexible enough to allow sufficient deformation for a snapping engagement of the detents 7174 and projections 7176.

The caps in the "staked needle" syringe such as 7126, 71124, and 71126 can be injection molded or otherwise formed, for example from thermoplastic material. Several examples of suitable thermoplastic material are a polyolefin, for example a cyclic olefin polymer (COP), a cyclic olefin copolymer (COC), polypropylene, or polyethylene. The cap 7126 can contain or be made of a thermoplastic elastomer (TPE) or other elastomeric material. The cap 7126 can also be made of polyethylene terephthalate (PET), polycarbonate resin, or any other suitable material. Optionally, a material for the cap 7126 can be selected that can withstand vacuum and maintain sterility within the syringe 7120.

The plasma 318 generated in the vessel 250 during at least a portion of processing can include hollow cathode plasma generated inside the restricted opening 294 and/or the processing vessel lumen 304. The generation of hollow cathode plasma 318 can contribute to the ability to successfully apply a barrier coating at the restricted opening 294, although the invention is not limited according to the accuracy or applicability of this theory of operation. Thus, in one contemplated mode of operation, the processing can be carried out partially under conditions generating a uniform plasma throughout the vessel 250 and the gas inlet, and partially under conditions generating a hollow cathode plasma, for example adjacent to the restricted opening 294.

The process is desirably operated under such conditions, as explained here and shown in the drawings, that the plasma 318 extends substantially throughout the syringe lumen 300 and the restricted opening 294. The plasma 318 also desirably extends substantially throughout the syringe lumen 300, the restricted opening 294, and the lumen 304 of the processing vessel 296. This assumes that a uniform coating of the interior 254 of the vessel 250 is desired. In other embodiments non-uniform plasma can be desired.

It is generally desirable that the plasma 318 have a substantially uniform color throughout the syringe lumen 300 and the restricted opening 294 during processing, and optionally a substantially uniform color substantially throughout the syringe lumen 300, the restricted opening 294, and the lumen 304 of the processing vessel 296. The plasma desirably

is substantially stable throughout the syringe lumen 300 and the restricted opening 294, and optionally also throughout the lumen 304 of the processing vessel 296.

The order of steps in this method is not contemplated to be critical.

In FIGS. 8 and 9, the restricted opening 294 has a first fitting 332 and the processing vessel opening 306 has a second fitting 334 adapted to seat to the first fitting 332 to establish communication between the lumen 304 of the processing vessel 296 and the lumen 300 of the vessel 250 to be processed.

In FIGS. 8 and 9, the first and second fittings are male and female Luer lock fittings 332 and 334, respectively integral with the structure defining the restricted opening 294 and the processing vessel opening 306. One of the fittings, in this case the male Luer lock fitting 332, comprises a locking collar 336 with a threaded inner surface and defining an axially facing, generally annular first abutment 338 and the other fitting 334 comprises an axially facing, generally annular second abutment 340 facing the first abutment 338 when the fittings 332 and 334 are engaged.

In the illustrated case a seal, for example an O-ring 342 can be positioned between the first and second fittings 332 and 334. For example, an annular seal can be engaged between the first and second abutments 338 and 340. The female Luer fitting 334 also includes dogs 344 that engage the threaded inner surface of the locking collar 336 to capture the O-ring 342 between the first and second fittings 332 and 334. Optionally, the communication established between the lumen 300 of the vessel 250 to be processed and the lumen 304 of the processing vessel 296 via the restricted opening 294 is at least substantially leak proof.

As a further option, either or both of the Luer lock fittings 332 and 334 can be made of electrically conductive material, for example stainless steel. This construction material forming or adjacent to the restricted opening 294 might contribute to formation of the plasma in the restricted opening 294.

The desirable volume of the lumen 304 of the processing vessel 296 is contemplated to be a trade-off between a small volume that will not divert much of the reactant flow away from the product surfaces desired to be coated and a large volume that will support a generous reactant gas flow rate through the restricted opening 294 before filling the lumen 304 sufficiently to reduce that flow rate to a less desirable value (by reducing the pressure difference across the restricted opening 294). The contemplated volume of the lumen 304, in an embodiment, is less than three times the volume of the lumen 300 of the vessel 250 to be processed, or less than two times the volume of the lumen 300 of the vessel 250 to be processed, or less than the volume of the lumen 300 of the vessel 250 to be processed, or less than 50% of the volume of the lumen 300 of the vessel 250 to be processed, or less than 25% of the volume of the lumen 300 of the vessel 250 to be processed. Other effective relationships of the volumes of the respective lumens are also contemplated.

The inventors have found that the uniformity of coating can be improved in certain embodiments by repositioning the distal end of the electrode 308 relative to the vessel 250 so it does not penetrate as far into the lumen 300 of the vessel 250 as the position of the inner electrode shown in previous Figures. For example, although in certain embodiments the distal opening 316 can be positioned adjacent to the restricted opening 294, in other embodiments the distal opening 316 can be positioned less than 7/8 the distance, optionally less than % the distance, optionally less than half the distance to the restricted opening 294 from the larger opening 302 of the vessel to be processed while feeding the reactant gas. Or, the distal opening 316 can be positioned less than 40%, less than 30%, less than 20%, less than 15%, less than 10%, less than 8%, less than 6%, less than 4%, less than 2%, or less than 1% of the distance to the restricted opening 294 from the larger opening of the vessel to be processed while feeding the reactant gas.

Or, the distal end of the electrode 308 can be positioned either slightly inside or outside or flush with the larger opening 302 of the vessel 250 to be processed while communicating with, and feeding the reactant gas to, the interior of the vessel 250. The positioning of the distal opening 316 relative to the vessel 250 to be processed can be optimized for particular dimensions and other conditions of treatment by testing it at various positions. One particular position of the electrode 308 contemplated for treating syringe barrels 250 is with the distal end 314 penetrating about a quarter inch (about 6 mm) into the vessel lumen 300 above the larger opening 302.

The inventors presently contemplate that it is advantageous to place at least the distal end 314 of the electrode 308 within the vessel 250 so it will function suitably as an electrode, though that is not necessarily a requirement. Surprisingly, the plasma 318 generated in the vessel 250 can be made more uniform, extending through the restricted opening 294 into the processing vessel lumen 304, with less penetration of the electrode 308 into the lumen 300 than has previously been employed. With other arrangements, such as processing a closed-ended vessel, the distal end 314 of the electrode 308 commonly is placed closer to the closed end of the vessel than to its entrance.

Or, the distal end 314 of the electrode 308 can be positioned at the restricted opening 294 or beyond the restricted opening 294, for example within the processing vessel lumen 304. Various expedients can optionally be provided, such as shaping the processing vessel 296 to improve the gas flow through the restricted opening 294.

In yet another contemplated embodiment, the inner electrode 308, as in FIG. 8, can be moved during processing, for example, at first extending into the processing vessel lumen 304, then being withdrawn progressively proximally as the process proceeds. This expedient is particularly contemplated if the vessel 250, under the selected processing conditions, is long, and movement of the inner electrode facilitates more uniform treatment of the interior surface 254.

Using this expedient, the processing conditions, such as the gas feed rate, the vacuum draw rate, the electrical energy applied to the outer electrode 160, the rate of withdrawing the inner electrode 308, or other factors can be varied as the process proceeds, customizing the process to different parts of a vessel to be treated.

Conveniently, the larger opening of the generally tubular vessel 250 to be processed can be placed on a vessel support, as by seating the larger opening 302 of the vessel 250 to be processed on a port of the vessel support. Then the inner electrode 308 can be positioned within the vessel 250 seated on the vessel support before drawing at least a partial vacuum within the lumen 300 of the vessel 250 to be processed.

### V.C. Method of Applying a Lubricity Coating

The present invention pertains to a method of applying a lubricity layer or coating derived from an organosilicon precursor. A "lubricity layer" or any similar term is generally defined as a coating that reduces the frictional resistance of the coated surface, relative to the uncoated surface. As the coated object is a syringe (or syringe part, e.g. syringe barrel), the frictional resistance has two main aspects - breakout force and plunger sliding force.

The plunger sliding force test is a specialized test of the coefficient of sliding friction of the plunger within a syringe, accounting for the fact that the normal force associated with a coefficient of sliding friction as usually measured on a flat surface is addressed by standardizing the fit between the plunger and the tube within which it slides. The parallel force associated with a coefficient of sliding friction as usually measured is comparable to the plunger sliding force measured as described in this specification. Plunger sliding force can be measured, for example, as provided in the ISO 7886-1:1993 test.

Also or instead of the plunger sliding force, the breakout force can be measured. The breakout force is the force required to start a stationary plunger moving within a syringe barrel. The breakout force is measured by applying a force to the plunger that starts at zero or a low value and increases until the plunger begins moving. The breakout force tends to increase with storage of a syringe, after the prefilled syringe plunger has pushed away the intervening lubricant or adhered to the barrel due to decomposition of the lubricant between the plunger and the barrel. The breakout force is the force needed to overcome "sticktion," an industry term for the adhesion between the plunger and barrel that needs to be overcome to break out the plunger and allow it to begin moving.

The break loose force (Fi) and the glide force (Fm) are important performance measures for the effectiveness of a lubricity coating. For Fi and Fm, it is desired to have a low, but not too low value. With too low Fi, which means a too low level of resistance (the extreme being zero), premature/unintended flow may occur, which might e.g. lead to an unintentional premature or uncontrolled discharge of the content of a prefilled syringe.

In order to achieve a sufficient lubricity (e.g. to ensure that a syringe plunger can be moved in the syringe, but to avoid uncontrolled movement of the plunger), the following ranges of Fi and Fm should be advantageously maintained:

Fi: 2.5 to 5 lbs, preferably 2.7 to 4.9 lbs, and in particular 2.9 to 4.7 lbs;
Fm: 2.5 to 8.0 lbs, preferably 3.3 to 7.6 lbs, and in particular 3.3 to 4 lbs.

Further advantageous Fi and Fm values can be found in the Tables of the Examples.

The lubricity coating optionally provides a consistent plunger force that reduces the difference between the break loose force (Fi) and the glide force (Fm).

Some utilities of coating a vessel in whole or in part with a lubricity layer, such as selectively at surfaces contacted in sliding relation to other parts, is to ease the passage of a sliding element such as a piston in a syringe. The vessel can be made of a polymer material such as polyester, for example polyethylene terephthalate (PET), a cyclic olefin copolymer (COC), an olefin such as polypropylene, or other materials. COC is particularly suitable for syringes. Applying a lubricity layer or coating by PECVD can avoid or reduce the need to coat the vessel wall with a sprayed, dipped, or otherwise applied organosilicon or other lubricant that commonly is applied in a far larger quantity than would be deposited by a PECVD process.

A plasma is formed in the vicinity of the substrate.

The precursor optionally can be provided in the substantial absence of nitrogen. The precursor optionally can be provided at less than 1 Torr absolute pressure.

The coating optionally can be applied to the substrate at a thickness of 1 to 5000 nm, or 10 to 1000 nm, or 10 to 500 nm, or 10 to 200 nm, or 20 to 100 nm, or 30 to 1000 nm, or 30 to 500 nm thick. A typical thickness is from 30 to 1000 nm or from 20 to 100 nm, a very typical thickness is from 80 to 150 nm. These ranges are representing average thicknesses, as a certain roughness may enhance the lubricious properties of the lubricity coating. Thus its thickness is advantageously not uniform throughout the coating (see below). However, a uniformly thick lubricity coating is also considered.

The absolute thickness of the lubricity coating at single measurement points can be higher or lower than the range limits of the average thickness, with maximum deviations of preferably +/- 50%, more preferably +/- 25% and even

more preferably +/-15% from the average thickness. However, it typically varies within the thickness ranges given for the average thickness in this description.

The thickness of this and other coatings can be measured, for example, by transmission electron microscopy (TEM). An exemplary TEM image for a lubricity coating is shown in Fig. 21. An exemplary TEM image for an $SiO_2$ barrier coating (described in more detail elsewhere) is shown in Fig. 22.

The TEM can be carried out, for example, as follows. Samples can be prepared for Focused Ion Beam (FIB) cross-sectioning in two ways. Either the samples can be first coated with a thin layer or coating of carbon (50-100nm thick) and then coated with a sputtered layer or coating of platinum (50-100nm thick) using a K575X Emitech coating system, or the samples can be coated directly with the protective sputtered Pt layer. The coated samples can be placed in an FEI FIB200 FIB system. An additional layer or coating of platinum can be FIB-deposited by injection of an oregano-metallic gas while rastering the 30kV gallium ion beam over the area of interest. The area of interest for each sample can be chosen to be a location half way down the length of the syringe barrel. Thin cross sections measuring approximately 15μm ("micrometers") long, 2μm wide and 15μm deep can be extracted from the die surface using a proprietary in-situ FIB lift-out technique. The cross sections can be attached to a 200 mesh copper TEM grid using FIB-deposited platinum. One or two windows in each section, measuring ~ 8μm wide, can be thinned to electron transparency using the gallium ion beam of the FEI FIB.

Cross-sectional image analysis of the prepared samples can be performed utilizing either a Transmission Electron Microscope (TEM), or a Scanning Transmission Electron Microscope (STEM), or both. All imaging data can be recorded digitally. For STEM imaging, the grid with the thinned foils can be transferred to a Hitachi HD2300 dedicated STEM. Scanning transmitted electron images can be acquired at appropriate magnifications in atomic number contrast mode (ZC) and transmitted electron mode (TE). The following instrument settings can be used.

| 1. Instrument | Scanning Transmission Electron Microscope |
|---|---|
| Manufacturer/Model | Hitachi HD2300 |
| Accelerating Voltage | 200kV |
| Objective Aperture | #2 |
| Condenser Lens 1 Setting | 1.672 |
| Condenser Lens 2 Setting | 1.747 |
| Approximate Objective Lens Setting | 5.86 |
| ZC Mode Projector Lens | 1.149 |
| TE Mode Projector Lens | 0.7 |
| Image Acquisition | |
| Pixel Resolution | 1280x960 |
| Acquisition Time | 20sec.(x4) |

For TEM analysis the sample grids can be transferred to a Hitachi HF2000 transmission electron microscope. Transmitted electron images can be acquired at appropriate magnifications. The relevant instrument settings used during image acquisition can be those given below.

| Instrument | Transmission Electron Microscope |
|---|---|
| Manufacturer/Model | Hitachi HF2000 |
| Accelerating Voltage | 200 kV |
| Condenser Lens 1 | 0.78 |
| Condenser Lens 2 | 0 |
| Objective Lens | 6.34 |
| Condenser Lens Aperture | #1 |
| Objective Lens Aperture for imaging | #3 |
| Selective Area Aperture for SAD | N/A |

The substrate is a polymer, for example a polycarbonate polymer, an olefin polymer, a cyclic olefin copolymer, a polypropylene polymer, a polyester polymer, a polyethylene terephthalate polymer or a combination of any two or more of these.

The PECVD optionally can be performed by energizing the gaseous reactant containing the precursor with electrodes powered at a RF frequency as defined above, for example a frequency from 10 kHz to less than 300 MHz, optionally from 1 to 50 MHz, even optionally from 10 to 15 MHz, optionally a frequency of 13.56 MHz.

The plasma can be generated by energizing the gaseous reactant comprising the precursor with electrodes supplied with electric power sufficient to form a lubricity layer. The plasma is generated by energizing the gaseous reactant containing the precursor with electrodes supplied with an electric power of from 0.1 to 25 W, optionally from 1 to 22 W, optionally from 1 to 10 W, even optionally from 1 to 5 W, optionally from 2 to 4 W, for example of 3 W, optionally from 3 to 17 W, even optionally from 5 to 14 W, for example 6 or 7.5 W, optionally from 7 to 11 W, for example of 8 W. The ratio of the electrode power to the plasma volume can be less than 10 W/ml, optionally is from 6 W/ml to 0.1 W/ml, optionally is from 5 W/ml to 0.1 W/ml, optionally is from 4 W/ml to 0.1 W/ml, optionally is from 2 W/ml to 0.2 W/ml. Low power levels are believed by the inventors to be most advantageous (e.g. power levels of from 2 to 3.5 W and the power levels given in the Examples) to prepare a lubricity coating. These power levels are suitable for applying lubricity coatings to syringes having a void volume of 1 to 3 mL in which PECVD plasma is generated. It is contemplated that for larger or smaller objects the power applied should be increased or reduced accordingly to scale the process to the size of the substrate.

In any of embodiment of the method according to the present invention, one preferred combination of process gases includes octamethylcyclotetrasiloxane (OMCTS) or another cyclic siloxane as the precursor, in the presence of oxygen as the oxidizing gas and argon as the carrier gas. Without being bound to the accuracy of this theory, the inventors believe this particular combination is effective for the following reasons.

It is believed that the OMCTS or other cyclic siloxane molecule provides several advantages over other siloxane materials. First, its ring structure results in a less dense coating (as compared to coatings prepared from HMDSO). The molecule also allows selective ionization so that the final structure and chemical composition of the coating can be directly controlled through the application of the plasma power. Other organosilicon molecules are readily ionized (fractured) so that it is more difficult to retain the original structure of the molecule.

Since the addition of Argon gas improves the lubricity performance (see the working examples below), it is believed that additional ionization of the molecule in the presence of Argon contributes to providing lubricity. The Si-O-Si bonds of the molecule have a high bond energy followed by the Si-C, with the C-H bonds being the weakest. Lubricity appears to be achieved when a portion of the C-H bonds are broken. This allows the connecting (cross-linking) of the structure as it grows. Addition of oxygen (with the Argon) is understood to enhance this process. A small amount of oxygen can also provide C-O bonding to which other molecules can bond. The combination of breaking C-H bonds and adding oxygen all at low pressure and power leads to a chemical structure that is solid while providing lubricity.

In a specific embodiment of the present invention, the lubricity can also be influenced by the roughness of the lubricity coating which results from the PECVD process using the precursors and conditions described herein. It has now surprisingly been found in the context of present invention by performing scanning electron microscopy (SEM) and atomic force microscopy (AFM), that a rough, non-continuous OMCTS plasma coating offers lower plunger force (Fi, Fm) than a smooth, continuous OMCTS plasma coating. This is demonstrated by Examples O to V.

While not bound by theory, the inventors assume that this particular effect could be, in part, based on one or both of the following mechanistic effects:

(a) lower surface contact of the plunger with the lubricity coating (e.g. the circular rigid plunger surface contacting only the peaks of a rough coating), either initially and/or throughout the plunger movement, resulting in overall lower contact and thus friction. (b) Upon plunger movement, the plunger causes the initial non-uniform, rough coating to be spread and smoothed into the uncoated "valleys".

The roughness of the lubricity coating is increased with decreasing power (in Watts) energizing the plasma, and by the presence of $O_2$ in the amounts described above. The roughness can be expressed as "RMS roughness" or "RMS" determined by AFM. RMS is the standard deviation of the difference between the highest and lowest points in an AFM image (the difference is designated as "Z"). It is calculated according to the formula:

$$Rq= \{\Sigma(Z1-Zavg)2/N\}-2$$

where Zavg is the average Z value within the image; Z1 is the current value of Z; and N is the number of points in the image.

The RMS range in this specific embodiment is typically from 7 to 20 nm, preferably from 12 to 20 nm. A lower RMS can, however, still lead to satisfying lubricity properties.

One contemplated product optionally can be a syringe having a barrel treated by the method of any one or more of embodiments. Said syringe can either have just a lubricity coating according to present invention, or it can have the lubricity coating and one or more other coatings in addition, e.g. a $SiO_x$ barrier coating under or over the lubricity coating.

## VI. PECVD TREATED VESSELS WITH A BARRIER COATING

Vessels are contemplated having a barrier coating 90 (shown in FIG. 1, for example), which can be an $SiO_x$ coating applied to a thickness of at least 2 nm, or at least 4 nm, or at least 7 nm, or at least 10 nm, or at least 20 nm, or at least 30 nm, or at least 40 nm, or at least 50 nm, or at least 100 nm, or at least 150 nm, or at least 200 nm, or at least 300 nm, or at least 400 nm, or at least 500 nm, or at least 600 nm, or at least 700 nm, or at least 800 nm, or at least 900 nm. The coating can be up to 1000 nm, or at most 900 nm, or at most 800 nm, or at most 700 nm, or at most 600 nm, or at most 500 nm, or at most 400 nm, or at most 300 nm, or at most 200 nm, or at most 100 nm, or at most 90 nm, or at most 80 nm, or at most 70 nm, or at most 60 nm, or at most 50 nm, or at most 40 nm, or at most 30 nm, or at most 20 nm, or at most 10 nm, or at most 5 nm thick. Specific thickness ranges composed of any one of the minimum thicknesses expressed above, plus any equal or greater one of the maximum thicknesses expressed above, are expressly contemplated. The thickness of the $SiO_x$ or other coating can be measured, for example, by transmission electron microscopy (TEM), and its composition can be measured by X-ray photoelectron spectroscopy (XPS).

It is contemplated that the choice of the material to be barred from permeating the coating and the nature of the $SiO_x$ coating applied can affect its barrier efficacy. For example, two examples of material commonly intended to be barred are oxygen and water/water vapor. Materials commonly are a better barrier to one than to the other. This is believed to be so at least in part because oxygen is transmitted through the coating by a different mechanism than water is transmitted.

Oxygen transmission is affected by the physical features of the coating, such as its thickness, the presence of cracks, and other physical details of the coating. Water transmission, on the other hand, is believed to commonly be affected by chemical factors, i.e. the material of which the coating is made, more than physical factors. The inventors also believe that at least one of these chemical factors is a substantial concentration of OH moieties in the coating, which leads to a higher transmission rate of water through the barrier. An $SiO_x$ coating often contains OH moieties, and thus a physically sound coating containing a high proportion of OH moieties is a better barrier to oxygen than to water. A physically sound carbon-based barrier, such as amorphous carbon or diamond-like carbon (DLC) commonly is a better barrier to water than is a $SiO_x$ coating because the carbon-based barrier more commonly has a lower concentration of OH moieties.

Other factors lead to a preference for an $SiO_x$ coating, however, such as its oxygen barrier efficacy and its close chemical resemblance to glass and quartz. Glass and quartz (when used as the base material of a vessel) are two materials long known to present a very high barrier to oxygen and water transmission as well as substantial inertness to many materials commonly carried in vessels. Thus, it is commonly desirable to optimize the water barrier properties such as the water vapor transmission rate (WVTR) of an $SiO_x$ coating, rather than choosing a different or additional type of coating to serve as a water transmission barrier.

Several ways contemplated to improve the WVTR of an $SiO_x$ coating are as follow.

The concentration ratio of organic moieties (carbon and hydrogen compounds) to OH moieties in the deposited coating can be increased. This can be done, for example, by increasing the proportion of oxygen in the feed gases (as by increasing the oxygen feed rate or by lowering the feed rate of one or more other constituents). The lowered incidence of OH moieties is believed to result from increasing the degree of reaction of the oxygen feed with the hydrogen in the silicone source to yield more volatile water in the PECVD exhaust and a lower concentration of OH moieties trapped or incorporated in the coating.

Higher energy can be applied in the PECVD process, either by raising the plasma generation power level, by applying the power for a longer period, or both. An increase in the applied energy must be employed with care when used to coat a plastic tube or other device, as it also has a tendency to distort the vessel being treated, to the extent the tube absorbs the plasma generation power. This is why RF power is contemplated in the context of present application. Distortion of the medical devices can be reduced or eliminated by employing the energy in a series of two or more pulses separated by cooling time, by cooling the vessels while applying energy, by applying the coating in a shorter time (commonly thus making it thinner), by selecting a frequency of the applied coating that is absorbed minimally by the base material selected for being coated, and/or by applying more than one coating, with time in between the respective energy application steps. For example, high power pulsing can be used with a duty cycle of 1 millisecond on, 99 milliseconds off, while continuing to feed the gaseous reactant or process gas. The gaseous reactant or process gas is then the coolant, as it keeps flowing between pulses. Another alternative is to reconfigure the power applicator, as by adding magnets to confine the plasma increase the effective power application (the power that actually results in incremental coating, as opposed to waste power that results in heating or unwanted coating). This expedient results in the application of more coating-formation energy per total Watt-hour of energy applied. See for example U.S. Patent 5,904,952.

An oxygen post-treatment of the coating can be applied to remove OH moieties from the previously-deposited coating. This treatment is also contemplated to remove residual volatile organosilicon compounds or silicones or oxidize the coating to form additional $SiO_x$.

The plastic base material tube can be preheated.

A different volatile source of silicon, such as hexamethyldisilazane (HMDZ), can be used as part or all of the silicone feed. It is contemplated that changing the feed gas to HMDZ will address the problem because this compound has no oxygen moieties in it, as supplied. It is contemplated that one source of OH moieties in the HMDSO-sourced coating is hydrogenation of at least some of the oxygen atoms present in unreacted HMDSO.

A composite coating can be used, such as a carbon-based coating combined with SiOx. This can be done, for example, by changing the reaction conditions or by adding a substituted or unsubstituted hydrocarbon, such as an alkane, alkene, or alkyne, to the feed gas as well as an organosilicon-based compound. See for example U.S. Patent 5,904,952, which states in relevant part: "For example, inclusion of a lower hydrocarbon such as propylene provides carbon moieties and improves most properties of the deposited films (except for light transmission), and bonding analysis indicates the film to be silicon dioxide in nature. Use of methane, methanol, or acetylene, however, produces films that are silicone in nature. The inclusion of a minor amount of gaseous nitrogen to the gas stream provides nitrogen moieties in the deposited films and increases the deposition rate, improves the transmission and reflection optical properties on glass, and varies the index of refraction in response to varied amounts of N2. The addition of nitrous oxide to the gas stream increases the deposition rate and improves the optical properties, but tends to decrease the film hardness." Suitable hydrocarbons include methane, ethane, ethylene, propane, acetylene, or a combination of two or more of these.

A diamond-like carbon (DLC) coating can be formed as the primary or sole coating deposited. This can be done, for example, by changing the reaction conditions or by feeding methane, hydrogen, and helium to a PECVD process. These reaction feeds have no oxygen, so no OH moieties can be formed. For one example, an $SiO_x$ coating can be applied on the interior of a tube or syringe barrel and an outer DLC coating can be applied on the exterior surface of a tube or syringe barrel. Or, the $SiO_x$ and DLC coatings can both be applied as a single layer or coating or plural layers of an interior tube or syringe barrel coating.

Referring to FIG. 1, the barrier or other type of coating 90 reduces the transmission of atmospheric gases into the vessel 80 through its interior surface 88. Or, the barrier or other type of coating 90 reduces the contact of the contents of the vessel 80 with the interior surface 88. The barrier or other type of coating can comprise, for example, $SiO_x$, amorphous (for example, diamond-like) carbon, or a combination of these.

### VII.A. Coated Vessels

The coatings described herein can be applied to a variety of vessels made from plastic, most prominently to plastic syringes. A process is contemplated for applying a lubricity layer or coating on a substrate, for example the interior of the barrel of a syringe, comprising applying one of the described precursors on or in the vicinity of a substrate at a thickness of 1 to 5000 nm, or 10 to 1000 nm, or 10 to 500 nm, or 10 to 200 nm, or 20 to 100 nm, or 30 to 1000 nm, or 30 to 500 nm thick, or 30 to 1000 nm, or 20 to 100 nm, or 80 to 150 nm, and crosslinking or polymerizing (or both) the coating in a PECVD process, to provide a lubricated surface. The coating applied by this process is also contemplated to be new.

A coating of $Si_wO_xC_yH_z$ as defined in the Definition Section can have utility as a hydrophobic layer. Coatings of this kind are contemplated to be hydrophobic, independent of whether they function as lubricity layers. A coating or treatment is defined as "hydrophobic" if it lowers the wetting tension of a surface, compared to the corresponding uncoated or untreated surface. Hydrophobicity is thus a function of both the untreated substrate and the treatment.

The degree of hydrophobicity of a coating can be varied by varying its composition, properties, or deposition method. For example, a coating of SiOx having little or no hydrocarbon content is more hydrophilic than a coating of $Si_wO_xC_yH_z$ as defined in the Definition Section. Generally speaking, the higher the C-$H_x$ (e.g. CH, $CH_2$, or $CH_3$) moiety content of the coating, either by weight, volume, or molarity, relative to its silicon content, the more hydrophobic the coating.

A hydrophobic layer or coating can be very thin, having a thickness of at least 4 nm, or at least 7 nm, or at least 10 nm, or at least 20 nm, or at least 30 nm, or at least 40 nm, or at least 50 nm, or at least 100 nm, or at least 150 nm, or at least 200 nm, or at least 300 nm, or at least 400 nm, or at least 500 nm, or at least 600 nm, or at least 700 nm, or at least 800 nm, or at least 900 nm. The coating can be up to 1000 nm, or at most 900 nm, or at most 800 nm, or at most 700 nm, or at most 600 nm, or at most 500 nm, or at most 400 nm, or at most 300 nm, or at most 200 nm, or at most 100 nm, or at most 90 nm, or at most 80 nm, or at most 70 nm, or at most 60 nm, or at most 50 nm, or at most 40 nm, or at most 30 nm, or at most 20 nm, or at most 10 nm, or at most 5 nm thick. Specific thickness ranges composed of any one of the minimum thicknesses expressed above, plus any equal or greater one of the maximum thicknesses expressed above, are expressly contemplated.

One utility for such a hydrophobic layer or coating is to isolate a thermoplastic tube wall, made for example of polyethylene terephthalate (PET), from blood collected within the tube. The hydrophobic layer or coating can be applied on top of a hydrophilic $SiO_x$ coating on the internal surface of the tube. The $SiO_x$ coating increases the barrier properties of the thermoplastic tube and the hydrophobic layer or coating changes the surface energy of blood contact surface with the tube wall. The hydrophobic layer or coating can be made by providing a precursor selected from those identified in this specification. For example, the hydrophobic layer or coating precursor can comprise hexamethyldisiloxane (HMDSO)

or octamethylcyclotetrasiloxane (OMCTS).

A lubricity layer can be applied as a subsequent coating after applying an $SiO_x$ barrier coating to the interior surface 88 of the vessel 80 .

Optionally, after the lubricity layer or coating is applied, it can be post-cured after the PECVD process. Radiation curing approaches, including UV-initiated (free radial or cationic), electron-beam (E-beam), and thermal as described in Development Of Novel Cycloaliphatic Siloxanes For Thermal And UV-Curable Applications (Ruby Chakraborty Dissertation, can 2008) be utilized.

A lubricity layer is particularly contemplated for the internal surface of a syringe barrel as further described below. A lubricated internal surface of a syringe barrel can reduce the plunger sliding force needed to advance a plunger in the barrel during operation of a syringe, or the breakout force to start a plunger moving after the prefilled syringe plunger has pushed away the intervening lubricant or adhered to the barrel, for example due to decomposition of the lubricant between the plunger and the barrel. As explained elsewhere in this specification, a lubricity layer or coating also can be applied to the interior surface 88 of the vessel 80 to improve adhesion of a subsequent coating of $SiO_x$.

Thus, the coating 90 can comprise a layer or coating of $SiO_x$ and/or a hydrophobic layer in addition to the lubricity layer or coating made according to the present invention, being characterized as defined in the Definition Section. The lubricity layer or coating and/or hydrophobic layer or coating of $Si_wO_xC_yH_z$ can be deposited between the layer or coating of $SiO_x$ and the interior surface of the vessel. Or, the layer or coating of $SiO_x$ can be deposited between the lubricity layer or coating and/or hydrophobic layer or coating and the interior surface of the vessel. Or, three or more layers, either alternating or graduated between these two coating compositions: (1) a layer or coating of $SiO_x$ and (2) the lubricity layer or coating and/or hydrophobic layer; can also be used. The layer or coating of $SiO_x$ can be deposited adjacent to the lubricity layer or coating and/or hydrophobic layer or coating or remotely, with at least one intervening layer or coating of another material. The layer or coating of $SiO_x$ can be deposited adjacent to the interior surface of the vessel. Or, the lubricity layer or coating and/or hydrophobic layer or coating can be deposited adjacent to the interior surface of the vessel.

Another expedient contemplated here, for adjacent layers of $SiO_x$ and a lubricity layer or coating and/or hydrophobic layer, is a graded composite of $Si_wO_xC_yH_z$, as defined in the Definition Section. A graded composite can be separate layers of a lubricity layer or coating and/or hydrophobic layer or coating and $SiO_x$ with a transition or interface of intermediate composition between them, or separate layers of a lubricity layer or coating and/or hydrophobic layer or coating and $SiO_x$ with an intermediate distinct layer or coating of intermediate composition between them, or a single layer or coating that changes continuously or in steps from a composition of a lubricity layer or coating and/or hydrophobic layer or coating to a composition more like $SiO_x$, going through the coating in a normal direction.

The grade in the graded composite can go in either direction. For example, the lubricity layer or coating and/or hydrophobic layer or coating can be applied directly to the substrate and graduate to a composition further from the surface of $SiO_x$. Or, the composition of $SiO_x$ can be applied directly to the substrate and graduate to a composition further from the surface of a lubricity layer or coating and/or hydrophobic layer. A graduated coating is particularly contemplated if a coating of one composition is better for adhering to the substrate than the other, in which case the better-adhering composition can, for example, be applied directly to the substrate. It is contemplated that the more distant portions of the graded coating can be less compatible with the substrate than the adjacent portions of the graded coating, since at any point the coating is changing gradually in properties, so adjacent portions at nearly the same depth of the coating have nearly identical composition, and more widely physically separated portions at substantially different depths can have more diverse properties. It is also contemplated that a coating portion that forms a better barrier against transfer of material to or from the substrate can be directly against the substrate, to prevent the more remote coating portion that forms a poorer barrier from being contaminated with the material intended to be barred or impeded by the barrier.

The coating, instead of being graded, optionally can have sharp transitions between one layer or coating and the next, without a substantial gradient of composition. Such coatings can be made, for example, by providing the gases to produce a layer or coating as a steady state flow in a non-plasma state, then energizing the system with a brief plasma discharge to form a coating on the substrate. If a subsequent coating is to be applied, the gases for the previous coating are cleared out and the gases for the next coating are applied in a steady-state fashion before energizing the plasma and again forming a distinct layer or coating on the surface of the substrate or its outermost previous coating, with little if any gradual transition at the interface.

**VII.B. Syringes**

The foregoing description has largely addressed applying a barrier coating to a tube with one permanently closed end, such as a blood collection tube or, more generally, a specimen receiving tube 80. The apparatus is not limited to such a device. It is also suitable to perform the method of to the present invention.

A vessel which can be provided with a lubricity coating by the method of the present invention, shown in FIG. 4, is a syringe barrel 250 for a medical syringe 252. Such syringes 252 are sometimes supplied prefilled with saline

solution, a pharmaceutical preparation, or the like for use in medical techniques. Pre-filled syringes 252 are also contemplated to benefit from an $SiO_x$ barrier or other type of coating on the interior surface 254 to keep the contents of the prefilled syringe 252 out of contact with the plastic of the syringe, for example of the syringe barrel 250 during storage. The barrier or other type of coating can be used to avoid leaching components of the plastic into the contents of the barrel through the interior surface 254.

A syringe barrel 250 as molded commonly can be open at both the back end 256, to receive a plunger 258, and at the front end 260, to receive a hypodermic needle, a nozzle, or tubing for dispensing the contents of the syringe 252 or for receiving material into the syringe 252. But the front end 260 can optionally be capped and the plunger 258 optionally can be fitted in place before the prefilled syringe 252 is used, closing the barrel 250 at both ends. A cap 262 can be installed either for the purpose of processing the syringe barrel 250 or assembled syringe, or to remain in place during storage of the prefilled syringe 252, up to the time the cap 262 is removed and (optionally) a hypodermic needle or other delivery conduit is fitted on the front end 260 to prepare the syringe 252 for use.

Another example of a suitable vessel, shown in FIGS 24-26, is a syringe including a plunger, a syringe barrel, and a staked needle (a "staked needle syringe"). The needle is hollow with a typical size ranging from 18-29 gauge. The syringe barrel has an interior surface slidably receiving the plunger. The staked needle may be affixed to the syringe during the injection molding of the syringe or may be assembled to the formed syringe using an adhesive. A cover is placed over the staked needle to seal the syringe assembly. The syringe assembly must be sealed so that a vacuum can be maintained within the syringe to enable the PECVD coating process.

The needle of the staked needle syringe has an outside surface, a delivery outlet at one end, a base at the other end, and an internal passage extending from the base to the delivery outlet. The barrel has a, for example generally cylindrical, interior surface defining a lumen. The barrel also has a front passage molded around and in fluid-sealing contact with the outside surface of the needle.

The "staked needle" syringe optionally can further include a cap configured to isolate the delivery outlet of the needle from ambient air.

The cap of any "staked needle" syringe optionally can further include a lumen having an opening defined by a rim and sized to receive the delivery outlet, and the rim can be seatable against an exterior portion of the barrel.

In any "staked needle" syringe, the barrel optionally can further include a generally hemispheric interior surface portion adjacent to its front passage.

In any "staked needle" syringe, the base of the needle optionally can be at least substantially flush with the hemispheric interior surface portion of the barrel.

The "staked needle" syringe optionally can further include a PECVD-applied barrier coating on at least the hemispheric interior surface portion of the barrel.

In any "staked needle" syringe, the barrier coating optionally can extend over at least a portion of the generally cylindrical interior surface portion of the barrel.

In any "staked needle" syringe, the barrier coating optionally can form a barrier between the base of the needle and the generally cylindrical interior surface portion of the barrel.

In the "staked needle" syringe of FIG. 24, the cap 7126 is held in place on the nose 71110 of the syringe 7120 by a conventional Luer lock arrangement. The tapered nose 71110 of the syringe mates with a corresponding tapered throat 71112 of the cap 7126, and the syringe has a collar 71114 with an interior thread 71116 receiving the dogs 71118 and 71120 of the cap 7126 to lock the tapers 71110 and 71112 together. The cap 7126 can be substantially rigid.

Referring now to FIG. 25, a variation on the syringe barrel 71122 and cap 71124 of the "staked needle" syringe is shown. In this syringe, the cap 71124 includes a flexible lip seal 7172 at its base to form a moisture-tight seal with the syringe barrel 71122.

Optionally in the "staked needle" syringes of FIGS. 24 and 25, the caps 7126 and 71124 can withstand vacuum during the PECVD coating process. The caps 7126 and 71124 can be made of LDPE. Alternative rigid plastic materials can be used as well, for example polypropylene. Additional sealing elements can be provided as well.

In another option of the "staked needle" syringe, illustrated in FIG. 26, the cap 71126 is flexible, and is designed to seal around the top end of the syringe 7120. A deformable material - like a rubber or a thermoplastic elastomer (TPE) can be used for the cap 71126. Preferred TPE materials include fluoroelastomers, and in particular, medical grade fluoroelastomers. Examples include VITON® and TECHNOFLON®. VITON® is preferable in some cases. An example of a suitable rubber is EPDM rubber.

During molding, in certain "staked needle" syringes (illustrated for example in FIG. 26) a small amount of the cap material 71132 will be drawn into the tip or delivery outlet 7134 of the needle 7122 to create a seal. The material 71132 should have a durometer such as to permit an appropriate amount of material to be drawn into the needle 7122, and to cause the material drawn into the needle 7122 to continue to adhere to the cap 71126 when it is removed, unplugging the needle 7122 for use.

In other "staked needle" syringes, the cap material 71132 can block the delivery outlet 7134 of the needle 7122 without being drawn into the delivery outlet 7134. Suitable material selection to accomplish the desired purposes is

within the capabilities of a person of ordinary skill in the art.

An additional seal can be created by coupling an undercut 71134 formed in the syringe barrel and projections 71138 in the interior of the cap 71126, defining a coupling to retain the cap 71126. Alternative "staked needle" syringescan include either one or both of the seals described above.

Optionally, with reference to FIG. 25, the cap 71124 can have a base 7168 and a coupling 7170 configured for securing the cap 7126 in a seated position on the barrel. Alternatively or in addition, a flexible lip seal 7172 can optionally be provided at the base 7168 of the cap 71124 for seating against the barrel 71122 when the cap 71124 is secured on the barrel 71122.

Optionally, referring now to FIG. 26, the delivery outlet 7134 of the needle 7122 can be seated on the cap 71126 when the cap 7126 is secured on the barrel. This expedient is useful for sealing the delivery outlet 7134 against the ingress or egress of air or other fluids, when that is desired.

Optionally, in the "staked needle" syringe the coupling 7170 can include a detent or groove 7174 on one of the barrel 71122 and the cap 71124 and a projection or rib 76 on the other of the barrel 71122 and the cap 71124, the projection 7176 being adapted to mate with the detent 7174 when the cap 7126 is in its seated position on the barrel. In one contemplated syringe, a detent 7174 can be on the barrel and a projection 7176 can be on the cap 7126. In another contemplated syringe, a detent 7174 can be on the cap 7126 and a projection 7176 can be on the barrel. In yet another contemplated syringe, a first detent 7174 can be on the barrel and a first projection 7176 mating with the detent 7174 can be on the cap 7126, while a second detent 7175 can be on the cap 7126 and the mating second projection 7177 can be on the barrel. A detent 7174 can be molded in the syringe barrel as an undercut by incorporating side draws such as 7192 and 7194 in the mold. The detents 7174 mate with the complementary projections 7176 to assemble (snap) the cap 7126 onto the syringe 7120. In this respect the cap 7126 is desirably flexible enough to allow sufficient deformation for a snapping engagement of the detents 7174 and projections 7176.

The caps in the "staked needle" syringe such as 7126, 71124, and 71126 can be injection molded or otherwise formed, for example from thermoplastic material. Several examples of suitable thermoplastic material are a polyolefin, for example a cyclic olefin polymer (COP), a cyclic olefin copolymer (COC), polypropylene, or polyethylene. The cap 7126 can contain or be made of a thermoplastic elastomer (TPE) or other elastomeric material. The cap 7126 can also be made of polyethylene terephthalate (PET), polycarbonate resin, or any other suitable material. Optionally, a material for the cap 7126 can be selected that can withstand vacuum and maintain sterility within the syringe 7120.

Typically, when the syringe barrel is coated, the PECVD coating methods described herein are performed such that the coated substrate surface is part or all of the inner surface of the barrel, the gas for the PECVD reaction fills the interior lumen of the barrel, and the plasma is generated within part or all of the interior lumen of the barrel.

### VII.B.1. Syringe Having Barrel Coated With Lubricity Layer

A syringe having a lubricity layer can be made by the process of the present invention.

A precursor is provided as defined above, optionally by vaporizing the precursor and providing it in the vicinity of the substrate.

A plasma is formed in the vicinity of the substrate. Optionally, the precursor is provided in the substantial absence of nitrogen. Optionally, the precursor is provided at less than 1 Torr absolute pressure. Optionally, the coating has an average thickness of 1 to 5000 nm, or 10 to 1000 nm, or 10 to 500 nm, or 10 to 200 nm, or 20 to 100 nm, or 30 to 1000 nm, or 30 to 500 nm, or 30 to 1000 nm, or 20 to 100 nm, or 80 to 150 nm thick. The substrate is a polymer, optionally a polycarbonate polymer, optionally an olefin polymer, optionally a cyclic olefin copolymer, optionally a polypropylene polymer, optionally a polyester polymer, optionally a polyethylene terephthalate polymer. COC is particularly considered for syringes and syringe barrels.

Optionally, the plasma is generated by energizing the gaseous reactant containing the precursor with electrodes powered, for example, at a RF frequency as defined above, for example a frequency of from 10 kHz to less than 300 MHz, optionally from 1 to 50 MHz, even optionally from 10 to 15 MHz, optionally a frequency of 13.56 MHz.

The plasma is generated by energizing the gaseous reactant containing the precursor with electrodes supplied with an electric power of from 0.1 to 25 W, optionally from 1 to 22 W, optionally from 3 to 17 W, even optionally from 5 to 14 W, optionally from 7 to 11 W, optionally 8 W. The ratio of the electrode power to the plasma volume can be less than 10 W/ml, optionally is from 6 W/ml to 0.1 W/ml, optionally is from 5 W/ml to 0.1 W/ml, optionally is from 4 W/ml to 0.1 W/ml, optionally is from 2 W/ml to 0.2 W/ml. Low power levels are believed by the inventors to be most advantageous (e.g. power levels of from 2 to 3.5 W and the power levels given in the Examples) to prepare a lubricity coating. These power levels are suitable for applying lubricity layers to syringes having a void volume of 1 to 3 mL in which PECVD plasma is generated. It is contemplated that for larger or smaller objects the power applied should be increased or reduced accordingly to scale the process to the size of the substrate.

A lubricity coating is produced on the inner wall of a syringe barrel by a PECVD process using the following materials and conditions. A cyclic precursor is optionally employed, selected from a monocyclic siloxane, a polycyclic

siloxane, or a combination of two or more of these, as defined elsewhere in this specification for lubricity layers. One example of a suitable cyclic precursor comprises octamethylcyclotetrasiloxane (OMCTS), optionally mixed with other precursor materials in any proportion. Optionally, the cyclic precursor consists essentially of octamethycyclotetrasiloxane (OMCTS), meaning that other precursors can be present in amounts which do not change the basic and novel properties of the resulting lubricity layer, i.e. its reduction of the plunger sliding force or breakout force of the coated surface.

A sufficient plasma generation power input, for example any power level successfully used in one or more working examples of this specification or described in the specification, is provided to induce coating formation.

The materials and conditions employed are effective to reduce the syringe plunger sliding force or breakout force moving through the syringe barrel at least 25 percent, alternatively at least 45 percent, alternatively at least 60 percent, alternatively greater than 60 percent, relative to an uncoated syringe barrel. Ranges of plunger sliding force or breakout force reduction of from 20 to 95 percent, alternatively from 30 to 80 percent, alternatively from 40 to 75 percent, alternatively from 60 to 70 percent, are contemplated.

A syringe including a plunger, a syringe barrel, and a lubricity layer is made by the method according to the present invention. The syringe barrel includes an interior surface receiving the plunger for sliding. The lubricity layer or coating is disposed on part or all of the interior surface of the syringe barrel. The lubricity layer or coating optionally can be less than 1000 nm thick and effective to reduce the breakout force or the plunger sliding force necessary to move the plunger within the barrel. Reducing the plunger sliding force is alternatively expressed as reducing the coefficient of sliding friction of the plunger within the barrel or reducing the plunger force; these terms are regarded as having the same meaning in this specification.

The syringe comprises a plunger and a syringe barrel. The syringe barrel has an interior surface receiving the plunger for sliding. The interior surface of the syringe barrel further receives a lubricity layer or coating by applying the method of the present invention. The lubricity layer or coating is less than 1000 nm thick, optionally less than 500 nm thick, optionally less than 200 nm thick, optionally less than 100 nm thick, optionally less than 50 nm thick, and is effective to reduce the breakout force necessary to overcome adhesion of the plunger after storage or the plunger sliding force necessary to move the plunger within the barrel after it has broken away. The lubricity layer or coating is characterized by having a plunger sliding force or breakout force lower than that of the uncoated surface.

Any of the above precursors of any type can be used alone or in combinations of two or more of them to provide a lubricity layer.

Yet another expedient contemplated for any coating or coatings described here is a coating that is not uniformly applied over the entire interior 88 of a vessel. This expedient is particularly contemplated for a syringe barrel, in which a lubricity layer or coating might be provided on part or all of the cylindrical portion of the barrel, where the plunger or piston or closure slides, and not elsewhere.

Optionally, the precursor can be provided in the presence, substantial absence, or absence of nitrogen.

Optionally, the precursor can be provided at less than 1 Torr absolute pressure.

Optionally, the precursor can be provided to the vicinity of a plasma emission.

In any of the above embodiments, the substrate is a polymer, for example one or more of a polycarbonate polymer, an olefin polymer (for example a cyclic olefin copolymer or a polypropylene polymer), or a polyester polymer (for example, a polyethylene terephthalate polymer).

In any of the above embodiments, the plasma is generated by energizing the gaseous reactant containing the precursor with electrodes supplied with sufficient electric power to generate a lubricity layer. The plasma is generated by energizing the gaseous reactant containing the precursor with electrodes supplied with an electric power of from 0.1 to 25 W, optionally from 1 to 22 W, optionally from 3 to 17 W, even optionally from 5 to 14 W, optionally from 7 to 11 W, optionally 8 W. The ratio of the electrode power to the plasma volume can be less than 10 W/ml, optionally is from 6 W/ml to 0.1 W/ml, optionally is from 5 W/ml to 0.1 W/ml, optionally is from 4 W/ml to 0.1 W/ml, optionally from 2 W/ml to 0.2 W/ml. Low power levels are believed by the inventors to be most advantageous (e.g. power levels of from 2 to 3.5 W and the power levels given in the Examples) to prepare a lubricity coating. These power levels are suitable for applying lubricity layers to syringes having a void volume of 1 to 3 mL in which PECVD plasma is generated. It is contemplated that for larger or smaller objects the power applied should be increased or reduced accordingly to scale the process to the size of the substrate.

### VII.B.1.a. $SiO_x$ Barrier, Lubricity Layer, Surface Treatment

#### Surface treatment

A syringe comprising a barrel defining a lumen and having an interior surface slidably receiving a plunger, i.e. receiving a plunger for sliding contact to the interior surface, is coated by the method of the present invention.

The syringe barrel is made of thermoplastic base material.

Optionally, the interior surface of the barrel is coated with an $SiO_x$ barrier layer or coating as described elsewhere

in this specification.

A lubricity layer or coating is applied to part or all the barrel interior surface, the plunger, or both, or to the previously applied $SiO_x$ barrier layer. The lubricity layer or coating can be provided, applied, and cured as set out in elsewhere in this specification.

The lubricity layer or coating is applied, in any embodiment, by PECVD. The lubricity layer or coating is deposited from an organosilicon precursor, and is less than 1000 nm thick.

In the present embodiment, a surface treatment is carried out on the lubricity layer or coating in an amount effective to reduce the leaching or extractables of the lubricity layer, the thermoplastic base material, or both. The treated surface can thus act as a solute retainer. This surface treatment can result in a skin coating, e.g. a skin coating which is at least 1 nm thick and less than 100 nm thick, or less than 50 nm thick, or less than 40 nm thick, or less than 30 nm thick, or less than 20 nm thick, or less than 10 nm thick, or less than 5 nm thick, or less than 3 nm thick, or less than 2 nm thick, or less than 1 nm thick, or less than 0.5 nm thick.

As used herein, "leaching" refers to material transferred out of a substrate, such as a vessel wall, into the contents of a vessel, for example a syringe. Commonly, leachables are measured by storing the vessel filled with intended contents, then analyzing the contents to determine what material leached from the vessel wall into the intended contents. "Extraction" refers to material removed from a substrate by introducing a solvent or dispersion medium other than the intended contents of the vessel, to determine what material can be removed from the substrate into the extraction medium under the conditions of the test.

The surface treatment resulting in a solute retainer optionally can be a $SiO_x$ layer or coating as previously defined in this specification or a hydrophobic layer, characterized as defined in the Definition Section. In one embodiment, the surface treatment can be applied by PECVD deposit of $SiO_x$ or a hydrophobic layer. Optionally, the surface treatment can be applied using higher power or stronger oxidation conditions than used for creating the lubricity layer, or both, thus providing a harder, thinner, continuous solute retainer. Surface treatment can be less than 100 nm deep, optionally less than 50 nm deep, optionally less than 40 nm deep, optionally less than 30 nm deep, optionally less than 20 nm deep, optionally less than 10 nm deep, optionally less than 5 nm deep, optionally less than 3 nm deep, optionally less than 1 nm deep, optionally less than 0.5 nm deep, optionally between 0.1 and 50 nm deep in the lubricity layer.

The solute retainer is contemplated to provide low solute leaching performance to the underlying lubricity and other layers, including the substrate, as required. This retainer would only need to be a solute retainer to large solute molecules and oligomers (for example siloxane monomers such as HMDSO, OMCTS, their fragments and mobile oligomers derived from lubricants, for example a "leachables retainer") and not a gas ($O_2$/$N_2$/$CO_2$/water vapor) barrier layer. A solute retainer can, however, also be a gas barrier (e.g. the SiOx coating described herein). One can create a good leachable retainer without gas barrier performance, either by vacuum or atmospheric-based PECVD processes. It is desirable that the "leachables barrier" will be sufficiently thin that, upon syringe plunger movement, the plunger will readily penetrate the "solute retainer" exposing the sliding plunger nipple to the lubricity layer or coating immediately below to form a lubricated surface having a lower plunger sliding force or breakout force than the untreated substrate.

The surface treatment can be performed by oxidizing the surface of a previously applied lubricity layer, as by exposing the surface to oxygen in a plasma environment. The plasma environment described in this specification for forming $SiO_x$ coatings can be used. Or, atmospheric plasma conditions can be employed in an oxygen-rich environment.

The lubricity layer or coating and solute retainer, however formed, optionally can be cured at the same time. The lubricity layer or coating can be at least partially cured, optionally fully cured, after which the surface treatment can be provided, applied, and the solute retainer can be cured.

The lubricity layer or coating and solute retainer are composed, and present in relative amounts, effective to provide a breakout force, plunger sliding force, or both that is less than the corresponding force required in the absence of the lubricity layer or coating and surface treatment. In other words, the thickness and composition of the solute retainer are such as to reduce the leaching of material from the lubricity layer or coating into the contents of the syringe, while allowing the underlying lubricity layer or coating to lubricate the plunger. It is contemplated that the solute retainer will break away easily and be thin enough that the lubricity layer or coating will still function to lubricate the plunger when it is moved.

In one contemplated embodiment, the lubricity and surface treatments can be applied on the barrel interior surface. In another contemplated embodiment, the lubricity and surface treatments can be applied on the plunger. In still another contemplated embodiment, the lubricity and surface treatments can be applied both on the barrel interior surface and on the plunger. In any of these embodiments, the optional $SiO_x$ barrier layer or coating on the interior of the syringe barrel can either be present or absent.

One configuration contemplated is a plural-layer, e.g. a 3-layer, configuration applied to the inside surface of a syringe barrel. Layer or coating 1 can be an $SiO_x$ gas barrier. Layer or coating 2 is a lubricity layer or coating made according to the present invention. Layer or coating 3 is a subsequent solute retainer.

Certain of these plural-layer coatings are contemplated to have one or more of the following optional advantages, at least to some degree. They can address the reported difficulty of handling silicone, since the solute retainer can

confine the interior silicone and prevent if from migrating into the contents of the syringe or elsewhere, resulting in fewer silicone particles in the deliverable contents of the syringe and less opportunity for interaction between the lubricity layer or coating and the contents of the syringe. They can also address the issue of migration of the lubricity layer or coating away from the point of lubrication, improving the lubricity of the interface between the syringe barrel and the plunger. For example, the break-free force can be reduced and the drag on the moving plunger can be reduced, or optionally both.

It is contemplated that when the solute retainer is broken, the solute retainer will continue to adhere to the lubricity layer or coating and the syringe barrel, which can inhibit any particles from being entrained in the deliverable contents of the syringe.

Certain of these coatings will also provide manufacturing advantages, particularly if the barrier coating, lubricity layer or coating and surface treatment are applied in the same apparatus, for example the illustrated PECVD apparatus. Optionally, the $SiO_x$ barrier coating, lubricity layer, and surface treatment can all be applied in one PECVD apparatus, thus greatly reducing the amount of handling necessary.

Further advantages can be obtained by forming the barrier coating, lubricity layer, and solute retainer using the same precursors and varying the process. For example, an $SiO_x$ gas barrier layer or coating can be applied using an OMCTS precursor under high power/high $O_2$ conditions, followed by applying a lubricity layer or coating applied using an OMCTS precursor under low power, finishing with a surface treatment using an OMCTS precursor under intermediate power and oxygen.

**VII.B.2. Plunger with Lubricity layer or coating Interfacing With Side Face**

A plunger for a syringe made according to the method of the present invention may include a piston, a lubricity layer, and a push rod. The piston has a front face, a generally cylindrical side face, and a back portion. The side face is configured to movably seat within a syringe barrel. The lubricity layer or coating interfaces with the side face. The push rod engages the back portion of the piston and is configured for advancing the piston in a syringe barrel.

**VII.B.3.a Two Piece Syringe and Luer Fitting**

Another syringe includes a plunger, a syringe barrel, and a Luer fitting. The syringe includes a barrel having an interior surface receiving the plunger for sliding. The Luer fitting includes a Luer taper having an internal passage defined by an internal surface. The Luer fitting is formed as a separate piece from the syringe barrel and joined to the syringe barrel by a coupling. The internal passage of the Luer taper optionally has a barrier coating of $SiO_x$.

**VII.B.3.b Staked Needle Syringe**

Another syringe includes a plunger, a syringe barrel, and a staked needle (a "staked needle syringe"). The needle is hollow with a typical size ranging from 18-29 gauge. The syringe barrel has an interior surface slidably receiving the plunger. The staked needle may be affixed to the syringe during the injection molding of the syringe or may be assembled to the formed syringe using an adhesive. A cover is placed over the staked needle to seal the syringe assembly. The syringe assembly must be sealed so that a vacuum can be maintained within the syringe to enable the PECVD coating process.

**VII.B.4. Lubricity layer or coating In general**

A lubricity layer or coating deposited by PECVD from a feed gas comprising an organosilicon precursor, optionally a linear siloxane, a linear silazane, a monocyclic siloxane, a monocyclic silazane, a polycyclic siloxane, a polycyclic silazane, or any combination of two or more of these can have a density between 1.25 and 1.65 $g/cm^3$, optionally between 1.35 and 1.55 $g/cm^3$, optionally between 1.4 and 1.5 $g/cm^3$, optionally between 1.44 and 1.48 $g/cm^3$ as determined by X-ray reflectivity (XRR).

Optionally, the feed gas comprises a monocyclic siloxane, a monocyclic silazane, a polycyclic siloxane, a polycyclic silazane, or any combination of two or more of these, for example a monocyclic siloxane, a monocyclic silazane, or any combination of two or more of these, for example octamethylcyclotetrasiloxane.

The lubricity layer or coating can have an average thickness measured by transmission electron microscopy (TEM) of from 1 to 5000 nm, or 10 to 1000 nm, or 10 to 200 nm, or 20 to 100 nm, or 30 to 1000 nm, or 30 to 500 nm thick. Preferred ranges are from 30 to 1000 nm and from 20 to 100 nm, and a particularly preferred range is from 80 to 150 nm. The absolute thickness of the coating at single measurement points can be higher or lower than the range limits of the average thickness. However, it typically varies within the thickness ranges given for the average thickness.

A lubricity layer or coating deposited by PECVD from a feed gas comprising a monocyclic siloxane, a monocyclic silazane, a polycyclic siloxane, a polycyclic silazane, or any combination of two or more of these may have an atomic

concentration of carbon, normalized to 100% of carbon, oxygen, and silicon, as determined by X-ray photoelectron spectroscopy (XPS), greater than the atomic concentration of carbon in the atomic formula for the feed gas.

Optionally, the atomic concentration of carbon increases by from 1 to 80 atomic percent (as calculated and based on the XPS conditions in Example 15 of EP 2 251 455), alternatively from 10 to 70 atomic percent, alternatively from 20 to 60 atomic percent, alternatively from 30 to 50 atomic percent, alternatively from 35 to 45 atomic percent, alternatively from 37 to 41 atomic percent in relation to the atomic concentration of carbon in the organosilicon precursor when a lubricity coating is made.

A lubricity layer or coating deposited by PECVD from a feed gas comprising a monocyclic siloxane, a monocyclic silazane, a polycyclic siloxane, a polycyclic silazane, or any combination of two or more of these may have an atomic concentration of silicon, normalized to 100% of carbon, oxygen, and silicon, as determined by X-ray photoelectron spectroscopy (XPS), less than the atomic concentration of silicon in the atomic formula for the feed gas. See Example 15 of EP 2 251 455.

Optionally, the atomic concentration of silicon decreases by from 1 to 80 atomic percent (as calculated and based on the XPS conditions in Example 15 of EP 2251 455), alternatively from 10 to 70 atomic percent, alternatively from 20 to 60 atomic percent, alternatively from 30 to 55 atomic percent, alternatively from 40 to 50 atomic percent, alternatively from 42 to 46 atomic percent.

### VII.C. Use of the coated Vessels

A coated vessel or container as prepared according to the method of the present invention can be used for reception and/or storage and/or delivery of a compound or composition. The compound or composition can be sensitive, for example air-sensitive, oxygen-sensitive, sensitive to humidity and/or sensitive to mechanical influences. It can be a biologically active compound or composition, for example a medicament like insulin or a composition comprising insulin. In another aspect, it can be a biological fluid, optionally a bodily fluid, for example blood or a blood fraction. In certain aspects, the compound or composition is a product to be administrated to a subject in need thereof, for example a product to be injected, like blood (as in transfusion of blood from a donor to a recipient or reintroduction of blood from a patient back to the patient) or insulin.

A coated vessel as prepared according to the method of the present invention can further be used for protecting a compound or composition contained in its interior space against mechanical and/or chemical effects of the surface of the uncoated vessel material. For example, it can be used for preventing or reducing precipitation and/or clotting or platelet activation of the compound or a component of the composition, for example insulin precipitation or blood clotting or platelet activation.

It can further be used for protecting a compound or composition contained in its interior against the environment outside of the vessel, for example by preventing or reducing the entry of one or more compounds from the environment surrounding the vessel into the interior space of the vessel. Such environmental compound can be a gas or liquid, for example an atmospheric gas or liquid containing oxygen, air, and/or water vapor.

A coated vessel as described herein can also be evacuated and stored in an evacuated state. For example, the coating allows better maintenance of the vacuum in comparison to a corresponding uncoated vessel.

## COMMON CONDITIONS FOR ALL EMBODIMENTS

In any embodiment contemplated here, many common conditions can be used, for example any of the following, in any combination. Alternatively, any different conditions described elsewhere in this specification or claims can be employed.

## I. GASEOUS REACTANT OR PROCESS GAS LIMITATIONS OF ANY EMBODIMENT

### I.A Deposition conditions of any embodiment

The plasma for PECVD can be generated at reduced pressure and the reduced pressure can be less than 300 mTorr, optionally less than 200 mTorr, even optionally less than 100 mTorr. The physical and chemical properties of the coating can be set by setting the ratio of $O_2$ to the organosilicon precursor in the gaseous reactant, and by setting the electric power used for generating the plasma.

### I.B. Precursor of any embodiment

The organosilicon precursor has been described elsewhere in this description.

The organosilicon compound can in certain aspects when a lubricity coating is formed comprise octamethylcyclotetrasiloxane (OMCTS). The organosilicon compound for any embodiment of said certain aspects can consist essentially of octamethycyclotetrasiloxane (OMCTS). The organosilicon compound can in certain aspects, particularly when a barrier coating is formed, be or comprise hexamethyldisiloxane.

The reaction gas can also include a hydrocarbon. The hydrocarbon can comprise methane, ethane, ethylene, propane, acetylene, or a combination of two or more of these.

The organosilicon precursor can be delivered at a rate of equal to or less than 6 sccm, optionally equal to or less than 2.5 sccm, optionally equal to or less than 1.5 sccm, optionally equal to or less than 1.25 sccm. Larger vessels or other changes in conditions or scale may require more or less of the precursor. The precursor can be provided at less than 1 Torr absolute pressure.

### I.C. Carrier gas of any embodiment

The carrier gas can comprise or consist of an inert gas, for example argon, helium, xenon, neon, another gas that is inert to the other constituents of the process gas under the deposition conditions, or any combination of two or more of these.

## II. PLASMA OF ANY EMBODIMENT

The plasma of any PECVD embodiment is formed in the vicinity of the substrate. The plasma can in certain cases, especially when preparing an SiOx coating, be a non-hollow-cathode plasma. In other certain cases, especially when preparing a lubricity coating, a non-hollow-cathode plasma is not desired. The plasma can be formed from the gaseous reactant at reduced pressure.

## III. RF POWER OF ANY EMBODIMENT

The precursor can be contacted with a plasma made by energizing the vicinity of the precursor with electrodes powered at a frequency of 10 kHz to 2.45 GHz, alternatively from about 13 to about 14 MHz.

The precursor can be contacted with a plasma made by energizing the vicinity of the precursor with electrodes powered at radio frequency, optionally at a frequency of from 10 kHz to less than 300 MHz, optionally from 1 to 50 MHz, even optionally from 10 to 15 MHz, optionally at 13.56 MHz.

The precursor is contacted with a plasma made by energizing the vicinity of the precursor with electrodes supplied with electric power at from 0.1 to 25 W, optionally from 1 to 22 W, optionally from 1 to 10 W, even optionally from 1 to 5 W, optionally from 2 to 4 W, for example of 3 W, optionally from 3 to 17 W, even optionally from 5 to 14 W, for example 6 or 7.5 W, optionally from 7 to 11 W, for example of 8 W.

The precursor can be contacted with a plasma made by energizing the vicinity of the precursor with electrodes supplied with electric power density at less than 10 W/ml of plasma volume, alternatively from 6 W/ml to 0.1 W/ml of plasma volume, alternatively from 5 W/ml to 0.1 W/ml of plasma volume, alternatively from 4 W/ml to 0.1 W/ml of plasma volume, alternatively from 2 W/ml to 0.2 W/ml of plasma volume.

## IV. OTHER PROCESS OPTIONS OF ANY EMBODIMENT

The applying step for applying a coating to the substrate is carried out by vaporizing the precursor and providing it in the vicinity of the substrate.

The chemical vapor deposition employed is PECVD and the deposition time is from 1 to 30 sec, alternatively from 2 to 10 sec, alternatively from 3 to 9 sec. The purposes for optionally limiting deposition time can be to avoid overheating the substrate, to increase the rate of production, and to reduce the use of process gas and its constituents. The purposes for optionally extending deposition time can be to provide a thicker coating for particular deposition conditions.

## V. COATING PROPERTIES OF ANY EMBODIMENT

### V.A. Lubricity properties of any embodiment

The vessels (i.e. syringe barrels and/or plungers) coated with a lubricity coating according to present invention have a higher lubricity (determined, e.g. by measuring the Fi and/or Fm) than the uncoated vessels. They also have a higher lubricity than vessels coated with a $SiO_x$ coating as described herein. An embodiment can be carried out under conditions effective to form a lubricated surface of the substrate having a lower sliding force or breakout force (or optionally both) than the untreated substrate. Optionally, the materials and conditions can be effective to reduce the sliding force

or breakout force by at least 25 percent, alternatively at least 45 percent, alternatively at least 60 percent, alternatively more than 60 percent relative to an uncoated syringe barrel. Expressed otherwise, the coating can have a lower frictional resistance than the uncoated surface, wherein optionally the frictional resistance can be reduced by at least 25%, optionally by at least 45%, even optionally by at least 60% in comparison to the uncoated surface.

The break loose force (Fi) and the glide force (Fm) are important performance measures for the effectiveness of a lubricity coating. For Fi and Fm, it is desired to have a low, but not too low value. With too low Fi, which means a too low level of resistance (the extreme being zero), premature/unintended flow may occur, which might e.g. lead to an unintentional premature or uncontrolled discharge of the content of a prefilled syringe.

In order to achieve a sufficient lubricity (e.g. to ensure that a syringe plunger can be moved in the syringe, but to avoid uncontrolled movement of the plunger), the following ranges of Fi and Fm should be advantageously maintained:

Fi: 2.5 to 5 lbs, preferably 2.7 to 4.9 lbs, and in particular 2.9 to 4.7 lbs;
Fm: 2.5 to 8.0 lbs, preferably 3.3 to 7.6 lbs, and in particular 3.3 to 4 lbs.

Further advantageous Fi and Fm values can be found in the Tables of the Examples.

The lubricity coating optionally provides a consistent plunger force that reduces the difference between the break loose force (Fi) and the glide force (Fm).

**V.B. Hydrophobicity properties of any embodiment**

Optionally, the hydrophobic characteristics of the coating can be set by setting the ratio of the $O_2$ to the organosilicon precursor in the gaseous reactant, and/or by setting the electric power used for generating the plasma. Optionally, the coating can have a lower wetting tension than the uncoated surface, optionally a wetting tension of from 20 to 72 dyne/cm, optionally from 30 to 60 dynes/cm, optionally from 30 to 40 dynes/cm, optionally 34 dyne/cm. Optionally, the coating can be more hydrophobic than the uncoated surface.

**V.C. Thickness of any embodiment**

Optionally, the coating can have a thickness determined by transmission electron microscopy (TEM), of any amount stated in this disclosure.

For the lubricity coatings described herein, the indicated thickness ranges are representing average thickness, as a certain roughness may enhance the lubricious properties of the lubricity coating. Thus the thickness of the lubricity coating is advantageously not uniform throughout the coating (see above). However, a uniformly thick lubricity coating is also considered. The absolute thickness of the lubricity coating at single measurement points can be higher or lower than the range limits of the average thickness, with maximum deviations of preferably +/- 50%, more preferably +/-25% and even more preferably +/- 15% from the average thickness. However, it typically varies within the thickness ranges given for the average thickness in this description.

**V.D. Composition of any embodiment**

The lubricity coating can be composed of $Si_wO_xC_yH_z$ or SiwNxCyHz. It generally has an atomic ratio $Si_wO_xC_y$ wherein w is 1, x is from about 0.5 to about 2.4, y is from about 0.6 to about 3, preferably w is 1, x is from about 0.5 to 1.5, and y is from 0.9 to 2.0, more preferably w is 1, x is from 0.7 to 1.2 and y is from 0.9 to 2.0. The atomic ratio can be determined by XPS (X-ray photoelectron spectroscopy). Taking into account the H atoms, the coating may thus in one aspect have the formula $Si_wO_xC_yH_z$, for example where w is 1, x is from about 0.5 to about 2.4, y is from about 0.6 to about 3, and z is from about 2 to about 9. Typically, the atomic ratios are Si 100 : O 80-110 : C 100-150 in a particular coating of present invention. Specifically, the atomic ratio may be Si 100 : O 92-107 : C 116-133, and such coating would hence contain 36% to 41% carbon normalized to 100% carbon plus oxygen plus silicon.

Alternatively, w can be 1, x can be from about 0.5 to 1.5 y can be from about 2 to about 3, and z can be from 6 to about 9. Alternatively, the coating can have atomic concentrations normalized to 100% carbon, oxygen, and silicon, as determined by X-ray photoelectron spectroscopy (XPS) of less than 50% carbon and more than 25% silicon. Alternatively, the atomic concentrations are from 25 to 45% carbon, 25 to 65% silicon, and 10 to 35% oxygen. Alternatively, the atomic concentrations are from 30 to 40% carbon, 32 to 52% silicon, and 20 to 27% oxygen. Alternatively, the atomic concentrations are from 33 to 37% carbon, 37 to 47% silicon, and 22 to 26% oxygen.

Optionally, the atomic concentration of carbon, normalized to 100% of carbon, oxygen, and silicon, as determined by X-ray photoelectron spectroscopy (XPS), can be greater than the atomic concentration of carbon in the atomic formula for the organosilicon precursor. For example, coatings are contemplated in which the atomic concentration of carbon increases by from 1 to 80 atomic percent, alternatively from 10 to 70 atomic percent, alternatively from 20 to 60

atomic percent, alternatively from 30 to 50 atomic percent, alternatively from 35 to 45 atomic percent, alternatively from 37 to 41 atomic percent.

Optionally, the atomic ratio of carbon to oxygen in the coating can be increased in comparison to the organosilicon precursor, and/or the atomic ratio of oxygen to silicon can be decreased in comparison to the organosilicon precursor.

Optionally, the coating can have an atomic concentration of silicon, normalized to 100% of carbon, oxygen, and silicon, as determined by X-ray photoelectron spectroscopy (XPS), less than the atomic concentration of silicon in the atomic formula for the feed gas. For example, embodiments are contemplated in which the atomic concentration of silicon decreases by from 1 to 80 atomic percent, alternatively by from 10 to 70 atomic percent, alternatively by from 20 to 60 atomic percent, alternatively by from 30 to 55 atomic percent, alternatively by from 40 to 50 atomic percent, alternatively by from 42 to 46 atomic percent.

### V.E. Other Coating Properties of any embodiment

The coating can have a density between 1.25 and 1.65 g/cm$^3$, alternatively between 1.35 and 1.55 g/cm$^3$, alternatively between 1.4 and 1.5 g/cm$^3$, alternatively between 1.4 and 1.5 g/cm$^3$, alternatively between 1.44 and 1.48 g/cm$^3$, as determined by X-ray reflectivity (XRR). Optionally, the organosilicon compound can be octamethylcyclotetrasiloxane and the coating can have a density which can be higher than the density of a coating made from HMDSO as the organosilicon compound under the same PECVD reaction conditions.

The coating optionally can prevent or reduce the precipitation of a compound or component of a composition in contact with the coating, in particular can prevent or reduce insulin precipitation or blood clotting, in comparison to the uncoated surface and/or to a barrier coated surface using HMDSO as precursor.

### VI. PLUS $SIO_x$ COATING, OPTIONAL FOR ANY EMBODIMENT

The coating on a substrate, for example a vessel wall, as well as comprising a lubricity coating, additionally can comprise at least one layer or coating of SiOx, wherein x can be from 1.5 to 2.9, adjacent to the coating on the substrate, alternatively between the coating and the substrate, alternatively on the opposite side of the coating as the substrate. Optionally, the layers of SiOx and the coating can either form a sharp interface or a graded composite of $Si_wO_xC_yH_z$ to $SiO_x$ or vice versa. The substrate coated with a lubricity coating can further comprise a surface treatment of the coating in an amount effective to reduce the leaching of the coating, the substrate, or both. For example, the coating and surface treatment can be composed and present in relative amounts effective to provide a breakout force, sliding force, or both less than the corresponding force required in the absence of the coating and surface treatment. Optionally, the surface treatment can be less than 100 nm deep, alternatively less than 50 nm deep, alternatively less than 40 nm deep, alternatively less than 30 nm deep, alternatively less than 20 nm deep, alternatively less than 10 nm deep, alternatively less than 5 nm deep, alternatively less than 3 nm deep, alternatively less than 1 nm deep, alternatively less than 0.5 nm deep in the lubricity layer. As another contemplated option, the surface treatment can be between 0.1 and 50 nm deep in the lubricity layer.

The optional surface treatment can comprise $SiO_x$, in which x can be from about 1.5 to about 2.9. Optionally, at least a second layer or coating of SiOx, wherein x can be from 1.5 to 2.9, can be applied between the coating and the substrate surface.

Embodiments are contemplated in which the substrate is a vessel having an interior surface defining a lumen and an exterior surface. The lubricity coating can be on the interior surface of the vessel, and the vessel can contain at least one further layer or coating on its exterior surface of $SiO_x$, wherein x can be from 1.5 to 2.9. Alternatively, the further layer or coating on the exterior surface can comprise polyvinylidene chloride (PVDC). The further layer or coating on the exterior surface optionally can be a barrier coating.

## EXAMPLES

The following Examples are in part already disclosed in EP 2 251 455. In order to avoid unnecessary repetition, not all of the Examples in EP 2 251 455 A2 are repeated here, but explicit reference is herewith made to them, in as far as they pertain to a syringe coated with a lubricity coating.

### Basic Protocols for Forming and Coating Syringe Barrels

The vessels tested in the subsequent working examples were formed and coated according to the following exemplary protocols, except as otherwise indicated in individual examples. Particular parameter values given in the following basic protocols, e.g. the electric power and gaseous reactant or process gas flow, are typical values. Whenever parameter values were changed in comparison to these typical values, this will be indicated in the subsequent working

examples. The same applies to the type and composition of the gaseous reactant or process gas.

**Protocol for Forming COC Syringe Barrel**

Syringe barrels for an extended barrel syringe ("COC syringe barrels"), CV Holdings Part 11447, can be used, each having a 2.8 mL overall volume (excluding the Luer fitting) and a nominal 1 mL delivery volume or plunger displacement, Luer adapter type, were injection molded from Topas® 8007-04 cyclic olefin copolymer (COC) resin, available from Hoechst AG, Frankfurt am Main, Germany, having these dimensions: about 51 mm overall length, 8.6 mm inner syringe barrel diameter and 1.27 mm wall thickness at the cylindrical portion, with an integral 9.5 millimeter length needle capillary Luer adapter molded on one end and two finger flanges molded near the other end.

**Protocol for Coating COC Syringe Barrel Interior with $SiO_x$**

An injection molded COC syringe barrel can be interior coated with SiOx. The apparatus as shown in FIG. 1 was modified to hold a COC syringe barrel with butt sealing at the base of the COC syringe barrel. Additionally a cap was fabricated out of a stainless steel Luer fitting and a polypropylene cap that sealed the end of the COC syringe barrel (illustrated in FIG. 8), allowing the interior of the COC syringe barrel to be evacuated.

The vessel holder 50 can be made from Delrin® with an outside diameter of 1.75 inches (44 mm) and a height of 1.75 inches (44 mm). The vessel holder 50 can be housed in a Delrin® structure that allowed the device to move in and out of the electrode 160.

The electrode 160 can be made from copper with a Delrin® shield. The Delrin® shield can be conformal around the outside of the copper electrode 160. The electrode 160 can be approximately 3 inches (76 mm) high (inside) and approximately 0.75 inches (19 mm) wide. The COC syringe barrel can be inserted into the vessel holder 50, base sealing with an Viton® O-rings.

The COC syringe barrel can be carefully moved into the sealing position over the extended (stationary) 1/8-inch (3-mm.) diameter brass probe or counter electrode 108 and pushed against a copper plasma screen. The copper plasma screen can be a perforated copper foil material (K&S Engineering Part #LXMUW5 Copper mesh) cut to fit the outside diameter of the COC syringe barrel and can be held in place by a abutment surface 494 that acted as a stop for the COC syringe barrel insertion. Two pieces of the copper mesh were fit snugly around the brass probe or counter electrode 108 insuring good electrical contact.

The probe or counter electrode 108 extended approximately 20 mm into the interior of the COC syringe barrel and can be open at its end. The brass probe or counter electrode 108 extended through a Swagelok® fitting located at the bottom of the vessel holder 50, extending through the vessel holder 50 base structure. The brass probe or counter electrode 108 can be grounded to the casing of the RF matching network.

The gas delivery port 110 can be connected to a stainless steel assembly comprised of Swagelok® fittings incorporating a manual ball valve for venting, a thermocouple pressure gauge and a bypass valve connected to the vacuum pumping line. In addition, the gas system can be connected to the gas delivery port 110 allowing the gaseous reactant or process gases, oxygen and hexamethyldisiloxane (HMDSO) to be flowed through the gas delivery port 110 (under process pressures) into the interior of the COC syringe barrel.

The gas system can be comprised of a Aalborg® GFC17 mass flow meter (Cole Parmer Part # EW-32661-34) for controllably flowing oxygen at 90 sccm (or at the specific flow reported for a particular example) into the process and a PEEK capillary (OD 1/16-inch (3-mm) ID 0.004 inches (0.1 mm)) of length 49.5 inches (1.26 m) or other length as indicated in a particular example. The PEEK capillary end can be inserted into liquid hexamethyldisiloxane (Alfa Aesar® Part Number L16970, NMR Grade). The liquid HMDSO can be pulled through the capillary due to the lower pressure in the COC syringe barrel during processing. The HMDSO can be then vaporized into a vapor at the exit of the capillary as it entered the low pressure region.

To ensure no condensation of the liquid HMDSO past this point, the gas stream (including the oxygen) can be diverted to the pumping line when it was not flowing into the interior of the COC syringe barrel for processing via a Swagelok® 3-way valve.

Once the COC syringe barrel was installed, the vacuum pump valve can be opened to the vessel holder 50 and the interior of the COC syringe barrel. An Alcatel rotary vane vacuum pump and blower comprised the vacuum pump system. The pumping system allowed the interior of the COC syringe barrel to be reduced to pressure(s) of less than 150 mTorr while the gaseous reactant or process gases were flowing at the indicated rates. A lower pumping pressure can be achievable with the COC syringe barrel, as opposed to the tube, because the COC syringe barrel has a much smaller internal volume.

After the base vacuum level was achieved, the vessel holder 50 assembly was moved into the electrode 160 assembly. The gas stream (oxygen and HMDSO vapor) was flowed into the brass gas delivery port 110 (by adjusting the 3-way valve from the pumping line to the gas delivery port 110). The pressure inside the COC syringe barrel is

approximately 200 mTorr as measured by a capacitance manometer (MKS) installed on the pumping line near the valve that controlled the vacuum. In addition to the COC syringe barrel pressure, the pressure inside the gas delivery port 110 and gas system is also measured with the thermocouple vacuum gauge that is connected to the gas system. This pressure is typically less than 8 Torr.

When the gas is flowing to the interior of the COC syringe barrel, the RF power supply is turned on to its fixed power level. A ENI ACG-6 600 Watt RF power supply is used (at 13.56 MHz) at a fixed power level of approximately 30 Watts. The RF power supply is connected to a COMDEL CPMX1000 auto match that matched the complex impedance of the plasma (to be created in the COC syringe barrel) to the 50 ohm output impedance of the ENI ACG-6 RF power supply. The forward power is 30 Watts (or whatever value is reported in a working example) and the reflected power is 0 Watts so that the power is delivered to the interior of the COC syringe barrel. The RF power supply is controlled by a laboratory timer and the power on time set to 5 seconds (or the specific time period reported for a particular example).

Upon initiation of the RF power, a uniform plasma is established inside the interior of the COC syringe barrel. The plasma is maintained for the entire 5 seconds (or other coating time indicated in a specific example) until the RF power is terminated by the timer. The plasma produced a silicon oxide coating of approximately 20 nm thickness (or the thickness reported in a specific example) on the interior of the COC syringe barrel surface.

After coating, the gas flow is diverted back to the vacuum line and the vacuum valve is closed. The vent valve is then opened, returning the interior of the COC syringe barrel to atmospheric pressure (approximately 760 Torr). The COC syringe barrel is then carefully removed from the vessel holder 50 assembly (after moving the vessel holder 50 assembly out of the electrode 160 assembly).

**Protocol for Coating COC Syringe Barrel Interior with OMCTS Lubricity Layer or Coating**

COC syringe barrels as previously identified were interior coated with a lubricity layer. The apparatus as shown in FIG. 1 is modified to hold a COC syringe barrel with butt sealing at the base of the COC syringe barrel. Additionally a cap is fabricated out of a stainless steel Luer fitting and a polypropylene cap that sealed the end of the COC syringe barrel (illustrated in FIG. 8). The installation of a Buna-N O-ring onto the Luer fitting allowed a vacuum tight seal, allowing the interior of the COC syringe barrel to be evacuated.

The vessel holder 50 is made from Delrin® with an outside diameter of 1.75 inches (44 mm) and a height of 1.75 inches (44 mm). The vessel holder 50 is housed in a Delrin® structure that allowed the device to move in and out of the electrode 160.

The electrode 160 is made from copper with a Delrin® shield. The Delrin® shield is conformal around the outside of the copper electrode 160. The electrode 160 measured approximately 3 inches (76 mm) high (inside) and is approximately 0.75 inches (19 mm) wide. The COC syringe barrel is inserted into the vessel holder 50, base sealing with Viton® O-rings around the bottom of the finger flanges and lip of the COC syringe barrel.

The COC syringe barrel is carefully moved into the sealing position over the extended (stationary) 1/8-inch (3-mm.) diameter brass probe or counter electrode 108 and pushed against a copper plasma screen. The copper plasma screen is a perforated copper foil material (K&S Engineering Part #LXMUW5 Copper mesh) cut to fit the outside diameter of the COC syringe barrel and is held in place by a abutment surface 494 that acted as a stop for the COC syringe barrel insertion. Two pieces of the copper mesh were fit snugly around the brass probe or counter electrode 108 insuring good electrical contact.

The probe or counter electrode 108 extended approximately 20mm (unless otherwise indicated) into the interior of the COC syringe barrel and is open at its end. The brass probe or counter electrode 108 extended through a Swagelok® fitting located at the bottom of the vessel holder 50, extending through the vessel holder 50 base structure. The brass probe or counter electrode 108 is grounded to the casing of the RF matching network.

The gas delivery port 110 is connected to a stainless steel assembly comprised of Swagelok® fittings incorporating a manual ball valve for venting, a thermocouple pressure gauge and a bypass valve connected to the vacuum pumping line. In addition, the gas system is connected to the gas delivery port 110 allowing the gaseous reactant or process gas, octamethylcyclotetrasiloxane (OMCTS) (or the specific gaseous reactant or process gas reported for a particular example) to be flowed through the gas delivery port 110 (under process pressures) into the interior of the COC syringe barrel.

The gas system is comprised of a commercially available Horiba VC1310/SEF8240 OMCTS 10SC 4CR heated mass flow vaporization system that heated the OMCTS to about 100°C. The Horiba system is connected to liquid octamethylcyclotetrasiloxane (Alfa Aesar® Part Number A12540, 98%) through a 1/8-inch (3-mm) outside diameter PFA tube with an inside diameter of 1/16 in (1.5 mm). The OMCTS flow rate is set to 1.25 sccm (or the specific organosilicon precursor flow reported for a particular example). To ensure no condensation of the vaporized OMCTS flow past this point, the gas stream is diverted to the pumping line when it is not flowing into the interior of the COC syringe barrel for processing via a Swagelok® 3-way valve.

Once the COC syringe barrel is installed, the vacuum pump valve is opened to the vessel holder 50 and the

interior of the COC syringe barrel. An Alcatel rotary vane vacuum pump and blower comprise- the vacuum pump system. The pumping system allows the interior of the COC syringe barrel to be reduced to pressure(s) of less than 100 mTorr while the gaseous reactant or process gases is flowing at the indicated rates. A lower pressure can be obtained in this instance, compared to the tube and previous COC syringe barrel examples, because the overall gaseous reactant or process gas flow rate is lower in this instance.

Once the base vacuum level is achieved, the vessel holder 50 assembly is moved into the electrode 160 assembly. The gas stream (OMCTS vapor) is flowed into the brass gas delivery port 110 (by adjusting the 3-way valve from the pumping line to the gas delivery port 110). Pressure inside the COC syringe barrel is approximately 140 mTorr as measured by a capacitance manometer (MKS) installed on the pumping line near the valve that controlled the vacuum. In addition to the COC syringe barrel pressure, the pressure inside the gas delivery port 110 and gas system is also measured with the thermocouple vacuum gauge that is connected to the gas system. This pressure is typically less than 6 Torr.

Once the gas is flowing to the interior of the COC syringe barrel, the RF power supply is turned on to its fixed power level. A ENI ACG-6 600 Watt RF power supply is used (at 13.56 MHz) at a fixed power level of approximately 6 Watts (or other power level indicated in a specific example). The RF power supply is connected to a COMDEL CPMX1000 auto match which matched the complex impedance of the plasma (to be created in the COC syringe barrel) to the 50 ohm output impedance of the ENI ACG-6 RF power supply. The forward power is 6 Watts and the reflected power is 0 Watts so that 6 Watts of power (or a different power level delivered in a given example) is delivered to the interior of the COC syringe barrel. The RF power supply is controlled by a laboratory timer and the power on time set to 10 seconds (or a different time stated in a given example).

Upon initiation of the RF power, a uniform plasma is established inside the interior of the COC syringe barrel. The plasma is maintained for the entire coating time, until the RF power is terminated by the timer. The plasma produced a lubricity layer or coating on the interior of the COC syringe barrel surface.

After coating, the gas flow is diverted back to the vacuum line and the vacuum valve is closed. The vent valve is then opened, returning the interior of the COC syringe barrel to atmospheric pressure (approximately 760 Torr). The COC syringe barrel is then carefully removed from the vessel holder 50 assembly (after moving the vessel holder 50 assembly out of the electrode 160 assembly).

**Protocol for Coating COC Syringe Barrel Interior with HMDSO Coating**

The Protocol for Coating COC Syringe Barrel Interior with OMCTS Lubricity layer or coating is also used for applying an HMDSO coating, except substituting HMDSO for OMCTS.

**Protocol for Lubricity Testing**

The following materials are used in this test:

- Commercial (BD Hypak® PRTC) glass prefillable syringes with Luer-lok® tip) (ca 1 mL)
- COC syringe barrels made according to the Protocol for Forming COC Syringe barrel;
- Commercial plastic syringe plungers with elastomeric tips taken from Becton Dickinson Product No. 306507 (obtained as saline prefilled syringes);
- Normal saline solution (taken from the Becton-Dickinson Product No. 306507 prefilled syringes);
- Dillon Test Stand with an Advanced Force Gauge (Model AFG-50N)
- Syringe holder and drain jig (fabricated to fit the Dillon Test Stand)

The following procedure is used in this test.

The jig is installed on the Dillon Test Stand. The platform probe movement is adjusted to 6 in/min (2.5 mm/sec) and upper and lower stop locations were set. The stop locations were verified using an empty syringe and barrel. The commercial saline-filled syringes were labeled, the plungers were removed, and the saline solution is drained via the open ends of the syringe barrels for re-use. Extra plungers were obtained in the same manner for use with the COC and glass barrels.

Syringe plungers were inserted into the COC syringe barrels so that the second horizontal molding point of each plunger is even with the syringe barrel lip (about 10 mm from the tip end). Using another syringe and needle assembly, the test syringes were filled via the capillary end with 2-3 milliliters of saline solution, with the capillary end uppermost. The sides of the syringe were tapped to remove any large air bubbles at the plunger/ fluid interface and along the walls, and any air bubbles were carefully pushed out of the syringe while maintaining the plunger in its vertical orientation.

Each filled syringe barrel/plunger assembly is installed into the syringe jig. The test is initiated by pressing the

down switch on the test stand to advance the moving metal hammer toward the plunger. When the moving metal hammer is within 5mm of contacting the top of the plunger, the data button on the Dillon module is repeatedly tapped to record the force at the time of each data button depression, from before initial contact with the syringe plunger until the plunger is stopped by contact with the front wall of the syringe barrel.

All benchmark and coated syringe barrels were run with five replicates (using a new plunger and barrel for each replicate).

COC syringe barrels made according to the Protocol for Forming COC Syringe barrel were coated with an OMCTS lubricity layer or coating according to the Protocol for Coating COC Syringe Barrel Interior with OMCTS Lubricity layer, except at a power of 7.5 Watts, assembled and filled with saline, and tested as described above in this Example for lubricity layers. The polypropylene chamber used per the Protocol for Coating COC Syringe Barrel Interior with OMCTS Lubricity layer or coating allowed the OMCTS vapor (and oxygen, if added) to flow through the syringe barrel and through the syringe capillary into the polypropylene chamber (although a lubricity layer or coating can not be needed in the capillary section of the syringe in this instance). Different coating conditions were tested. All of the depositions were completed on COC syringe barrels from the same production batch.

The samples were created by coating COC syringe barrels according to the Protocol for Coating COC Syringe Barrel Interior with OMCTS Lubricity layer. An alternative embodiment of the technology herein, would apply the lubricity layer or coating over another thin film coating, such as $SiO_x$, for example applied according to the Protocol for Coating COC Syringe barrel Interior with $SiO_x$.

Instead of the Dillon Test Stand and drain jig, a Genesis Packaging Plunger Force Tester (Model SFT-01 Syringe Force Tester, manufactured by Genesis Machinery, Lionville, PA) can also be used following the manufacturer's instructions for measuring Fi and Fm. The parameters that are used on the Genesis tester are:

| | |
|---|---|
| Start: | 10mm |
| Speed: | 100mm/min |
| Range: | 20 |
| Units: | Newtons |

## WORKING EXAMPLES

in addition to the Working Examples concerning lubricity coating of a syringe presented in EP 2 251 455 A2 which are also understood as Working Examples for the present invention.

### Examples A-D

Syringe samples were produced as follows. A COC 8007 extended barrel syringe was produced according to the Protocol for Forming COC Syringe Barrel. An SiOx coating was applied to some of the syringes according to the Protocol for Coating COC Syringe Barrel Interior with SiOx. A lubricity coating was applied to the SiOx coated syringes according to the Protocol for Coating COC Syringe Barrel Interior with OMCTS Lubricity layer, modified as follows. The OMCTS was supplied from a vaporizer, due to its low volatility. Argon carrier gas was used. The process conditions were set to the following:

- OMCTS - 3 sccm
- Argon gas - 65 sccm
- Power - 6 watts
- Time - 10 seconds

The coater was later determined to have a small leak while producing the L2 samples identified in the Table, which resulted in an estimated oxygen flow of 1.0 sccm. The L3 samples were produced without introducing oxygen.

Several syringes were then tested for lubricity using a Genesis Packaging Plunger Force Tester (Model SFT-01 Syringe Force Tester, manufactured by Genesis Machinery, Lionville, PA) according to the Protocol for Lubricity Testing. Both the initiation force and maintenance forces (in Newtons) were noted relative to an uncoated sample, and are reported in Table 1.

Syringes coated with silicon oil were included as a reference since this is the current industry standard.

### Examples E-H

Syringe samples were produced as follows. A COC 8007 extended barrel syringe was produced according to

the Protocol for Forming COC Syringe Barrel. An SiOx coating was applied to the syringe barrels according to the Protocol for Coating COC Syringe Barrel Interior with SiOx. A lubricity coating was applied to the SiOx coated syringes according to the Protocol for Coating COC Syringe Barrel Interior with OMCTS Lubricity layer, modified as follows. The OMCTS was supplied from a vaporizer, due to its low volatility. Argon carrier gas and oxygen were used where noted in Table 2. The process conditions were set to the following, or as indicated in Table 2:

- OMCTS -3 sccm (when used)
- Argon gas -7.8 sccm (when used)
- Oxygen 0.38 sccm (when used)
- Power - 3 watts
- Power on time - 10 seconds

Syringes E and F prepared under these conditions, Syringes G prepared under these conditions except without a lubricity coating, and Syringes H (a commercial syringe coated with silicon oil) were then tested for lubricity using a Genesis Packaging Plunger Force Tester according to the Protocol for Lubricity Testing. Both the initiation force and maintenance forces (in Newtons) were noted relative to an uncoated sample, and are reported in Table 2. Syringes coated with silicon oil were included as a reference since this is the current industry standard.

The lubricity results are shown in Table 2 (Initiation Force and Maintenance Force), illustrating under these test conditions as well that the lubricity coating on Syringes E and F markedly improved their lubricity compared to Syringes G which lacked any lubricity coating. The lubricity coating on Syringes E and F also markedly improved their lubricity compared to Syringes H which contained the standard lubricity coating in the industry.

Syringes E, F, and G were also tested to determine total extractable silicon levels (representing extraction of the organosilicon-based PECVD coatings) using an Inductively Coupled Plasma-Mass Spectrometry (ICP-MS) analysis.

The silicon was extracted using saline water digestion. The tip of each syringe plunger was covered with PTFE tape to prevent extracting material from the elastomeric tip material, then inserted into the syringe barrel base. The syringe barrel was filled with two milliliters of 0.9% aqueous saline solution via a hypodermic needle inserted through the Luer tip of the syringe. This is an appropriate test for extractables because many prefilled syringes are used to contain and deliver saline solution. The Luer tip was plugged with a piece of PTFE beading of appropriate diameter. The syringe was set into a PTFE test stand with the Luer tip facing up and placed in an oven at 50°C for 72 hours.

Then, either a static or a dynamic mode was used to remove the saline solution from the syringe barrel. According to the static mode indicated in Table 2, the syringe plunger was removed from the test stand, and the fluid in the syringe was decanted into a vessel. According to the dynamic mode indicated in Table 2, the Luer tip seal was removed and the plunger was depressed to push fluid through the syringe barrel and expel the contents into a vessel. In either case, the fluid obtained from each syringe barrel was brought to a volume of 50ml using 18.2MΩ*cm deionized water and further diluted 2x to minimize sodium background during analysis. The CVH barrels contained two milliliters and the commercial barrels contained 2.32 milliliters.

Next, the fluid recovered from each syringe was tested for extractable silicon using Inductively Coupled Plasma-Mass Spectrometry (ICP-MS) Analysis. The instrument: used was a Perkin Elmer Elan DRC II equipped with a Cetac ASX-520 autosampler. The following ICP - MS conditions were employed:

- Nebulizer: Quartz Meinhardt
- Spray Chamber: Cyclonic
- RF (radio frequency) power: 1550 Watts
- Argon (Ar) Flow: 15.0 L/min
- Auxiliary Ar Flow: 1.2 L/min
- Nebulizer Gas Flow: 0.88 L/min
- Integration time: 80 sec
- Scanning mode: Peak hopping
- RPq (The RPq is a rejection parameter) for Cerium as CeO (m/z 156): < 2 %

Aliquots from aqueous dilutions obtained from Syringes E, F, and G were injected and analyzed for Si in concentration units of micrograms per liter. The results of this test are shown in Table 2. While the results are not quantitative, they do indicate that extractables from the lubricity coating are not clearly higher than the extractables for the SiOx barrier layer only. Also, the static mode produced far less extractables than the dynamic mode, which was expected.

**Examples I-K**

Syringe samples I, J, and K, employing three different lubricity coatings, were produced in the same manner as for Examples E-H except as follows or as indicated in Table 3:

- OMCTS -2.5 sccm
- Argon gas -7.6 sccm (when used)
- Oxygen 0.38 sccm (when used)
- Power - 3 watts
- Power on time - 10 seconds

Syringe I had a three-component coating employing OMCTS, oxygen, and carrier gas. Syringe J had a two component coating employing OMCTS and oxygen, but no carrier gas. Syringe K had a one-component coating (OMCTS only). Syringes I, J, and K were then tested for lubricity as described for Examples E-H.

The lubricity results are shown in Table 3 (Initiation Force and Maintenance Force). Syringe I with a three-component coating employing OMCTS, oxygen, and carrier gas provided the best lubricity results for both initiation force and maintenance force. Syringe J omitting the carrier gas yielded intermediate results. Syringe K had a one-component coating (OMCTS only), and provided the lowest lubricity. This example shows that the addition of both a carrier gas and oxygen to the process gas improved lubricity under the tested conditions.

**Examples L-N**

Examples I-K using an OMCTS precursor gas were repeated in Examples L-N, except that HMDSO was used as the precursor in Examples L-N. The results are shown in Table 3. The results show that for the tested three-component, two-component, and one-component lubricity coatings, the OMCTS coatings provided lower resistance, thus better lubricity, than the HMDSO coatings, demonstrating the value of OMCTS as the precursor gas for lubricity coatings.

**Examples O-V, W, X, Y**

In these examples the surface roughness of the lubricity coatings was correlated with lubricity performance.

OMCTS coatings were applied with previously described equipment with the indicated specific process conditions (Table 5) onto one milliliter COC 6013 molded syringe barrels. Plunger force measurements ($F_i$, $F_m$) (Table 5) were performed with previously described equipment under the same protocols. Scanning electron spectroscopy (SEM) photomicrographs (Table 5, Figs. 63 to 67) and atomic force microscopy (AFM) Root Mean Square (RMS) and other roughness determinations (Tables 5 and 6) were made using the procedures indicated below. Average RMS values are taken from three different RMS readings on the surface. The plunger force tests, AFM and SEM tests reported in table 5 were performed on different samples due to the nature of the individual tests which prohibited a performance of all tests on one sample.

Comparison of Fi/Fm to SEM photomicrograph to AFM Average RMS values clearly indicates that lower plunger forces are realized with non-continuous, rougher OMCTS plasma-coated surfaces (cf. Samples O to Q vs. R to V; Fig. 18 to 20).

Further testing was carried out on sister samples Examples W, X, and Y, respectively made under conditions similar to Example Q, T, and V, to show the $F_i$ and $F_m$ values corresponding to the AFM roughness data. Example W which has a higher surface roughness (compare Example Q in Fig. 18, Table 5) has much lower $F_i$ and $F_m$ friction values (Table 6) than Example X (compare Example T in Fig. 19) or Y. The $F_m$ test shown in Table 6 was interrupted before reaching the measured value of $F_m$ for Examples X and Y because the $F_m$ value was too high.

**SEM Procedure**

SEM Sample Preparation: Each syringe sample was cut in half along its length (to expose the interior surface). The top of the syringe (Luer end) was cut off to make the sample smaller.

The sample was mounted onto the sample holder with conductive graphite adhesive, then put into a Denton Desk IV SEM Sample Preparation System, and a thin (approximately 50 Å) thick gold coating was sputtered onto the interior surface of the syringe. The gold coating is required to eliminate charging of the surface during measurement.

The sample was removed from the sputter system and mounted onto the sample stage of a Jeol JSM 6390 SEM (Scanning Electron Microscope). The sample was pumped down to at least 1 x $10^{-6}$ Torr in the sample compartment. Once the sample reached the required vacuum level, the slit valve was opened and the sample was moved into the analysis station.

The sample was imaged at a coarse resolution first, then higher magnification images were accumulated. The SEM images provided in the Figures are 5 μm edge-to-edge (horizontal and vertical).

**AFM (Atomic Force Microscopy) Procedure.**

AFM images were collected using a NanoScope III Dimension 3000 machine (Digital Instruments, Santa Barbara, California, USA). The instrument was calibrated against a NIST traceable standard. Etched silicon scanning probe microscopy (SPM) tips were used. Image processing procedures involving auto-flattening, plane fitting or convolution were employed. One 10 μm x 10 μm area was imaged. Roughness analyses were performed and were expressed in: (1) Root-Mean-Square Roughness, RMS; (2) Mean Roughness, Ra; and (3) Maximum Height (Peak-to-Valley), Rmax, all measured in nm (see Table 5 and Fig. 18 to 20). For the roughness analyses, each sample was imaged over the 10 μm x 10 μm area, followed by three cross sections selected by the analyst to cut through features in the 10 μm x 10 μm images. The vertical depth of the features was measures using the cross section tool. For each cross section, a Root-Mean-Square Roughness (RMS) in nanometers was reported. These RMS values along with the average of the three cross sections for each sample are listed in Table 5.

Additional analysis of the 10 μm x 10 μm images represented by Figs. 65 to 67 (Examples Q, T and V) was carried out. For this analysis three cross sections were extracted from each image. The locations of the cross sections were selected by the analyst to cut through features in the images. The vertical depth of the features was measured using the cross section tool.

The Digital Instruments Nanoscope III AFM/STM acquires and stores 3-dimensional representations of surfaces in a digital format. These surfaces can be analyzed in a variety of ways.

The Nanoscope III software can perform a roughness analysis of any AFM or STM image. The product of this analysis is a single color page reproducing the selected image in top view. To the upper right of the image is the "Image Statistics" box, which lists the calculated characteristics of the whole image minus any areas excluded by a stopband (a box with an X through it). Similar additional statistics can be calculated for a selected portion of the image and these are listed in the "Box Statistics" in the lower right portion of the page. What follows is a description and explanation of these statistics.

Image Statistics:

Z Range ($R_p$): The difference between the highest and lowest points in the image. The value is not corrected for tilt in the plane of the image; therefore, plane fitting or flattening the data will change the value.

Mean: The average of all of the Z values in the imaged area. This value is not corrected for the tilt in the plane of the image; therefore, plane fitting or flattening the data will change this value.

RMS ($R_q$): This is the standard deviation of the Z values (or RMS roughness) in the image. It is calculated according to the formula:

$$R_q= \{\Sigma(Z_1-Z_{avg})2/N\}$$

where $Z_{avg}$ is the average Z value within the image; $Z_1$ is the current value of Z; and N is the number of points in the image. This value is not corrected for tilt in the plane of the image; therefore, plane fitting or flattening the data will change this value.

Mean roughness ($R_a$): This is the mean value of the surface relative to the Center Plane and is calculated using the formula:

$$R_a=[1/(L_xL_y)]\int_o^{Ly}\int_o^{Lx}\{f(x,y)\}dxdy$$

where f(x,y) is the surface relative to the Center plane, and $L_x$ and $L_y$ are the dimensions of the surface.

Max height ($R_{max}$): This is the difference in height between the highest and lowest points of the surface relative to the Mean Plane.

Surface area: (Optical calculation): This is the area of the 3-dimensional surface of the imaged area. It is calculated by taking the sum of the areas of the triangles formed by 3 adjacent data points throughout the image.

Surface area diff: (Optional calculation) This is the amount that the Surface area is in excess of the imaged area. It is expressed as a percentage and is calculated according to the formula:

$$\text{Surface area diff} = 100[(\text{Surface area}/S_1 2)-1]$$

where $S_1$ is the length (and width) of the scanned area minus any areas excluded by stopbands.

Center Plane: A flat plane that is parallel to the Mean Plane. The volumes enclosed by the image surface above and below the center plane are equal.

Mean Plane: The image data has a minimum variance about this flat plane. It results from a first order least squares fit on the Z data.

**Summary of Lubricity Measurements**

Table 8 shows a summary of the above OMCTS coatings and their Fi and Fm values. It has to be understood that the initial lubricity coating work (C-K; roughness not known) was to identify the lowest possible plunger force attainable. From subsequent market input, it was determined that the lowest achievable plunger force was not necessarily most desirable, for reasons explained in the generic description (e.g. premature release). Thus, the PECVD reaction parameters were varied to obtain a plunger force of practical market use.

**Example Z: Lubricity Coating Extractables**

Total silicon extractables were measured using ICP-MS analysis. The syringes were evaluated in both static and dynamic situations. The following describes the test procedure:

- Syringe filled with 2 ml of 0.9% saline solution
- Syringe placed in a stand - stored at 50°C for 72 hours.
- After 72 hours saline solution test for total silicon
- Total silicon measure before and after saline solution expelled through syringe.

The extractable Silicon Levels from a silicon oil coated glass syringe and a Lubricity coated and $SiO_2$ coated COC syringe are shown in Table 7. Precision of the ICP-MS total silicon measurement is +/- 3%.

**TABLE 1: PLUNGER SLIDING FORCE MEASUREMENTS OF OMCTS-BASED PLASMA COATINGS MADE WITH CARRIER GAS**

| Example | Coating Type | Lubricity Layer or coating Monomer | Lubricity Coating Time (sec) | Lubricity OMCTS Flow Rate (sccm) | Lubricity O2 Flow Rate (sccm) | Carrier Gas (Ar) Flow Rate (sccm) | Coating Power (Watts) | Initiation Force, $F_i$ (N, Kg.) | Maintenance Force, $F_m$ (N, Kg.) |
|---|---|---|---|---|---|---|---|---|---|
| A (Control) | Uncoated COC | n/a | n/a | n/a | n/a | n/a | n/a | >11 N >1.1 Kg. | >11 N >1.1 Kg. |
| B (Industry Standard) | Silicon oil on COC | n/a | n/a | n/a | n/a | n/a | n/a | 8.2 N 0.84 Kg. | 6.3 N 0.64 Kg. |
| C (without Oxygen) | L3 Lubricity layer or coating over $SiO_x$ on COC | OMCTS | 10 sec | 3 | 0 | 65 | 6 | 4.6 N 0.47 Kg. | 4.6 N 0.47 Kg. |
| D (with Oxygen) | L2 Lubricity layer or coating over $SiO_x$ on COC | OMCTS | 10 sec | 3 | 1 | 65 | 6 | 4.8 N 0.49 Kg. | 3.5 N 0.36 Kg. |

**TABLE 2: OMCTS Lubricity Coatings (E and F)**

| Example | OMCTS (sccm) | $O_2$ (sccm) | Ar (sccm) | Initiation Force, Fi (N) | Mainten- ance Force, Fm (N) | ICPMS (μg /liter) | ICPMS Mode |
|---|---|---|---|---|---|---|---|
| E | 3.0 | 0.38 | 7.8 | 4.8 | 3.5 | <5 | static |
| F | 3.0 | 0.38 | 7.8 | 5.4 | 4.3 | 38 | dynamic |
| G (SiOx only) | n/a | n/a | n/a | 13 | 11 | <5 | static |
| H (silicon oil) | n/a | n/a | n/a | 8.2 | 6.3 | | |

**TABLE 3: OMCTS Lubricity Coatings**

| Example | OMCTS (sccm) | $O_2$ (sccm) | Ar (sccm) | Initiation Force, Fi (N) | Mainten ance Force, Fm (N) |
|---|---|---|---|---|---|
| I | 2.5 | 0.38 | 7.6 | 5.1 | 4.4 |
| J | 2.5 | 0.38 | - | 7.1 | 6.2 |
| K | 2.5 | - | - | 8.2 | 7.2 |

**TABLE 4: HMDSO Coatings**

| Example | HMDSO (sccm) | $O_2$ (sccm) | Ar (sccm) | Initiation Force, Fi (N) | Mainten ance Force, Fm (N) |
|---|---|---|---|---|---|
| L | 2.5 | 0.38 | 7.6 | 9 | 8.4 |
| M | 2.5 | 0.38 | - | >11 | >11 |
| N | 2.5 | - | - | >11 | >11 |

**TABLE 5**

| Example | | OMCTS (seem) | $Ar/O_2$ (seem) | Power (Watts) | Dep. Time (sec) | Plunger Force Fi (lbs, Kg) | Plunger Force Fm (lbs, Kg) | SEM Micrograph (5 micronAF Vertical) | AFM RMS (nanometers) |
|---|---|---|---|---|---|---|---|---|---|
| **O** | Baseline OMCTS Lubricity | 2.0 | 10/0.38 | 3.5 | 10 | 4.66,2.11 (ave) | 3.47,1.57 (ave) | | |
| **P** | | | | | | | | Fig. 16 | |
| **Q** | | | | | | | | | 19.6, 9.9, 9.4 (Average=13.0) Figs. 18A, 18B, 18C |
| | | | | | | | | | |
| **R** | High Power OMCTS Lubricity | 2.0 | 10/0.38 | 4.5 | 10 | 4.9, 2.2 | 7.6, 3.4 | | |
| **S** | | | | | | | | Fig. 17 | |
| **T** | | | | | | | | | 12.5, 8.4, 6.1 (Average=6.3) Fig. 19A, 19B, 19C |
| | | | | | | | | | |
| **U** | No $O_2$ OMCTS Lubricity | 2.0 | 10/0 | 3.4 | 10 | 4.9, 2.2 | 9.7, 4.4 (stopped) | | |
| **V** | | | | | | | | | 1.9, 2.6, 3.0 (Average=2.3) Fig 20A, 20B, 20C |

TABLE 6

| | $SiO_x$/Lub | Coater | Mode | Siloxane Feed | $Ar/O_2$ | Power (W) | Dep. Time (Sec.) | $F_i$ (lb., Kg.) | $F_m$ (lb., Kg.) |
|---|---|---|---|---|---|---|---|---|---|
| **Example W SiOx/ Baseline OMCTS Lub** | **$SiO_x$:** | Auto-Tube | Auto | HMDSO 52.5 in, 133.4 cm. | 0 sccm Ar, 90 seem $O_2$ | 37 | 7 | ~ | ~ |
| | **Lubricity:** | Auto-S | same | OMCTS, 2.0 seem | 10 seem Ar 0.38 seem $O_2$ | 3,4 | 10 | 2.9, 1.3 | 3.3, 1.5 |
| | | | | | | | | | |
| **Example X SiOx/High Pwr OMCTS Lub** | **$SiO_x$:** | same | same | same | same | 37 | 7 | ~ | ~ |
| | **Lubricity:** | same | same | same | same | 4,5 | 10 | 5, 2.3 | 9.5, 4.3 stopped |
| | | | | | | | | | |
| **Example Y SiOx/No $O_2$ OMCTS Lub** | **SiOx:** | Auto-Tube | same | same | 0 seem Ar, 90 seem $O_2$ | 37 | 7 | ~ | ~ |
| | **Lubricity:** | Auto-S | same | same | 10 sccm Ar 0 sccm $O_2$ | 3,4 | 10 | 5.6, | 9.5, 4.3 stopped |

TABLE 7

| Silicon Extractables Comparison of Lubricity Coatings | | |
|---|---|---|
| **Package Type** | **Static (ug/L)** | **Dynamic (ug/L)** |
| Cyclic Olefin Syringe with CV Holdings SiOCH Lubricity Coating | 70 | 81 |
| Borocilicate Glass Syringe with silicone oil | 825 | 835 |

TABLE 8: Summary Table of OMCTS coatings from Tables 1, 2, 3 and 5

| Example | OMCTS (sccm) | $O_2$ (sccm) | Ar (sccm) | Power (Watt) | Dep Time (sec) | $F_i$(lbs) | $F_m$(lbs) |
|---|---|---|---|---|---|---|---|
| **C** | 3.0 | 0.00 | 65 | 6 | 10 | 1.0 | 1.0 |
| **D** | 3.0 | 1.00 | 65 | 6 | 10 | 1.1 | 0.8 |
| **E** | 3.0 | 0.38 | 7.8 | 6 | 10 | 0.8 | 1.1 |
| **F** | 3.0 | 0.38 | 7.8 | 6 | 10 | 1.2 | 1.0 |
| **I** | 2.5 | 0.38 | 7.6 | 6 | 10 | 1.1 | 1.0 |
| **J** | 2.5 | 0.38 | 0.0 | 6 | 10 | 1.6 | 1.4 |
| **K** | 2.5 | 0.00 | 0.0 | 6 | 10 | 1.8 | 1.6 |
| | | | | | | | |
| **O** | 2.0 | 0.38 | 10 | 3.5 | 10 | 4.6 | 3.5 |
| **R** | 2.0 | 0.38 | 10 | 4.5 | 10 | 4.9 | 7.6 |
| **U** | 2.0 | 0.00 | 10 | 3.4 | 10 | 4.9 | 9.7(stop) |
| **W** | 2.0 | 0.38 | 10 | 3.4 | 10 | 2.9 | 3.3 |

(continued)

| Example | OMCTS (sccm) | $O_2$ (sccm) | Ar (sccm) | Power (Watt) | Dep Time (sec) | $F_i$(lbs) | $F_m$(lbs) |
|---|---|---|---|---|---|---|---|
| **X** | 2.0 | 0.38 | 10 | 4.5 | 10 | 5.0 | 9.5 (stop) |
| **Y** | 2.0 | 0.00 | 10 | 3.4 | 10 | 5.6 | 9.5 (stop) |

## Claims

1. A method for preparing a lubricity coating on a plastic substrate, wherein the substrate which is coated is at least a part of the interior surface of a syringe comprising a barrel having an inner surface, a piston or plunger having an outer surface engaging the inner surface of the barrel, wherein at least one of said inner surface and outer surface is coated, the method comprising the steps

   (a) providing a gas comprising an organosilicon precursor and $O_2$, and optionally a noble gas, in the vicinity of the substrate surface, wherein $O_2$ is present in a volume-volume ratio to the organosilicon precursor of from 0.01:1 to 5:1; and
   (b) generating a plasma in the gas with an electric power of from 0.1 to 25 W for a deposition time from 1 to 30 sec, thus forming a lubricity coating on the substrate surface by plasma enhanced chemical vapor deposition (PECVD).

2. The method of claim 1, wherein the organosilicon precursor is a monocyclic siloxane, preferably is OMCTS.

3. The method according to any one of claims 1 to 2, wherein $O_2$ is present in a volume-volume ratio to the organosilicon precursor of from 0.01:1 to 0.5:1.

4. The method according to any one of claims 1 to 3, wherein Ar is present as the noble gas.

5. The method according to any one of the preceding claims, wherein the plasma is generated with an electric power of from 2 to 4 W.

6. The method according to any one of the preceding claims, wherein the ratio of the electrode power to the plasma volume is less than 10 W/ml, preferably from 6 W/ml to 0.1 W/ml, more preferably of from 2.2 W/ml to 1 W/ml.

7. The method according to any one of the preceding claims, wherein the substrate is a polymer selected from the group consisting of a polycarbonate, an olefin polymer, a cyclic olefin copolymer and a polyester, and preferably is a cyclic olefin copolymer, a polyethylene terephthalate or a polypropylene, and more preferably is COC.

8. The method according to any one of the preceding claims, wherein the plasma is generated with electrodes powered at a radiofrequency, preferably at a frequency of from 10 kHz to less than 300 MHz, more preferably of from 1 to 50 MHz, even more preferably of from 10 to 15 MHz, most preferably at 13.56 MHz.

9. The method according to any one of the preceding claims, wherein the resulting lubricity coating has an atomic ratio SiwOxCy or SiwNxCy wherein w is 1, x is from about 0.5 to about 2.4, y is from about 0.6 to about 3.

10. The method according to any one of claims 1 to 9, wherein the plastic substrate is COC, wherein the gas in step (a) comprises octamethylcyclotetrasiloxane, $O_2$ and Ar, and wherein the power for generating the plasma is from 6 W/ml to 0.1 W/ml in relation to the volume of the syringe lumen.

## Patentansprüche

1. Verfahren zum Herstellen einer Gleitfähigkeitsbeschichtung auf einem Kunststoffsubstrat, wobei das Substrat, das beschichtet wird, mindestens ein Teil der Innenfläche einer Spritze ist, die einen Zylinder mit einer Innenfläche, einen Kolben oder Stößel mit einer Außenfläche, der in die Innenfläche des Zylinders eingreift, umfasst, wobei

mindestens eine von Innenfläche und Außenfläche beschichtet ist, wobei das Verfahren die folgenden Schritte umfasst:

(a) Bereitstellen eines Gases, das einen Organosiliciumvorläufer und $O_2$ und gegebenenfalls ein Edelgas umfasst, in der Nähe der Substratoberfläche, wobei $O_2$ in einem Volumenverhältnis mit dem Organosiliciumvorläufer von 0,01:1 bis 5:1 vorliegt; und
(b) Erzeugen eines Plasmas in dem Gas mit einer elektrischen Leistung von 0,1 bis 25 W für eine Abscheidungszeit von 1 bis 30 Sekunden, wodurch eine Gleitfähigkeitsbeschichtung auf der Substratoberfläche durch plasmaunterstützte chemische Gasphasenabscheidung (PECVD) gebildet wird.

**2.** Verfahren nach Anspruch 1, wobei der Organosiliciumvorläufer ein monocyclisches Siloxan ist, vorzugsweise OMCTS.

**3.** Verfahren nach einem der Ansprüche 1 bis 2, wobei $O_2$ in einem Volumenverhältnis zu dem Organosiliciumvorläufer von 0,01:1 bis 0,5:1 vorliegt.

**4.** Verfahren nach einem der Ansprüche 1 bis 3, wobei AR als Edelgas vorliegt.

**5.** Verfahren nach einem der vorhergehenden Ansprüche, wobei das Plasma mit einer elektrischen Leistung von 2 bis 4 W erzeugt wird.

**6.** Verfahren nach einem der vorhergehenden Ansprüche, wobei das Verhältnis der Elektrodenleistung zum Plasmavolumen weniger als 10 W/ml, vorzugsweise von 6 W/ml bis 0,1 W/ml, mehr bevorzugt von 2,2 W/ml bis 1 W/ml beträgt.

**7.** Verfahren nach einem der vorhergehenden Ansprüche, wobei das Substrat ein Polymer ist, ausgewählt aus der Gruppe, bestehend aus einem Polycarbonat, einem Olefinpolymer, einem cyclischen Olefincopolymer und einem Polyester, und vorzugsweise einem cyclischen Olefincopolymer, einem Polyethylenterephthalat oder einem Polypropylen und mehr bevorzugt COC.

**8.** Verfahren nach einem der vorhergehenden Ansprüche, wobei das Plasma mit Elektroden erzeugt wird, die mit einer Hochfrequenz betrieben werden, vorzugsweise mit einer Frequenz von 10 kHz bis weniger als 300 MHz, mehr bevorzugt von 1 bis 50 MHz, noch mehr bevorzugt von 10 bis 15 MHz, am meisten bevorzugt 13,56 MHz.

**9.** Verfahren nach einem der vorhergehenden Ansprüche, wobei die resultierende Gleitfähigkeitsbeschichtung ein Atomverhältnis SiwOxCy oder SiwNxCy aufweist, wobei w 1 ist, x etwa 0,5 bis etwa 2,4 ist, y etwa 0,6 bis etwa 3 ist.

**10.** Verfahren nach einem der Ansprüche 1 bis 9, wobei das Kunststoffsubstrat COC ist, wobei das Gas in Schritt (a) Octamethylcyclotetrasiloxan, $O_2$ und Ar umfasst und wobei die Leistung zum Erzeugen des Plasmas 6 W/ml bis 0,1 W/ml im Verhältnis zum Volumen des Spritzenlumens beträgt.

## Revendications

**1.** Procédé pour la préparation d'un revêtement lubrifiant sur un substrat en plastique, dans lequel le substrat qui est revêtu est au moins une partie de la surface intérieure d'une seringue comprenant un corps ayant une surface interne, un piston ou poussoir ayant une surface externe venant en contact avec la surface interne du corps, dans lequel au moins l'une de ladite surface interne et de ladite surface externe est revêtue, le procédé comprenant les étapes

(a) disposition d'un gaz comprenant un précurseur organosilicié et de l'$O_2$, et éventuellement un gaz noble, au voisinage de la surface du substrat, l'$O_2$ étant présent en un rapport en volume/volume par rapport au précurseur organosilicié de 0,01:1 à 5:1 ; et
(b) production d'un plasma dans le gaz avec une puissance électrique de 0,1 à 25 W pendant une durée de dépôt de 1 à 30 s, ce qui forme ainsi un revêtement lubrifiant sur la surface du substrat par dépôt chimique en phase de vapeur assisté par plasma (PECVD).

**2.** Procédé selon la revendication 1, dans lequel le précurseur organosilicié est un siloxane monocyclique, de préférence

le précurseur organosilicié est l'OMCTS.

3. Procédé selon l'une quelconque des revendications 1 à 2, dans lequel l'$O_2$ est présent en un rapport en volume/volume par rapport au précurseur organosilicié de 0,01:1 à 0,5:1.

4. Procédé selon l'une quelconque des revendications 1 à 3, dans lequel de l'Ar est présent en tant que gaz noble.

5. Procédé selon l'une quelconque des revendications précédentes, dans lequel le plasma est produit avec une puissance électrique de 2 à 4 W.

6. Procédé selon l'une quelconque des revendications précédentes, dans lequel le rapport de la puissance des électrodes au volume de plasma est inférieur à 10 W/ml, de préférence de 6 W/ml à 0,1 W/ml, de préférence encore de 2,2 W/ml à 1 W/ml.

7. Procédé selon l'une quelconque des revendications précédentes, dans lequel le substrat est un polymère choisi dans le groupe constitué par un polycarbonate, un polymère d'oléfine, un copolymère d'oléfine cyclique et un polyester et de préférence le substrat est un copolymère d'oléfine cyclique, un poly(téréphtalate d'éthylène) ou un polypropylène et de préférence encore le substrat est un COC.

8. Procédé selon l'une quelconque des revendications précédentes, dans lequel le plasma est produit avec des électrodes alimentées à une radiofréquence, de préférence à une fréquence de 10 kHz à moins de 300 MHz, de préférence encore de 1 à 50 MHz, de préférence même encore de 10 à 15 MHz, de préférence par-dessus tout à 13,56 MHz.

9. Procédé selon l'une quelconque des revendications précédentes, dans lequel le revêtement lubrifiant résultant a un rapport atomique $Si_wO_xC_y$ ou $Si_wN_xC_y$ dans lequel w vaut 1, x va d'environ 0,5 à environ 2,4, y va d'environ 0,6 à environ 3.

10. Procédé selon l'une quelconque des revendications 1 à 9, dans lequel le substrat en plastique est un COC, dans lequel le gaz dans l'étape (a) comprend de l'octaméthylcyclotétrasiloxane, de l'$O_2$ et de l'Ar et dans lequel la puissance pour la production du plasma est de 6 W/ml à 0,1 W/ml par rapport au volume de la lumière de la seringue.

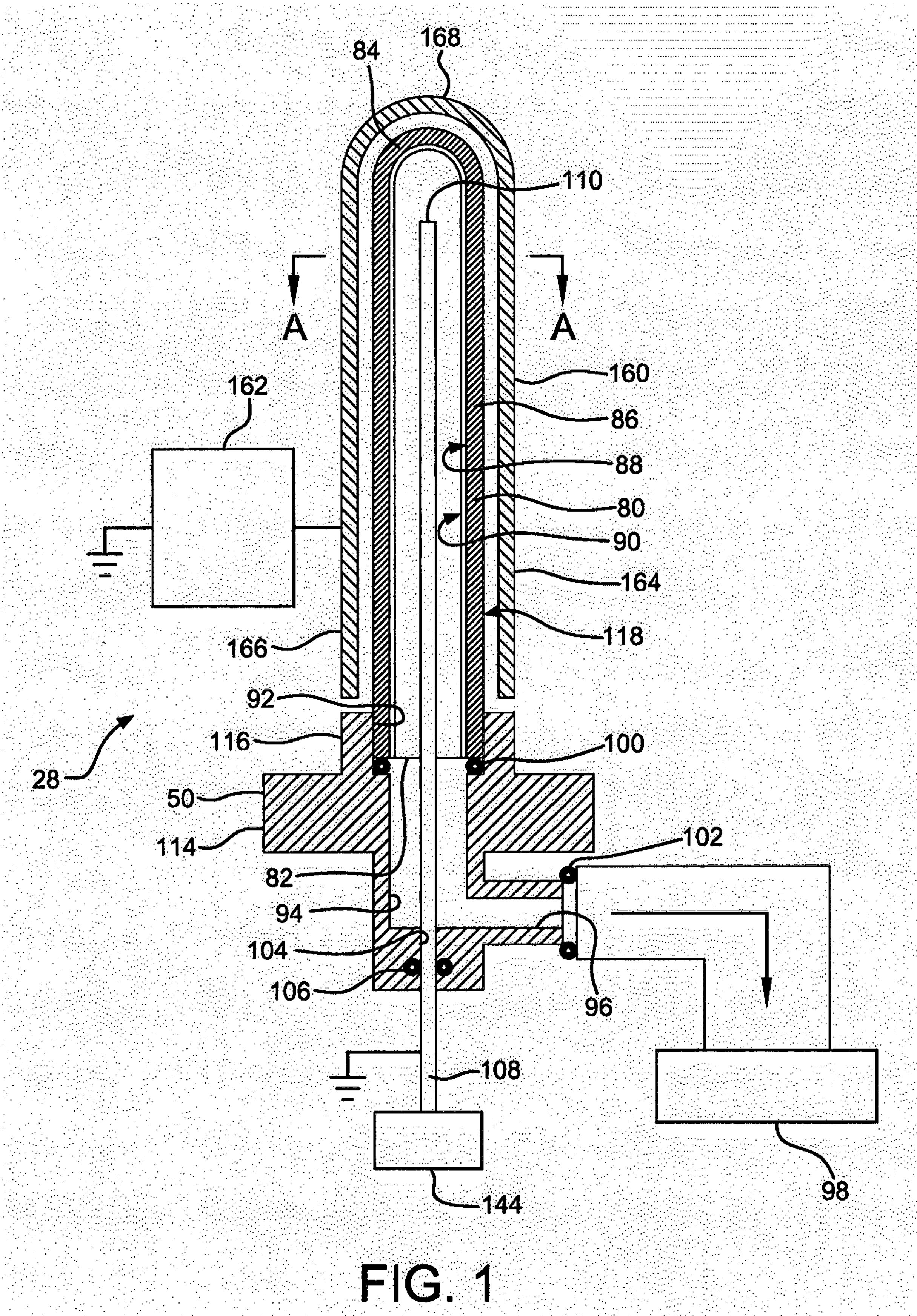
168
84
110
A
A
160
86
88
80
90
164
118
162
166
92
116
100
28
50
114
102
82
94
104
96
106
108
98
144

FIG. 1

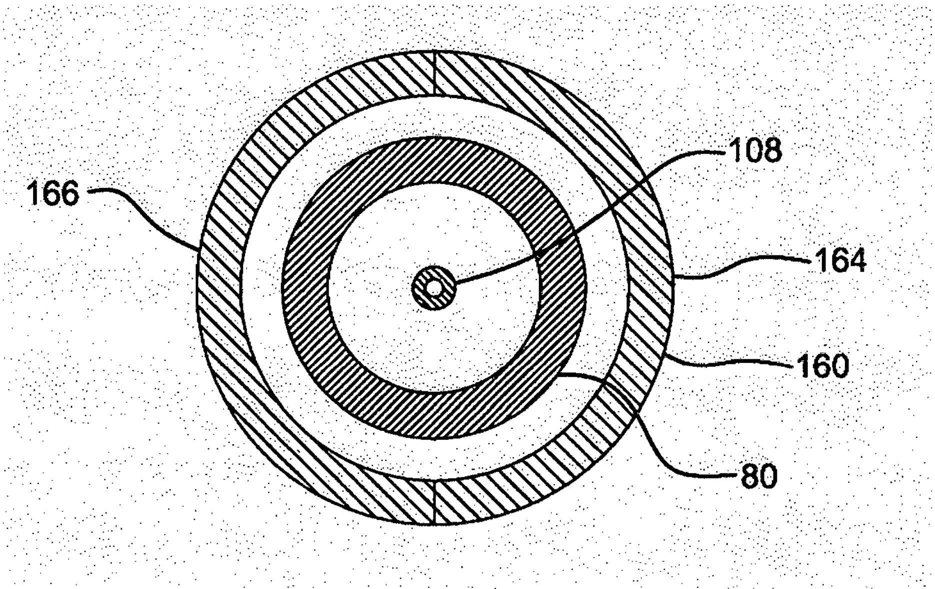


FIG. 2

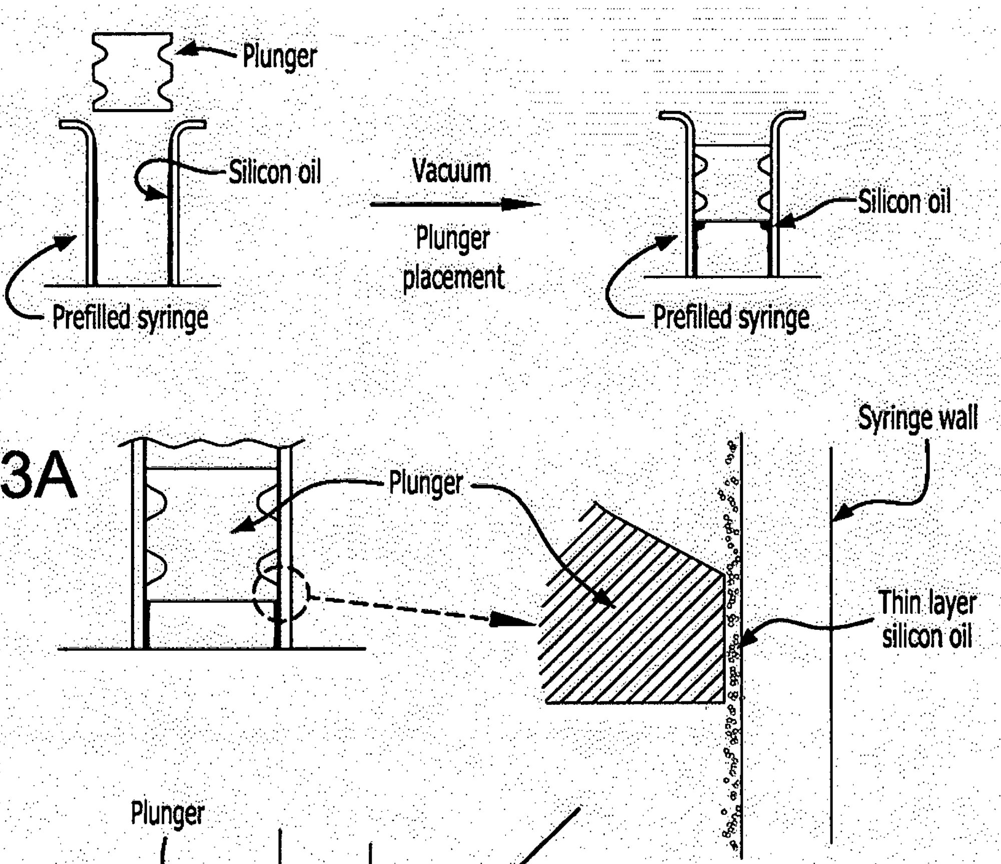


FIG. 3A

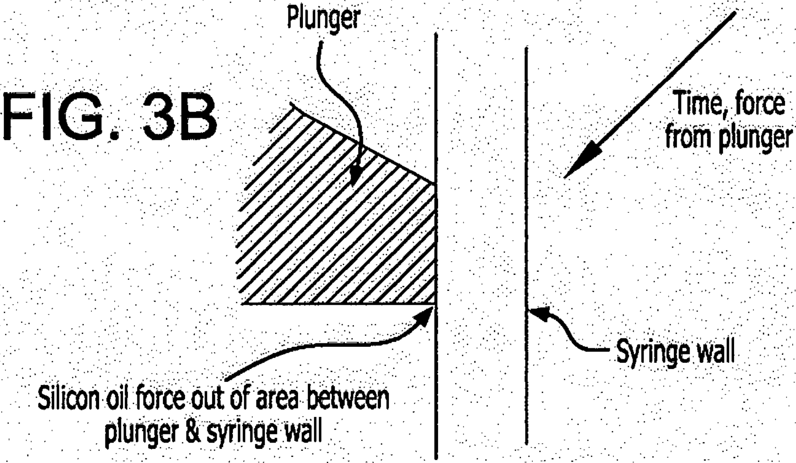


FIG. 3B

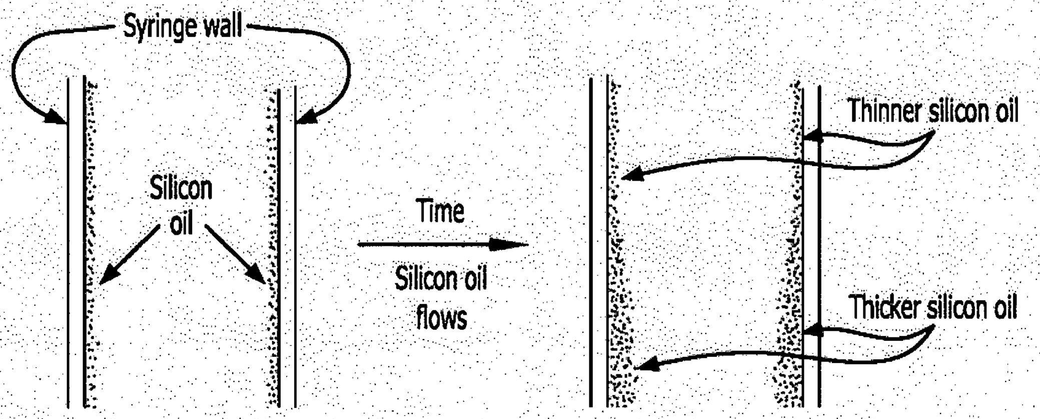


FIG. 3C

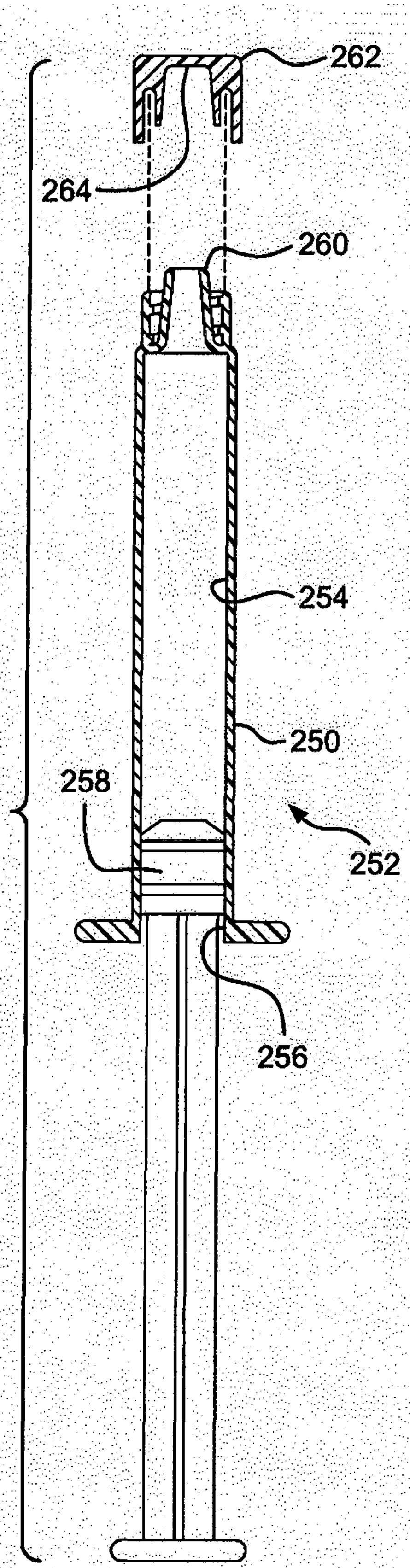


FIG. 4

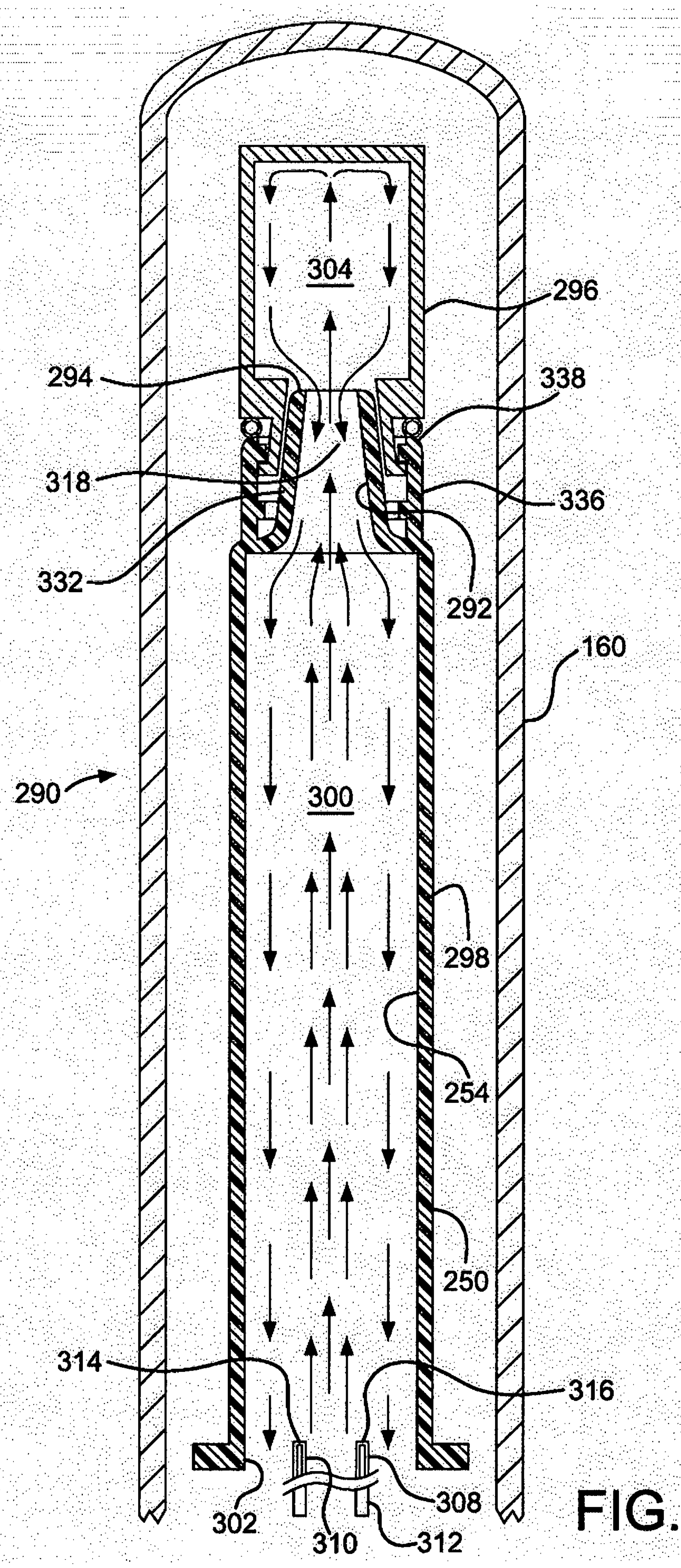
304
296
294
338
318
336
332
292
160
290
300
298
254
250
314
316
302
308
310
312

FIG. 8

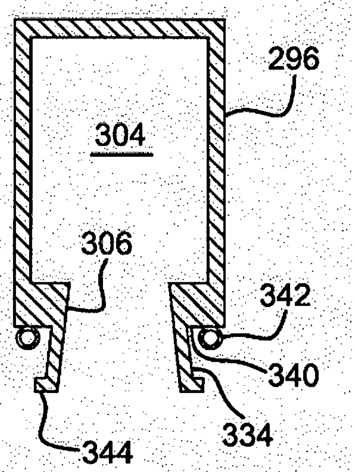
296
304
306
342
340
334
344

FIG. 9

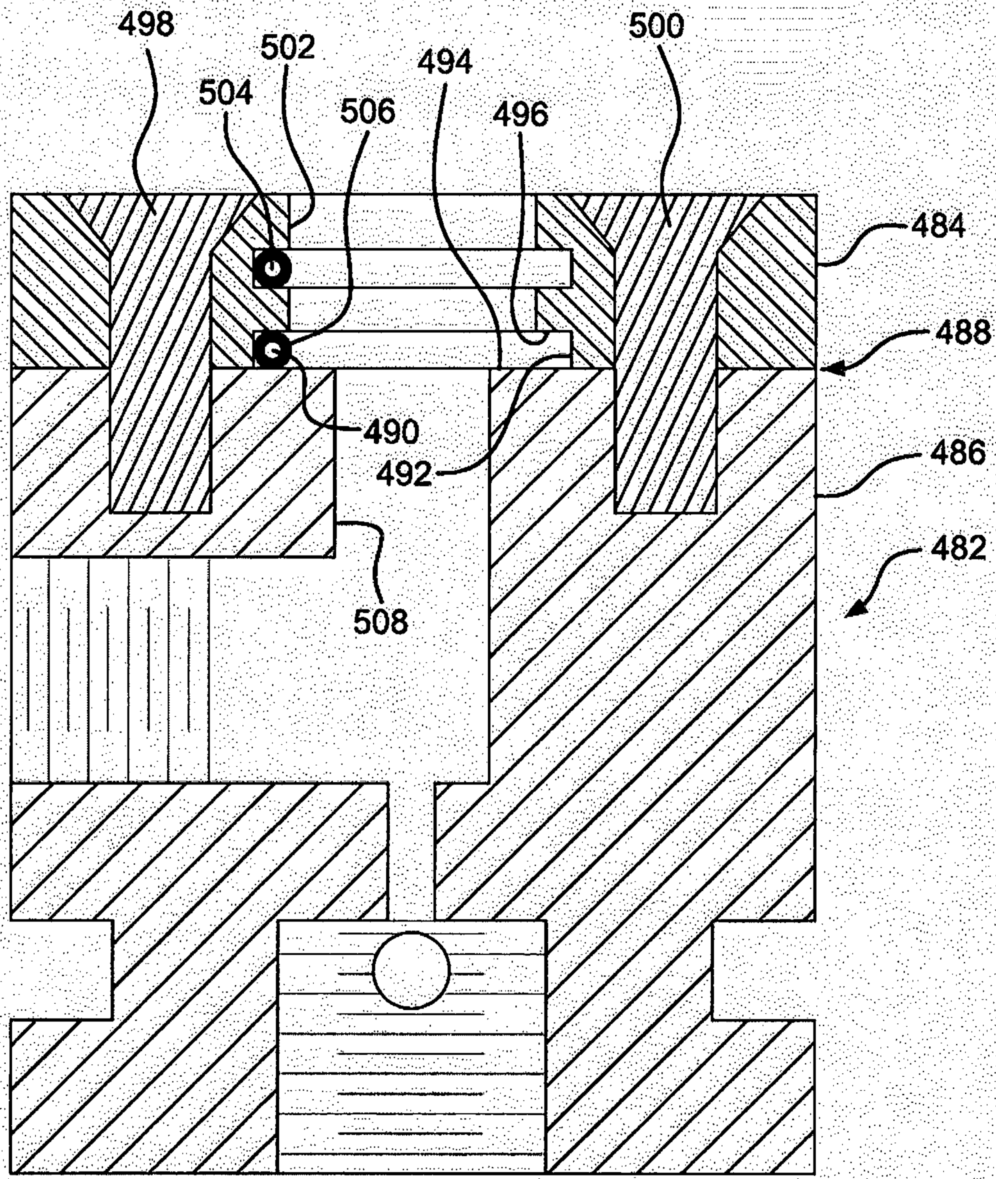


FIG. 10

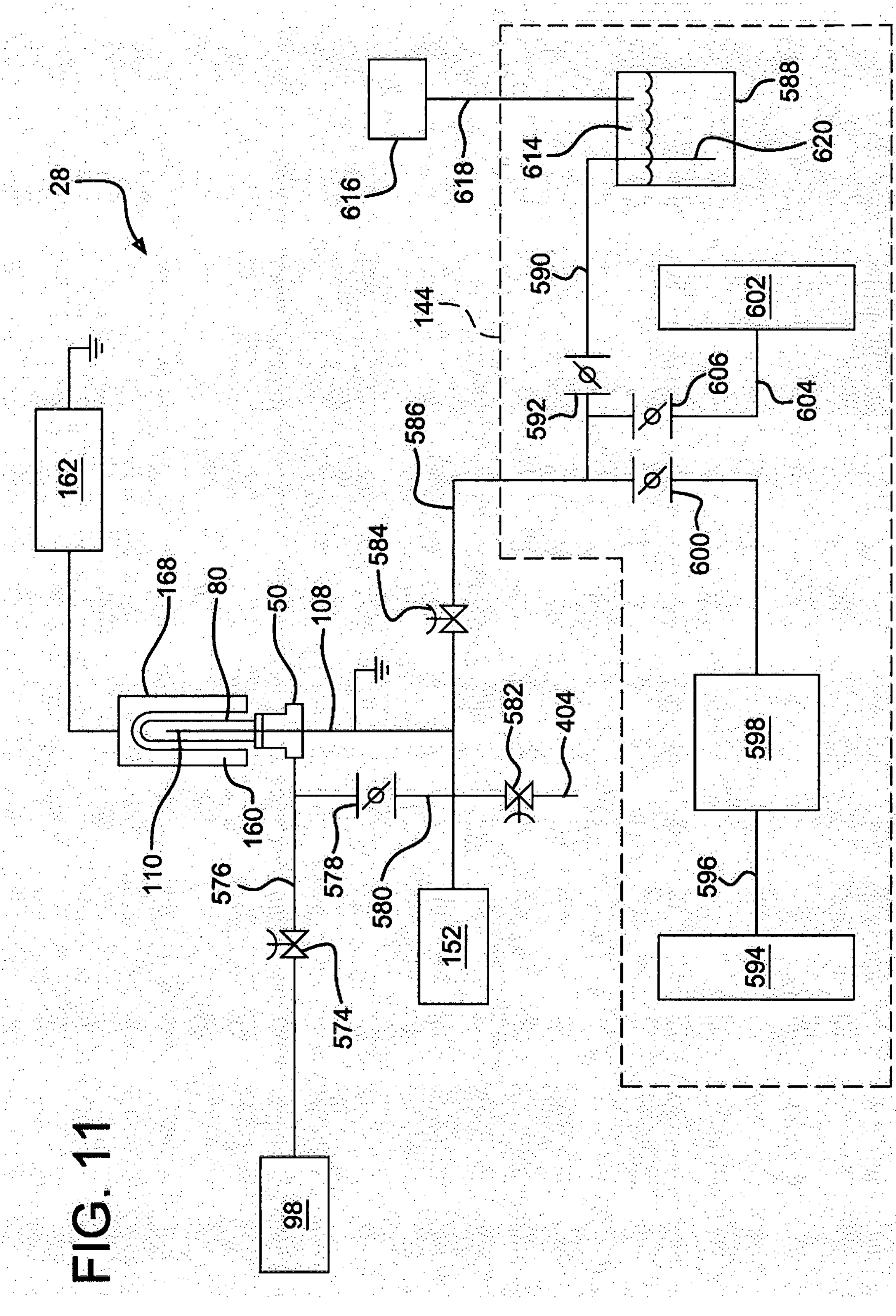
28
162
168
80
50
108
110
160
576
578
580
574
584
582
404
586
152
98
616
618
614
588
620
590
144
602
592
606
604
600
598
596
594

FIG. 11

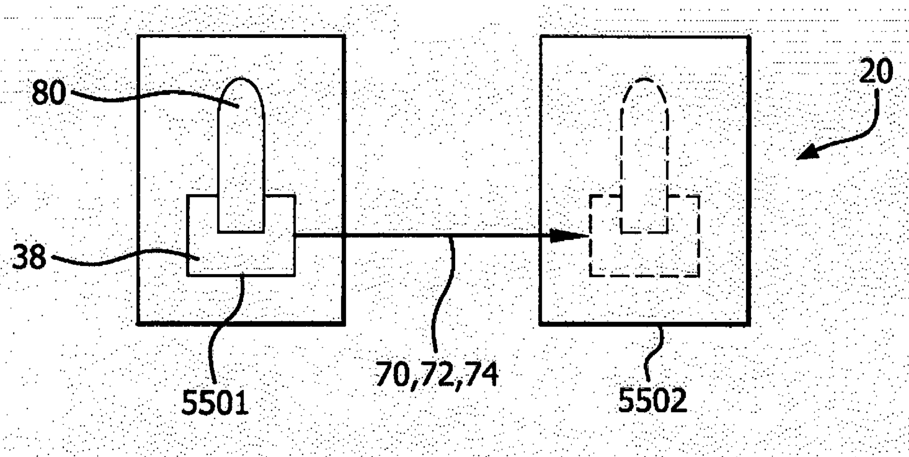


FIG. 12

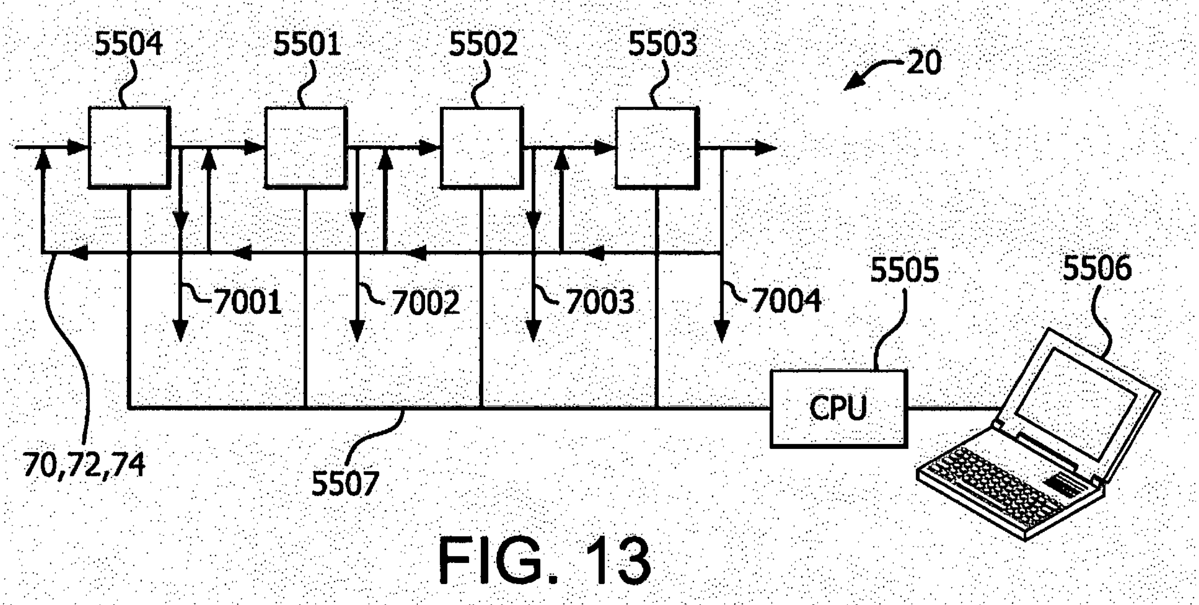


FIG. 13

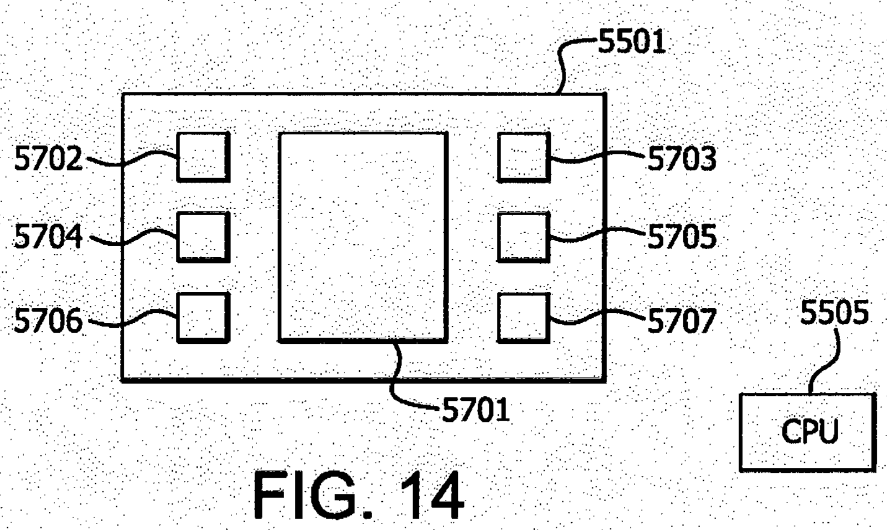


FIG. 14

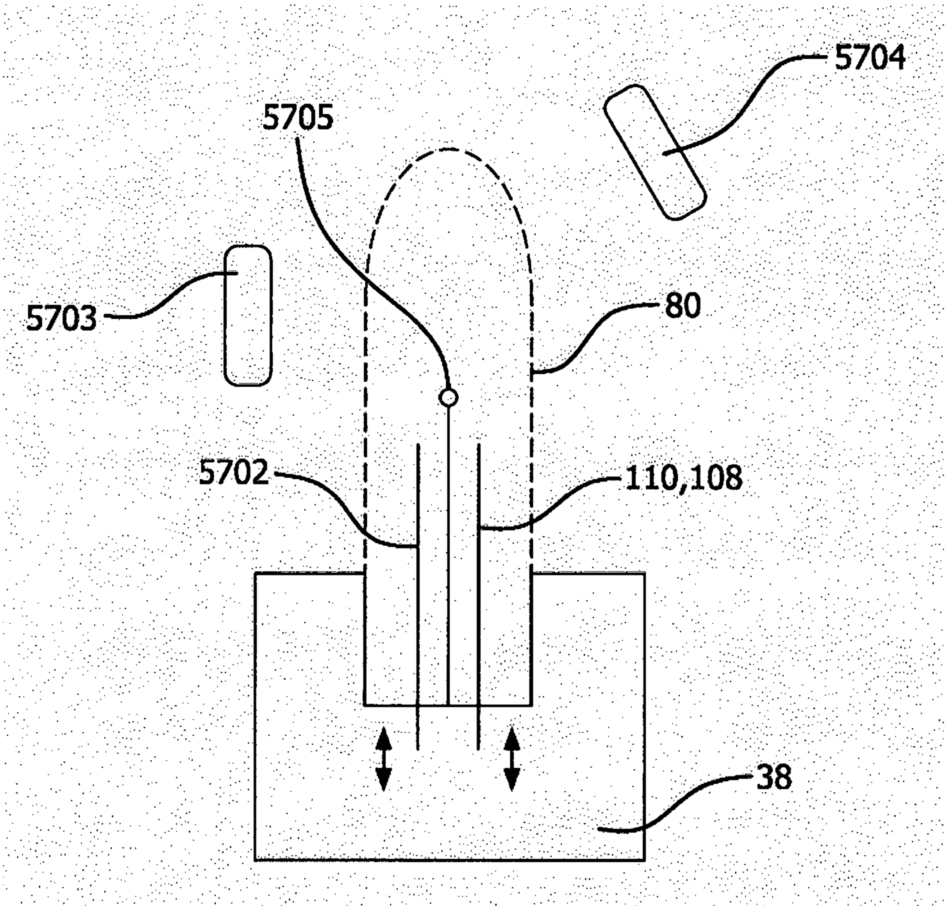


FIG. 15

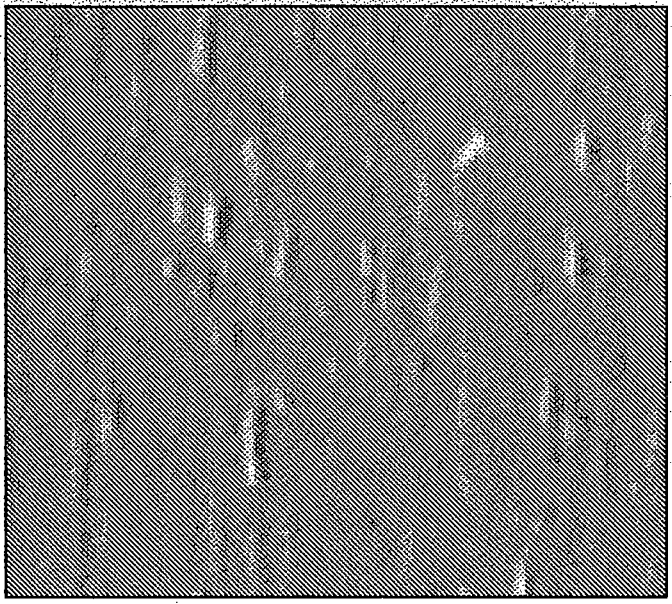

FIG. 16

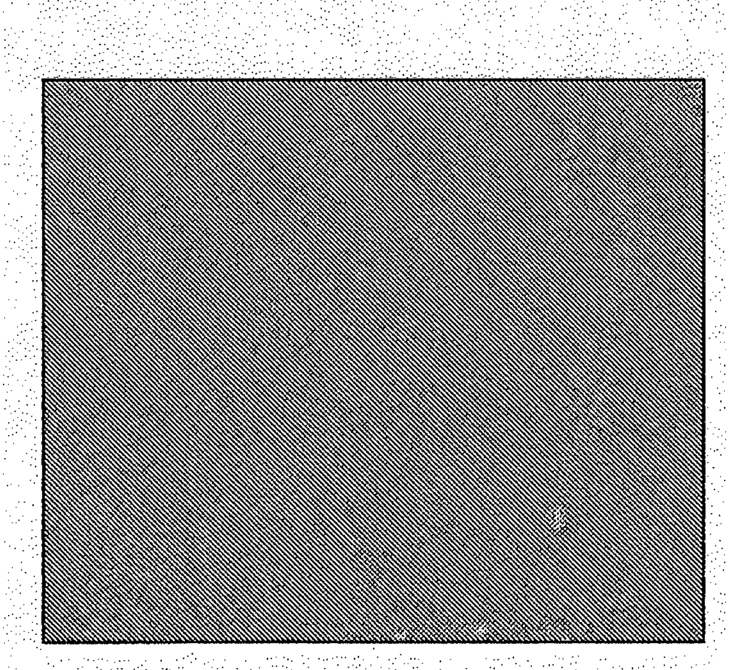

FIG. 17

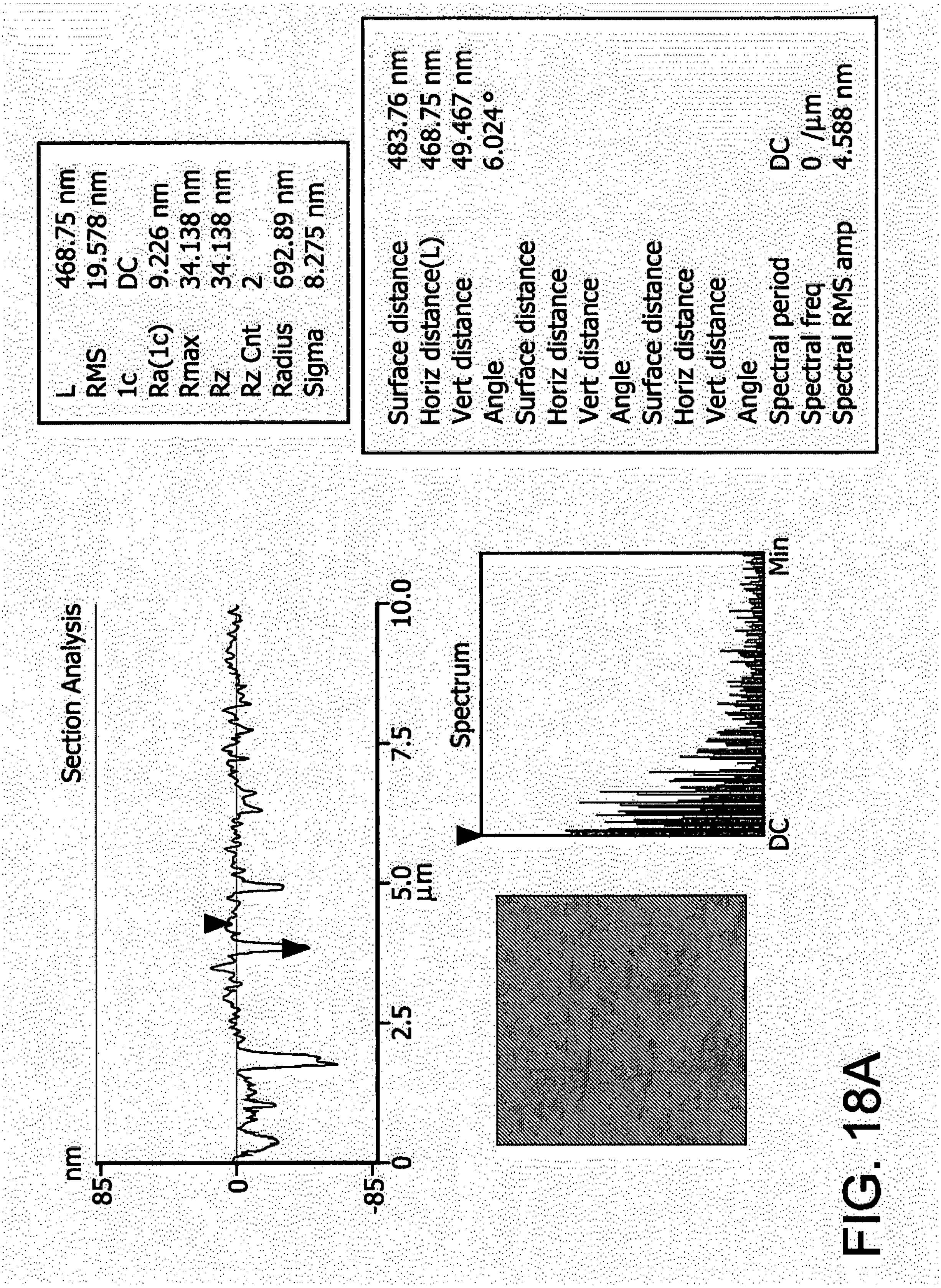
Section Analysis
nm
85
0
-85
0
2.5
5.0
7.5
10.0
µm
Spectrum
DC
Min
L 468.75 nm
RMS 19.578 nm
1c DC
Ra(1c) 9.226 nm
Rmax 34.138 nm
Rz 34.138 nm
Rz Cnt 2
Radius 692.89 nm
Sigma 8.275 nm
Surface distance 483.76 nm
Horiz distance(L) 468.75 nm
Vert distance 49.467 nm
Angle 6.024°
Surface distance
Horiz distance
Vert distance
Angle
Surface distance
Horiz distance
Vert distance
Angle
Spectral period DC
Spectral freq 0 /µm
Spectral RMS amp 4.588 nm

FIG. 18A

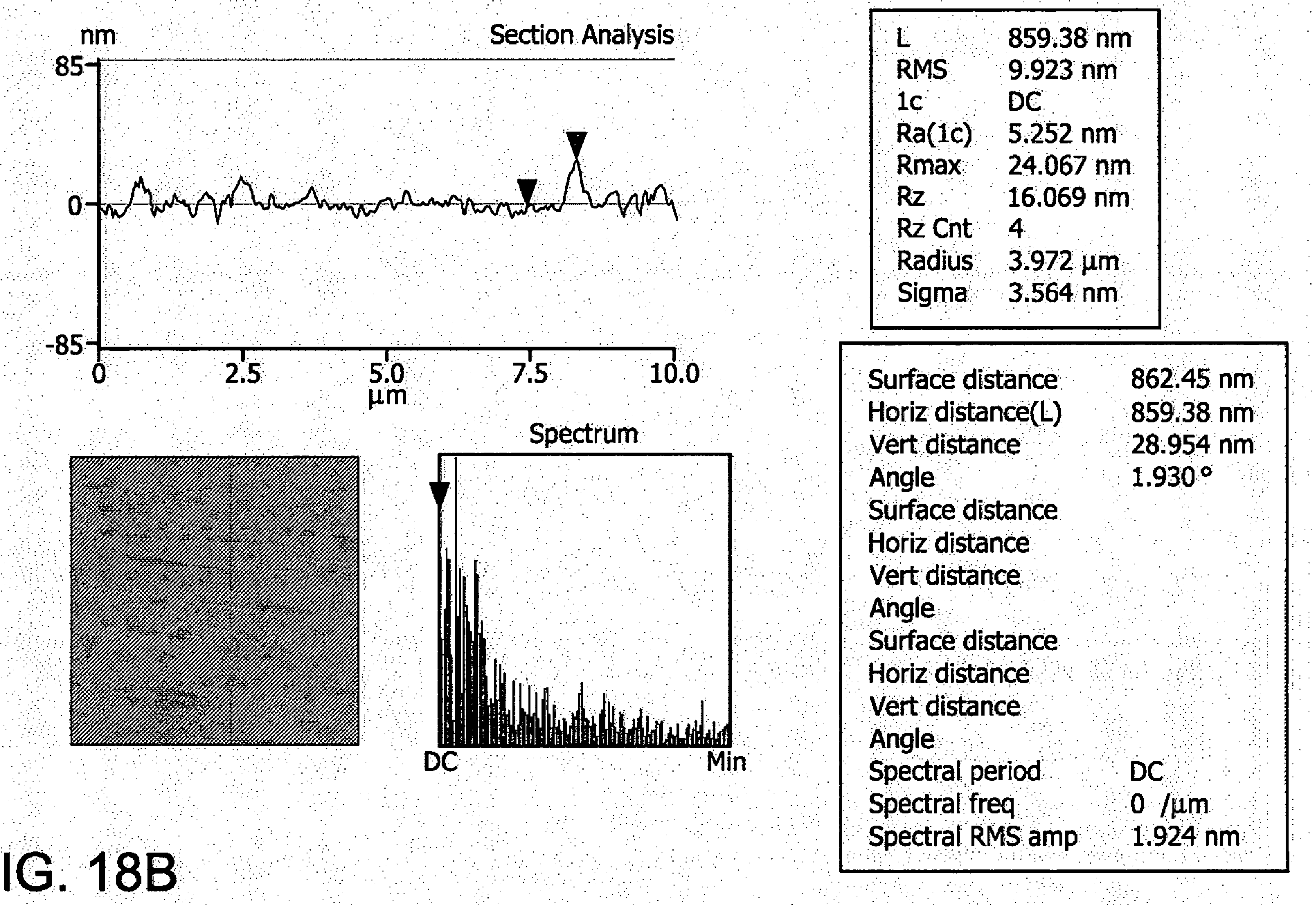
nm
Section Analysis
85
0
-85
0
2.5
5.0
µm
7.5
10.0
Spectrum
DC
Min
L 859.38 nm
RMS 9.923 nm
1c DC
Ra(1c) 5.252 nm
Rmax 24.067 nm
Rz 16.069 nm
Rz Cnt 4
Radius 3.972 µm
Sigma 3.564 nm
Surface distance 862.45 nm
Horiz distance(L) 859.38 nm
Vert distance 28.954 nm
Angle 1.930°
Surface distance
Horiz distance
Vert distance
Angle
Surface distance
Horiz distance
Vert distance
Angle
Spectral period DC
Spectral freq 0 /µm
Spectral RMS amp 1.924 nm

FIG. 18B

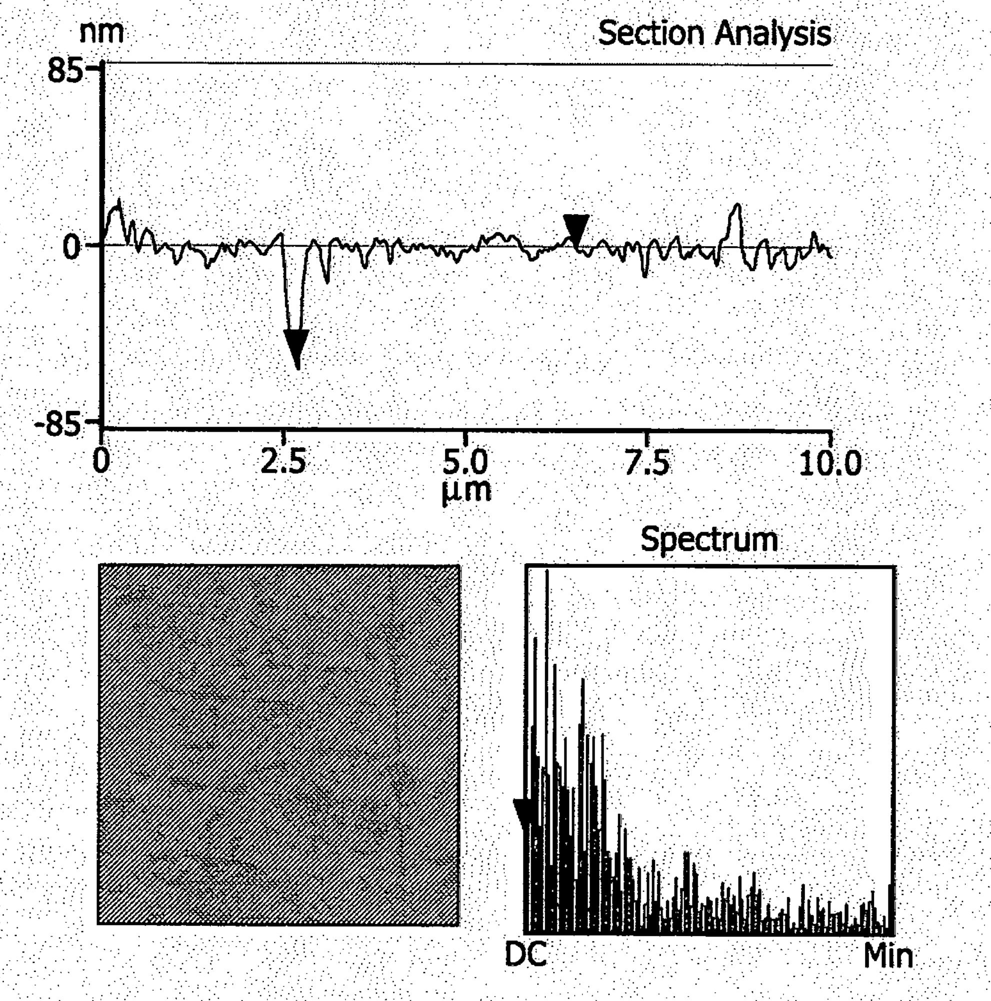


| | | |
|---|---|---|
| L | 3.828 | µm |
| RMS | 9.380 | nm |
| 1c | DC | |
| Ra(1c) | 4.843 | nm |
| Rmax | 61.241 | nm |
| Rz | 24.807 | nm |
| Rz Cnt | valid | |
| Radius | 22.470 | µm |
| Sigma | 23.311 | nm |

| | |
|---|---|
| Surface distance | 3.862 µm |
| Horiz distance(L) | 3.828 µm |
| Vert distance | 53.169 nm |
| Angle | 0.796 ° |
| Surface distance | |
| Horiz distance | |
| Vert distance | |
| Angle | |
| Surface distance | |
| Horiz distance | |
| Vert distance | |
| Angle | |
| Spectral period | DC |
| Spectral freq | 0 /µm |
| Spectral RMS amp | 0.827 nm |

FIG. 18C

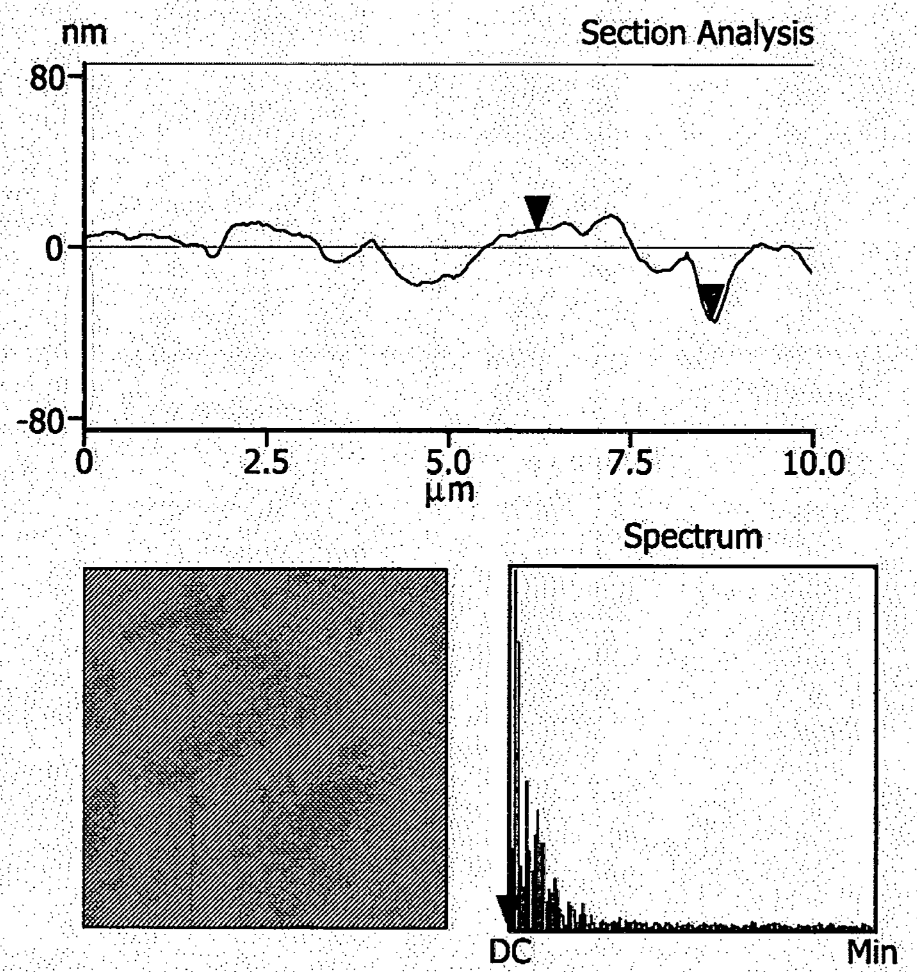


| | |
|---|---|
| L | 2.383 µm |
| RMS | 12.496 nm |
| 1c | DC |
| Ra(1c) | 5.481 nm |
| Rmax | 28.584 nm |
| Rz | 24.067 nm |
| Rz Cnt | 4 |
| Radius | 20.281 µm |
| Sigma | 8.379 nm |

| | |
|---|---|
| Surface distance | 2.386 µm |
| Horiz distance(L) | 2.383 µm |
| Vert distance | 42.061 nm |
| Angle | 1.011 ° |
| Surface distance | |
| Horiz distance | |
| Vert distance | |
| Angle | |
| Surface distance | |
| Horiz distance | |
| Vert distance | |
| Angle | |
| Spectral period | |
| Spectral freq | |
| Spectral RMS amp | |

FIG. 19A

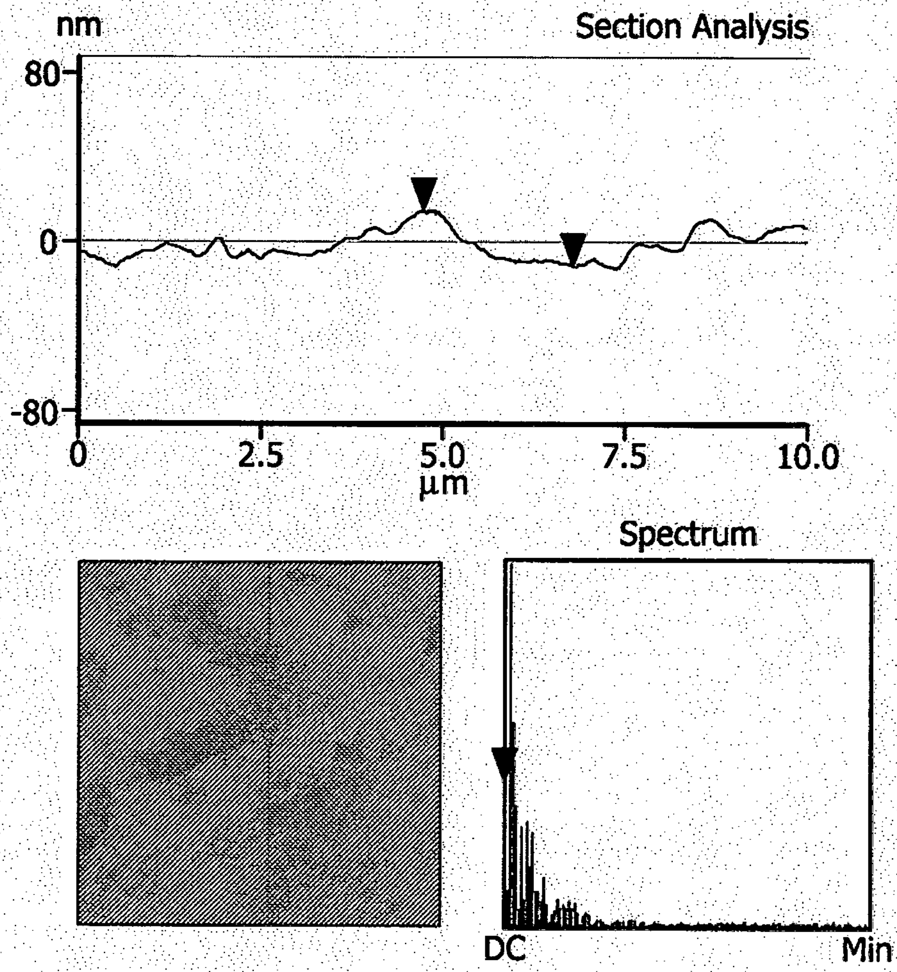


| | |
|---|---|
| L | 2.031 µm |
| RMS | 8.367 nm |
| 1c | DC |
| Ra(1c) | 3.247 nm |
| Rmax | 11.478 nm |
| Rz | 11.478 nm |
| Rz Cnt | 2 |
| Radius | 41.267 µm |
| Sigma | 1.441 nm |

| | |
|---|---|
| Surface distance | 2.032 µm |
| Horiz distance(L) | 2.031 µm |
| Vert distance | 26.140 nm |
| Angle | 0.737 ° |
| Surface distance | |
| Horiz distance | |
| Vert distance | |
| Angle | |
| Surface distance | |
| Horiz distance | |
| Vert distance | |
| Angle | |
| Spectral period | DC |
| Spectral freq | 0 /µm |
| Spectral RMS amp | 1.996 nm |

FIG. 19B

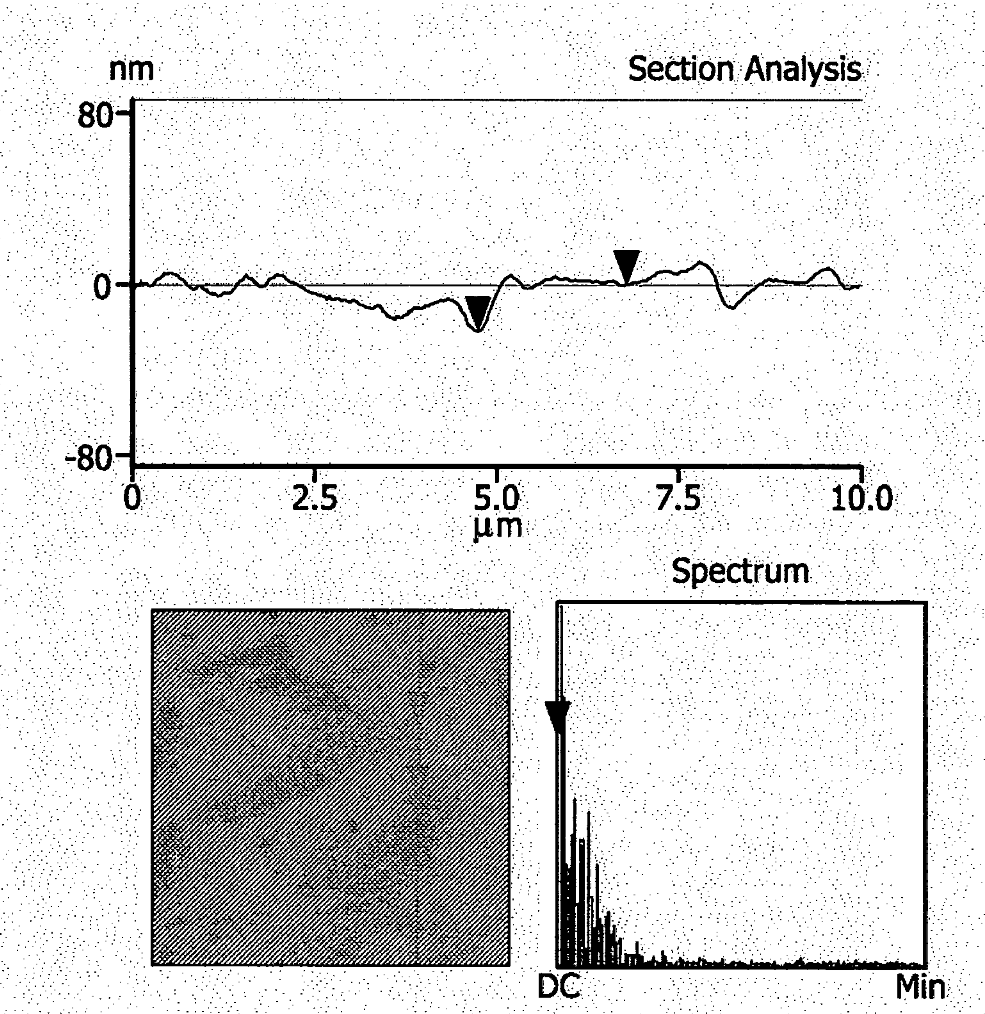


| | |
|---|---|
| L | 2.070 µm |
| RMS | 6.113 nm |
| 1c | DC |
| Ra(1c) | 3.597 nm |
| Rmax | 23.400 nm |
| Rz | 23.400 nm |
| Rz Cnt | 2 |
| Radius | 23.794 µm |
| Sigma | 4.764 nm |

| | |
|---|---|
| Surface distance | 2.072 µm |
| Horiz distance(L) | 2.070 µm |
| Vert distance | 21.697 nm |
| Angle | 0.600 ° |
| Surface distance | |
| Horiz distance | |
| Vert distance | |
| Angle | |
| Surface distance | |
| Horiz distance | |
| Vert distance | |
| Angle | |
| Spectral period | DC |
| Spectral freq | 0 /µm |
| Spectral RMS amp | 2.512 nm |

FIG. 19C

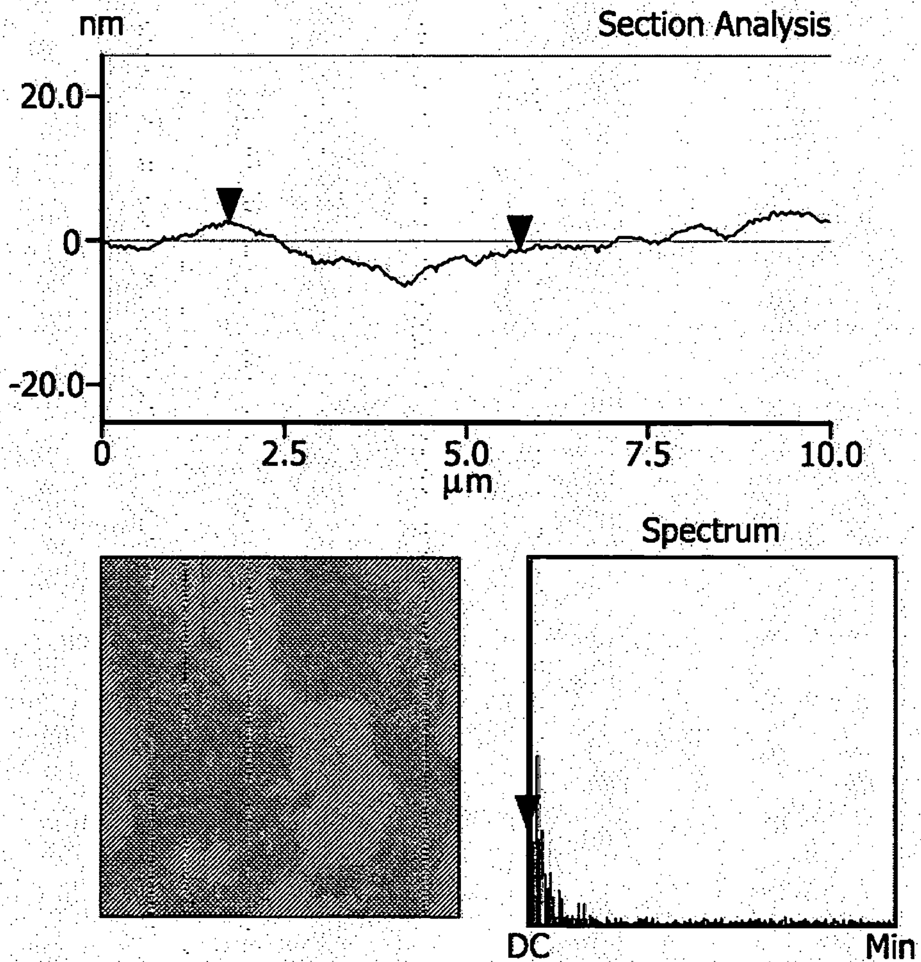


| | | |
|---|---|---|
| L | 4.023 | µm |
| RMS | 1.929 | nm |
| 1c | DC | |
| Ra(1c) | 1.488 | nm |
| Rmax | 6.666 | nm |
| Rz | 4.740 | nm |
| Rz Cnt | 4 | |
| Radius | 329.97 | µm |
| Sigma | 0.718 | nm |

| | |
|---|---|
| Surface distance | 4.024 µm |
| Horiz distance(L) | 4.023 µm |
| Vert distance | 3.777 nm |
| Angle | 0.054 ° |
| Surface distance | |
| Horiz distance | |
| Vert distance | |
| Angle | |
| Surface distance | |
| Horiz distance | |
| Vert distance | |
| Angle | |
| Spectral period | DC |
| Spectral freq | 0 /µm |
| Spectral RMS amp | 0.489 nm |

FIG. 20A

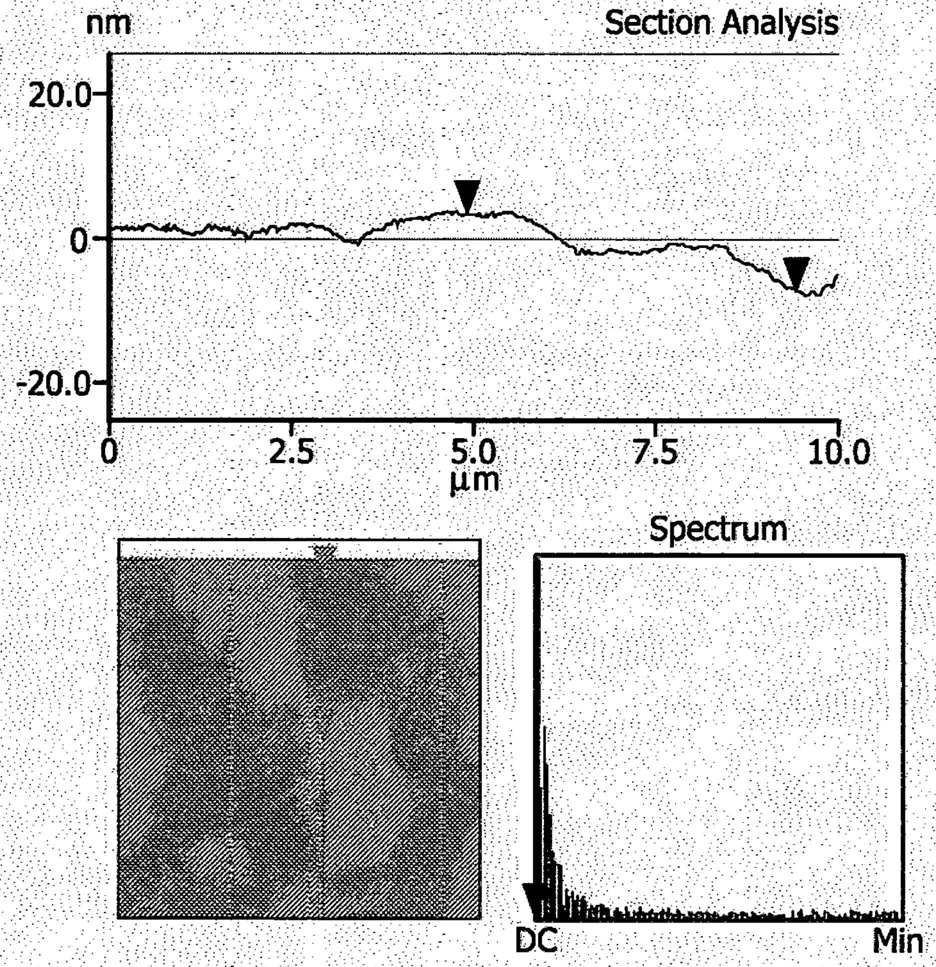


| | |
|---|---|
| L | 4.492 nm |
| RMS | 2.642 nm |
| 1c | DC |
| Ra(1c) | 1.068 nm |
| Rmax | 4.740 nm |
| Rz | 2.889 nm |
| Rz Cnt | 6 |
| Radius | 45.842 µm |
| Sigma | 16.712 nm |

| | |
|---|---|
| Surface distance | 4.492 µm |
| Horiz distance(L) | 4.492 µm |
| Vert distance | 10.517 nm |
| Angle | 0.134° |
| Surface distance | |
| Horiz distance | |
| Vert distance | |
| Angle | |
| Surface distance | |
| Horiz distance | |
| Vert distance | |
| Angle | |
| Spectral period | DC |
| Spectral freq | 0 /µm |
| Spectral RMS amp | 0 nm |

FIG. 20B

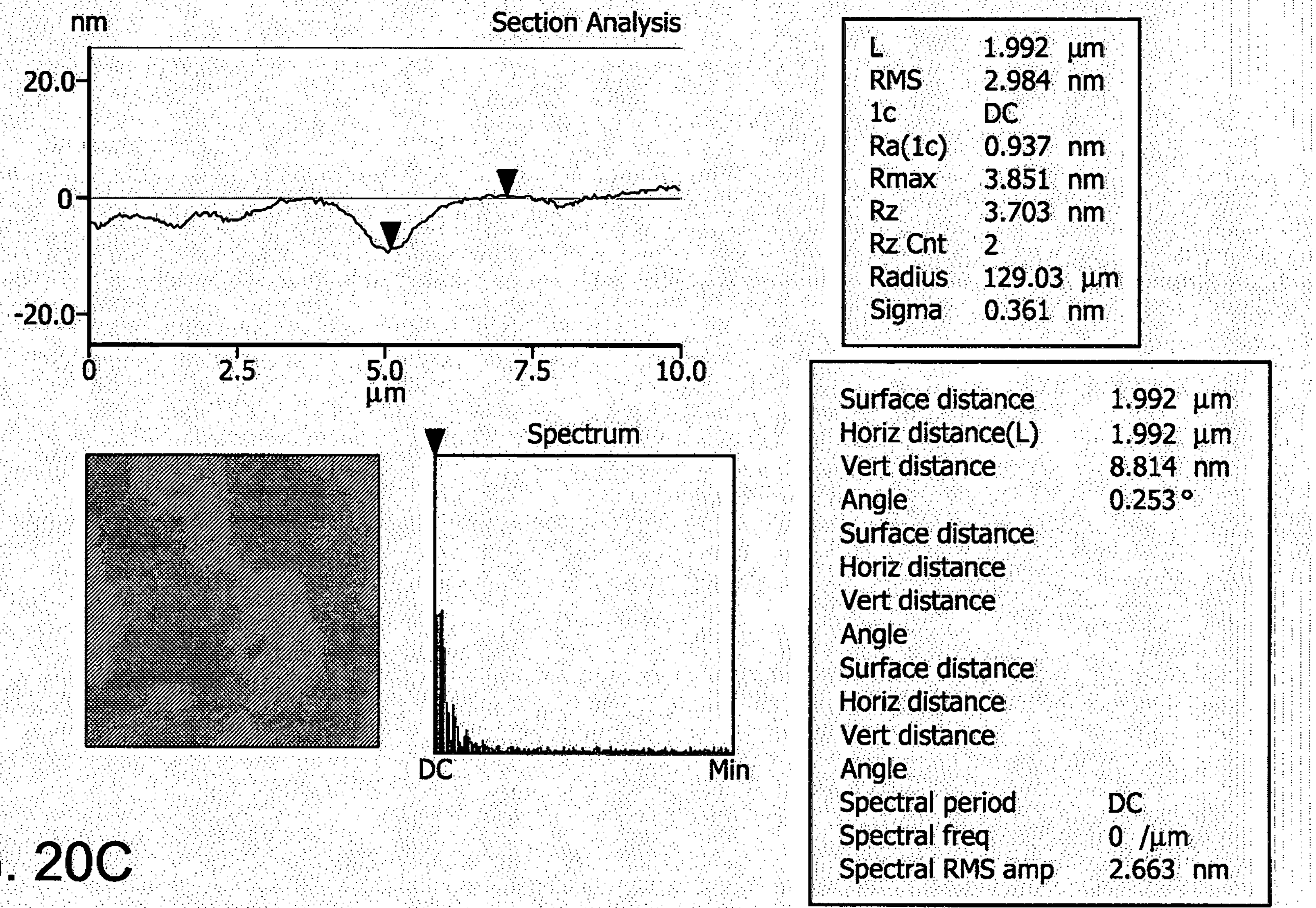
Section Analysis
nm
20.0
0
-20.0
0
2.5
5.0
7.5
10.0
µm
Spectrum
DC
Min
L 1.992 µm
RMS 2.984 nm
1c DC
Ra(1c) 0.937 nm
Rmax 3.851 nm
Rz 3.703 nm
Rz Cnt 2
Radius 129.03 µm
Sigma 0.361 nm
Surface distance 1.992 µm
Horiz distance(L) 1.992 µm
Vert distance 8.814 nm
Angle 0.253°
Surface distance
Horiz distance
Vert distance
Angle
Surface distance
Horiz distance
Vert distance
Angle
Spectral period DC
Spectral freq 0 /µm
Spectral RMS amp 2.663 nm

FIG. 20C

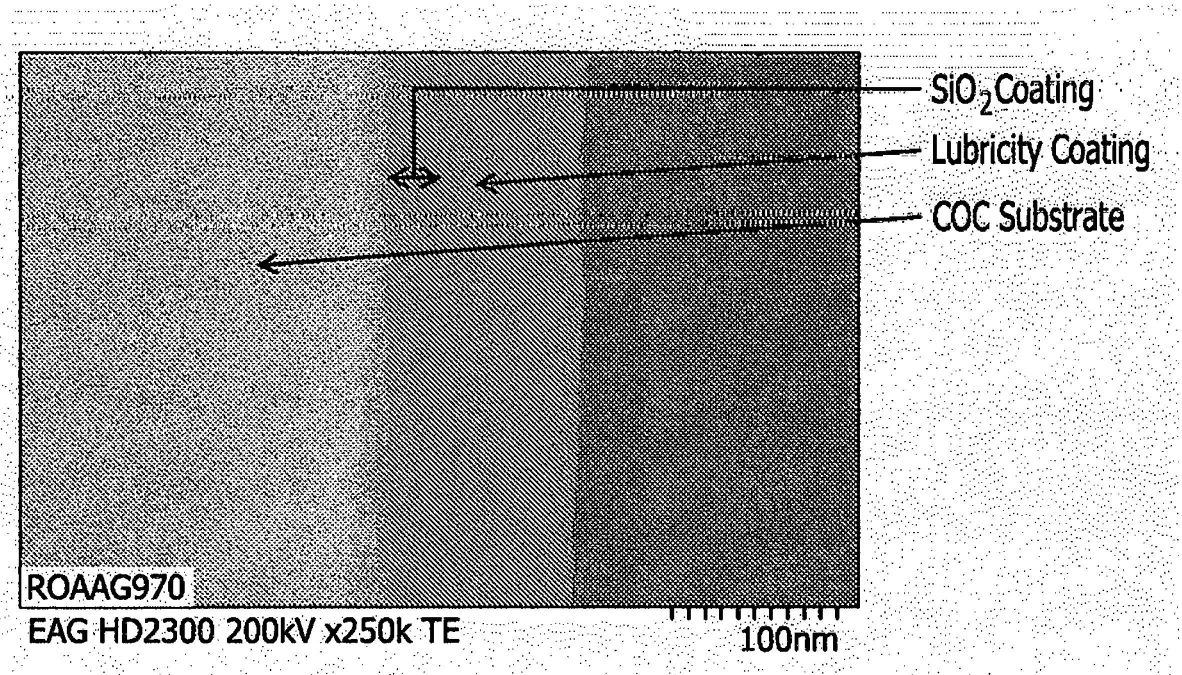
SiO2 Coating
Lubricity Coating
COC Substrate
ROAAG970
EAG HD2300 200kV x250k TE
100nm

FIG. 21

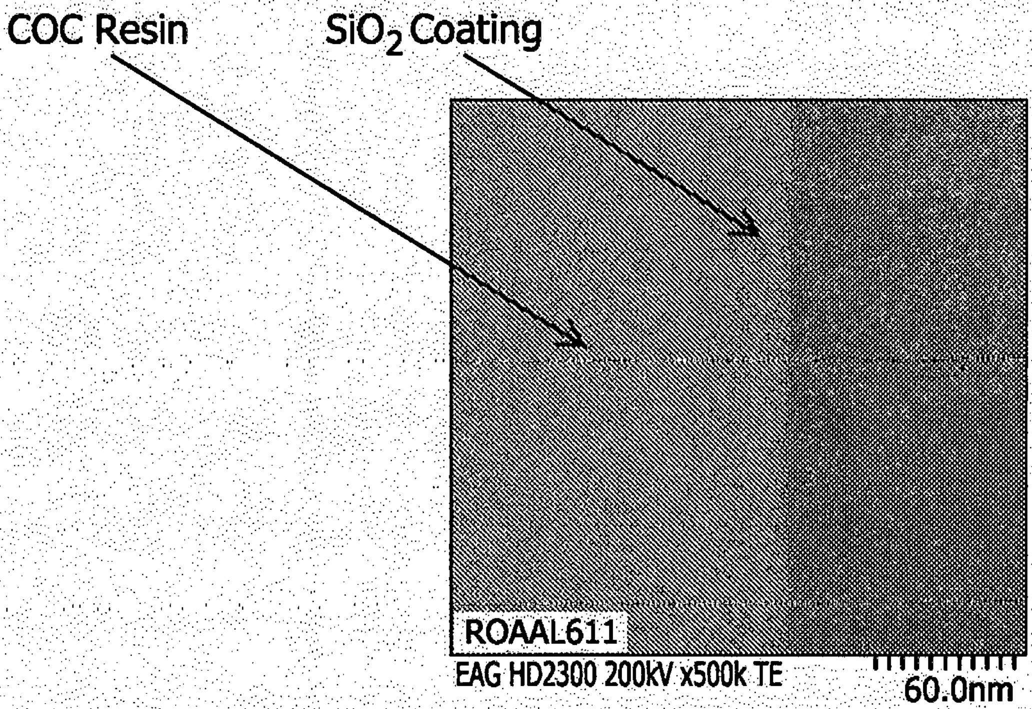
COC Resin
SiO2 Coating
ROAAL611
EAG HD2300 200kV x500k TE
60.0nm

FIG. 22

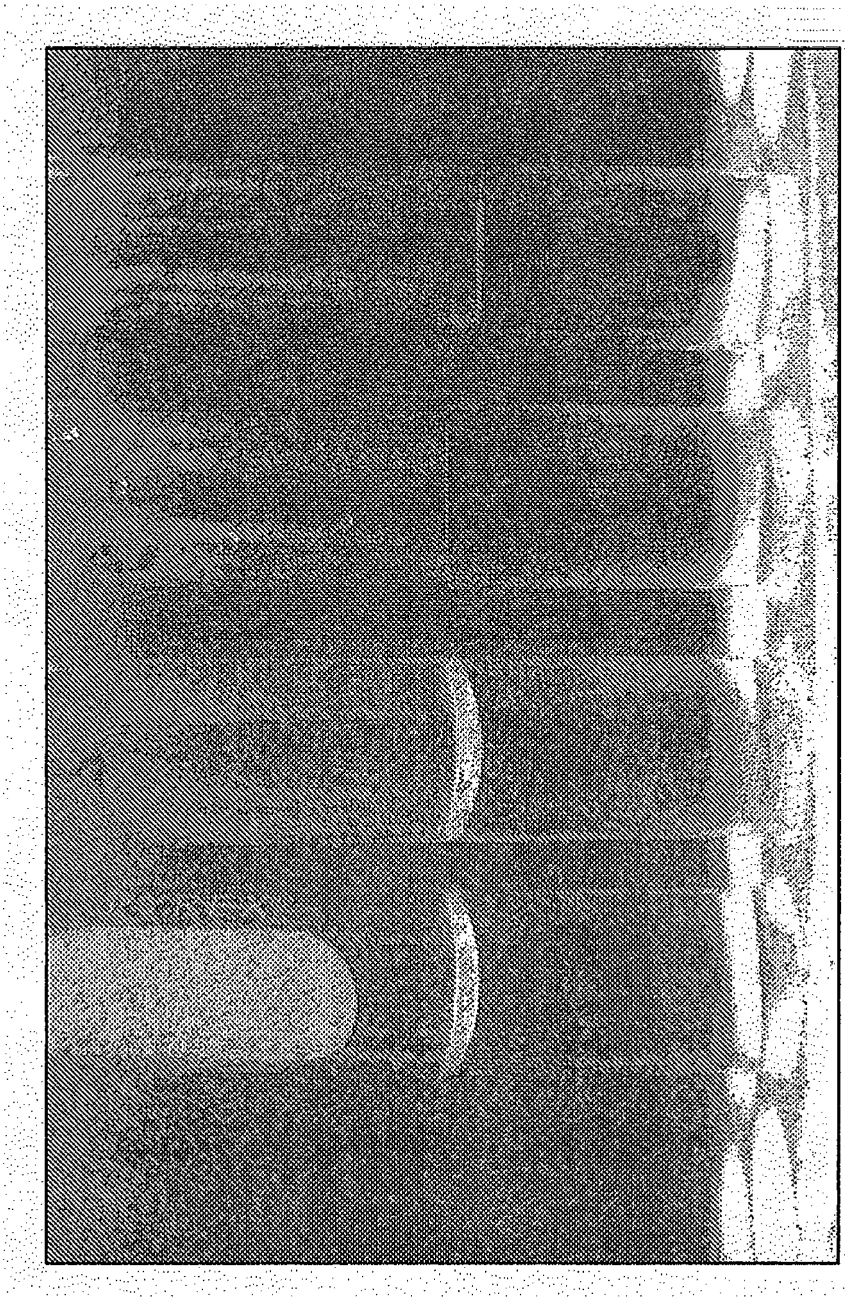


FIG. 23

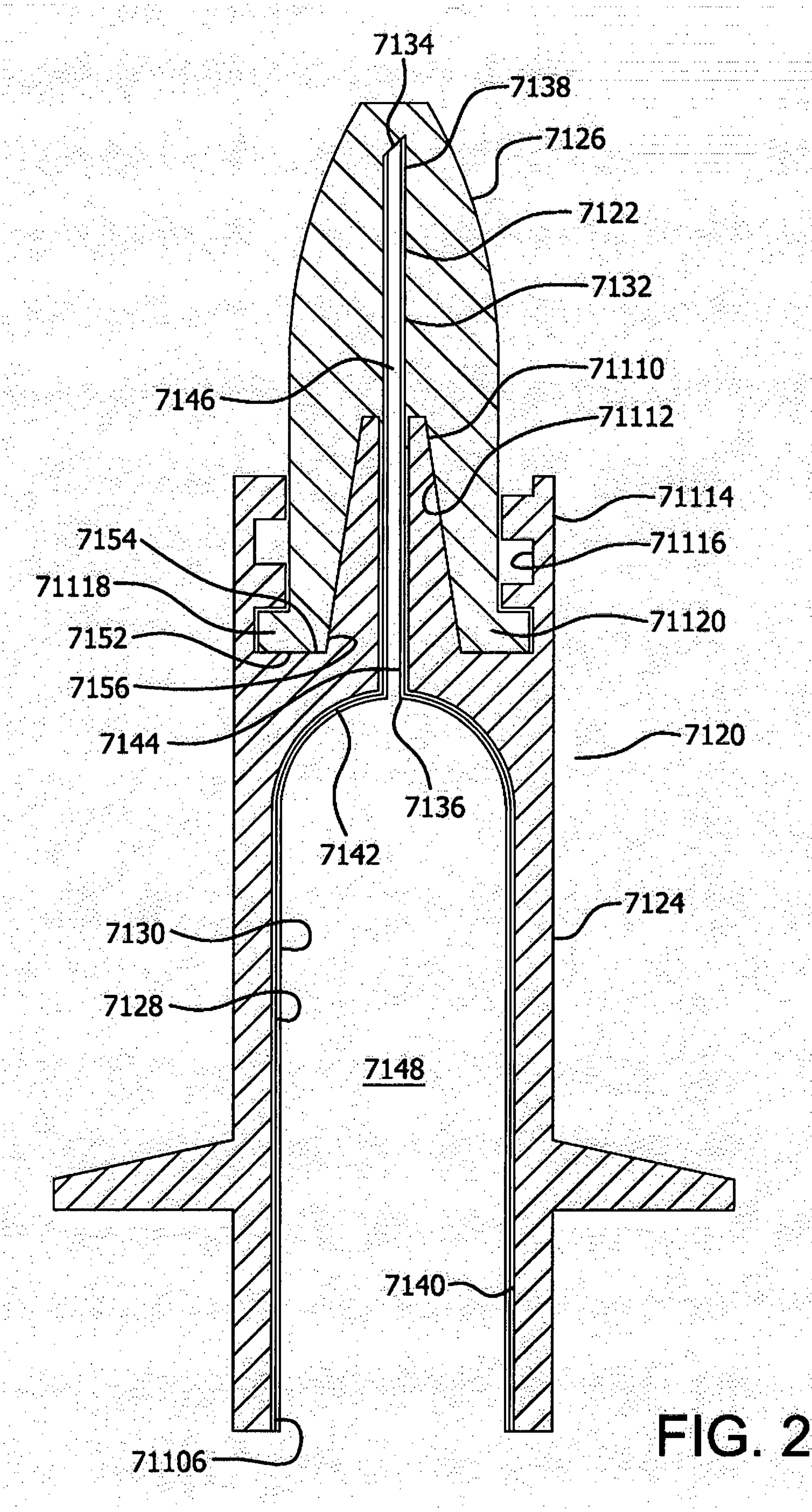
7134
7138
7126
7122
7132
7146
71110
71112
71114
7154
71116
71118
71120
7152
7156
7144
7120
7136
7142
7124
7130
7128
7148
7140
71106

FIG. 24

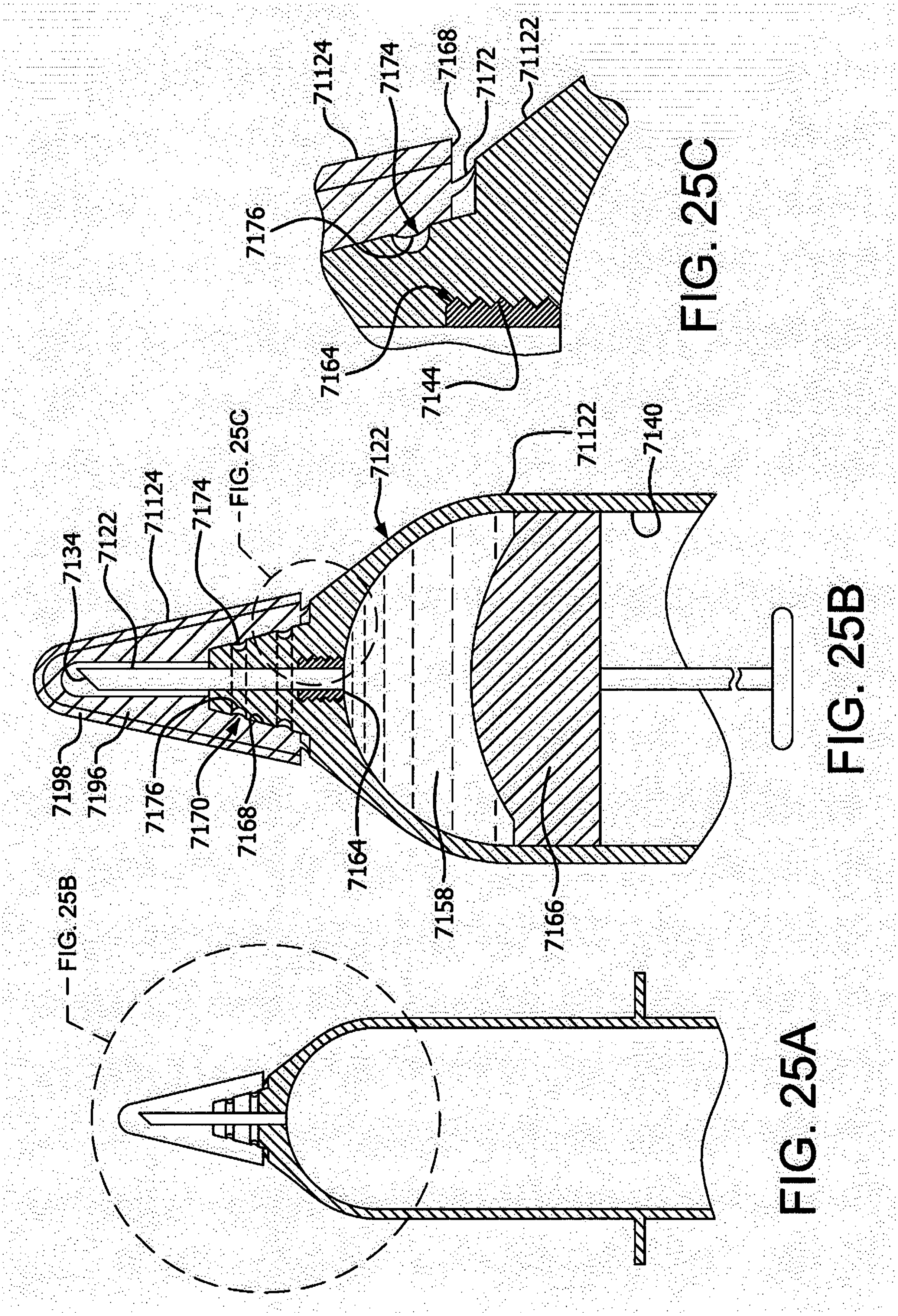


FIG. 25A

FIG. 25B

FIG. 25C

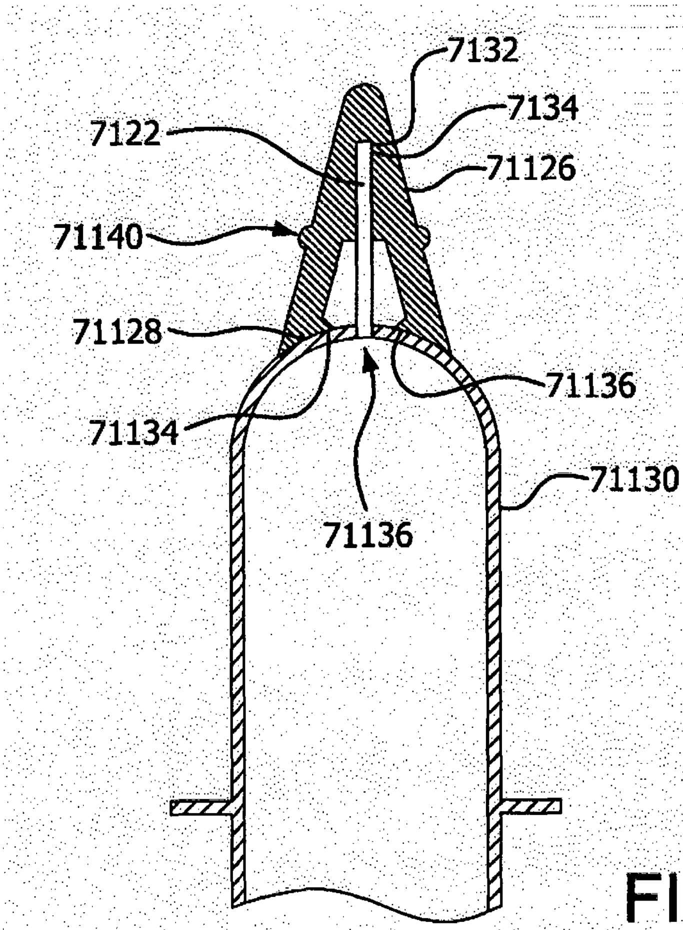
7132
7134
7122
71126
71140
71128
71136
71134
71130
71136

FIG. 26

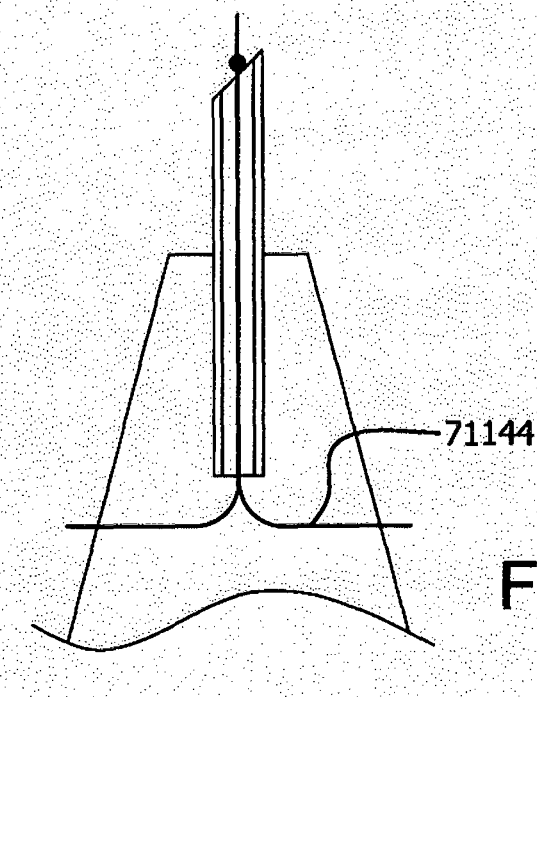
71144

FIG. 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 10162755 A
- EP 10162760 A
- EP 10162756 A
- EP 10162758 A
- EP 10162761 A
- EP 10162757 A
- US 6068884 A
- US 4844986 A
- US 20060046006 A
- US 20040267194 A
- EP 0329041 A2
- US 5494712 A
- US 5679413 A
- US 61359434 A
- US 20100298738 A1
- EP 2251455 A2
- US 61359434 B
- US 5904952 A
- EP 2251455 A